(19) Europäisches Patentamt
European Patent Office
Office européen des brevets

(11) EP 2 518 653 B1

(12) **EUROPEAN PATENT SPECIFICATION**

(45) Date of publication and mention of the grant of the patent:
**20.02.2019 Bulletin 2019/08**

(51) Int Cl.:
***G16H 50/20*** *(2018.01)*

(21) Application number: **12165579.9**

(22) Date of filing: **25.04.2012**

(54) **Information processing apparatus and user terminal**

Informationsverarbeitungsvorrichtung und Benutzerendgerät

Appareil de traitement d'informations et terminal utilisateur

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2011 JP 2011097097**
**14.10.2011 JP 2011227033**
**14.02.2012 JP 2012029658**

(43) Date of publication of application:
**31.10.2012 Bulletin 2012/44**

(73) Proprietor: **ARKRAY, Inc.**
**Minami-ku**
**Kyoto-shi**
**Kyoto 601-8045 (JP)**

(72) Inventors:
- **Sekimoto, Shinjiro**
**Kyoto, Kyoto 602-0008 (JP)**
- **Iketani, Kazuya**
**kyoto, Kyoto 602-0008 (JP)**

(74) Representative: **MacDougall, Alan John Shaw et al**
**Mathys & Squire LLP**
**The Shard**
**32 London Bridge Street**
**London SE1 9SG (GB)**

(56) References cited:
**US-A1- 2010 162 786** **US-A1- 2010 330 598**

- **D. M. NATHAN ET AL: "Translating the A1C Assay Into Estimated Average Glucose Values", DIABETES CARE, vol. 31, no. 8, 7 June 2008 (2008-06-07), pages 1473-1478, XP055099114, ISSN: 0149-5992, DOI: 10.2337/dc08-0545**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

BACKGROUND OF THE INVENTION

Field of the Invention:

The present invention relates to an information processing apparatus and a user terminal.

Description of the Related Art:

In recent years, the number of diabetes patients is in an increasing tendency worldwide. The diabetes is such a disease that a state in which the blood sugar level (blood glucose level) to indicate the concentration of grape sugar in blood (glucose concentration in blood) is high, i.e., the hyperglycemia continues chronically. When the grape sugar, which is the energy source, is incorporated into cells in the human body, a hormone called "insulin" is required. When the amount of secretion of insulin is decreased and/or the sensitivity of the cell to insulin is lowered, then the grape sugar (glucose) is flooded in the blood, and the hyperglycemia is caused.

At present, it is said that the number of diabetes patients is several millions in Japan. It is also said that the number arrives at several ten millions all over the country if the number includes the latent or potential borderline diabetes patients who are considered as people at risk of diabetes. The daily management of blood sugar level is extremely important for the diabetes patients as a mater of course as well as for the borderline diabetes patients who are people at risk of diabetes.

At present, a variety of blood sugar level measuring apparatuses are commercially available in order to measure the blood sugar level of a person himself/herself. Those known as the blood sugar level measuring apparatus as described above include, for example, an SMBG measuring apparatus for performing the self-monitoring of blood sugar level (SMBG: self-monitoring of blood glucose) and a CGM measuring apparatus for performing the continuous blood sugar measurement (CGM: Continuous Glucose Monitoring). SMBG resides in such a method that the blood, which is collected, for example, from a fingertip by using a puncture instrument (device), is adhered to a test strip (test piece) installed to a measuring device to measure the blood sugar level. On the other hand, CGM resides in such a method that a minute sensor, which includes an electrode and an enzyme that is reactive with glucose, is retained subcutaneously to continuously measure the grape sugar concentration in the subcutaneous intercellular fluid. In general, in the case of the CGM measuring apparatus, a glucose sensor is retained subcutaneously over a span of about several days to several weeks to continuously measure the blood sugar level at every interval of several ten seconds to several minutes.

The index, which is usable to evaluate the ability of glucose metabolism in the body fluid (in the blood or the intercellular fluid), is exemplified, for example, by the fasting blood glucose level or fasting plasma glucose level (FPG) and the blood glucose level based on the oral glucose tolerance test (hereinafter referred to as "OGTT" as well). FPG and OGTT as described above are used to diagnose and judge whether or not the disease is borderline diabetes.

On the other hand, glycohemoglobin (HbA1c) is known as an index to evaluate the metabolic result (result of metabolism) of glucose in the body fluid in a certain period of time. Glycohemoglobin is hemoglobin (red pigment protein in blood) bonded to glucose, which reflects the blood sugar state provided 2 to 3 months ago from the present time. Therefore, glycohemoglobin is the index which is useful for the diabetes care in order to grasp the life and the condition of the disease of the patient. At present, it is necessary that glycohemoglobin should be measured by using the measurement principle which is distinct from that for the blood sugar level. However, in recent years, a close correlation has been found out between the glycohemoglobin (HbA1c) and the average blood sugar level (AG: Average Glucose) which is the average value of blood sugar levels provided from the past to the present as measured by using the CGM measuring apparatus. It has been reported that the present glycohemoglobin can be estimated from the average blood sugar level, or the average blood sugar level can be estimated from the present glycohemoglobin (see, for example, Non-Patent Document 1).

Other than the above, a method has been suggested, for example, in Patent Document 1, in which the level of glycohemoglobin (HbA1c) is predicted or anticipated in the blood of a patient by using a glucose level measured beforehand. In this method, a mathematical model is previously derived for the behavior of the glycohemoglobin level with respect to the blood glucose level by using the blood glucose level and the glycohemoglobin level measured beforehand. When the glycohemoglobin level is newly measured, the mathematical model is updated. Subsequently, the glycohemoglobin level is predicted by using the updated mathematical model and the blood glucose level to be newly measured in future.

Patent Documents are as follows.

Patent Document 1: JP10-332704A;
Patent Document 2: JP4523082B1;

Patent Document 3: WO2007091654A;
Patent Document 4: JP2003-528330W;
Patent Document 5: JP2005-535885W;
Patent Document 6: JP2009-210549A.

Non-Patent Documents are as follows.

Non-Patent Document 1: Diabetes Care Volume 31 Number 8 August 2008, "Translating the A1c assay into Estimated Average Glucose Values", David M. Nathan (ADAG Study Group);
Non-Patent Document 2: Diabetes Care Volume 33 Number 8 August 2010, "2010 Consensus Statement on the Worldwide Standardization of the Hemoglobin A1c Measurement";
Non-Patent Document 3: Diabetes Care Volume 33 Number 9 September 2010, "Postchallenge Glucose, A1c, and Fasting Glucose as Predictors of Type 2 Diabetes and Cardiovascular Diseases";
Non-Patent Document 4: Diabetes Care Volume 33 Number 10 October 2010, "Impact of A1c Screening Criterion on the Diagnosis of Pre-Diabetes Among U.S. Adults";
Non-Patent Document 5: The Lancet, 25 June 2011 (Early Online Publication), "HbA1c 5.7-6.4% and impaired fasting plasma glucose for diagnosis of prediabetes and risk of progression to diabetes in Japan (TOPICS 3): a longitudinal cohort study".

US 2010/162786 A1 discloses systems, methods and/or devices for calibrating sensor data to be used in estimating a blood glucose concentration. A relationship between sensor measurements and reference readings may be used to estimate a relationship between sensor measurements and blood glucose concentration. Such sensor measurements may be weighted according to a decreasing function of uncertainty associated with sensor values.

US 2010/330598 A1 discloses a method and system for providing both an estimated true mean blood glucose value and estimated glycated hemoglobin (HbA1C) value from spot blood glucose (bG) measurements are disclosed. The bG measurements and associated context of the bG measurement are collected at daily times specified by a structured sampling schema, and the collected bG measurements are weighted based on the associated context. The estimated true mean bG value and the estimate HbAlC value are then determined from the weighted measurements of the collected bG measurements. A computer program for implementing the method for providing both an estimated true mean blood glucose value and estimated glycated hemoglobin (HbA1C) value from spot blood glucose (bG) measurements is also disclosed.

Translating the A1C Assay into Estimated Average Glucose Values; David M Nathan, MD; discloses a mathematical relationship between A1C and average glucose (AG) levels and determines whether A1C could be expressed and reported as AG in the same units as used in self-monitoring. Approximately 2,700 glucose values were obtained by each subject during 3 months. Linear regression analysis between the A1C and AG values provided the tightest correlations ($AG_{mg/dl}$ = 28.7 x A1C - 46.7, $R^2$ = 0.84, P < 0.0001), allowing calculation of an estimated average glucose (eAG) for A1C values. The linear regression equations did not differ significantly across subgroups based on age, sex, diabetes type, race/ethnicity, or smoking status.

SUMMARY OF THE INVENTION

In this context, it is required that a first index value is obtained accurately on the basis of a first biological component in such a user terminal that the first index value, which reflects the state of the first biological component and which is provided to evaluate the metabolic result thereof, is acquired on the basis of a measured value of the first biological component of a user, and the acquired result of the first index value is provided for the user. The present invention has been made taking the foregoing problem into consideration, an object of which is to provide such a technique that a user terminal can accurately acquire a first index value on the basis of a first biological component in relation to an information processing apparatus capable of communicating with the user terminal for providing, to a user, the first index value which reflects the state of the first biological component of the user and which is provided to evaluate the metabolic result thereof.

The present invention provides an information processing apparatus capable of communicating with a user terminal which is configured to acquire a HbA1c concentration for reflecting a state of an average glucose concentration of a user in a certain period of time, and to provide the acquired HbA1c concentration to the user: the information processing apparatus comprising: a storage device which is configured to store common calibration curve data in which a correlation between the average glucose concentration and the HbA1c concentration averaged for multiple users is reflected, and inherent calibration curve data in which a correlation inherent in the user is reflected in relation to the average glucose concentration and the HbA1c concentration, wherein: the information processing apparatus is configured to: deliver the inherent calibration curve data to the user terminal for acquiring the HbA1c concentration; acquire a measured value of the average glucose concentration of the user; acquired measured value of the average glucose concentration to the common calibration curve data and the inherent calibration curve data, respectively, to obtain a common calibration curve corresponding value and an inherent calibration curve corresponding value of the HbA1c concentration; determine whether the amount of observed deviation between the common calibration curve corresponding value and the inherent calibration curve corresponding value exceeds a predetermined first reference value; transmit measured value request information to request the actual measurement of the HbA1c concentration if the amount of the observed deviation exceeds the first reference value; update the inherent calibration curve data when the observed deviation exceeds the first reference value by using both the measured concentration of HbA1c and the measured value of the average glucose concentration; and transmit the updated inherent calibration curve data to the user terminal.

The present invention also provides an information processing apparatus capable of communicating with a user terminal which is configured to acquire a HbA1c concentration for reflecting a state of an average glucose concentration of a user in a certain period of time , and to provide the acquired HbA1c concentration to the user: the information processing apparatus comprising: a storage device which is configured to store common calibration curve data in which a correlation between the average glucose concentration and the HbA1c concentration averaged for multiple users is reflected, and inherent calibration curve data in which a correlation inherent in the user is reflected in relation to the average glucose concentration and the HbA1c concentration, wherein: the information processing apparatus is configured to: deliver the inherent calibration curve data to the user terminal for acquiring the HbA1c concentration; acquire a measured value of the HbAlc concentration actually measured from a specimen of the user; acquired measured value of the HbA1c concentration to the common calibration curve data and the inherent calibration curve data, respectively, to obtain a common calibration curve corresponding value and an inherent calibration curve corresponding value of the average glucose concentration; determine whether the amount of observed deviation between the common calibration curve corresponding value and the inherent calibration curve corresponding value exceeds a predetermined first reference value; transmit measured value request information to request the actual measurement of the average glucose concentration if the amount of the observed deviation exceeds the first reference value; update the inherent calibration curve data when the observed deviation exceeds the first reference value by using both the measured concentration of HbA1c and the measured value of the average glucose concentration from the user terminal; and transmit the updated inherent calibration curve data to the user terminal.

In an example there is provided an information processing apparatus capable of communicating with a user terminal which acquires a first index value for reflecting a state of a first biological component of a user and evaluating a metabolic result of the first biological component in a certain period of time on the basis of the first biological component and which provides the acquired first index value to the user; wherein calibration curve data is delivered to the user terminal in order to use the calibration curve data for acquiring the first index value. According to the construction as described above, the calibration curve data, which is used by the user terminal when the first index value is acquired on the basis of the measured value of the first biological component, is delivered from the information processing apparatus to the user terminal. Therefore, the user terminal, which receives the delivery of the calibration curve data, can accurately acquire the first index value.

The information processing apparatus is preferably constructed such that the information processing apparatus further comprises a storage device which stores common calibration curve data and inherent calibration curve data in which a correlation inherent in the user is reflected in relation to the first biological component and the first index value; wherein any one of a measured value of the first index value actually measured from a specimen of the user and a measured value of the first biological component of the user is acquired as a first measured value; the first measured value is substituted for the common calibration curve data and the inherent calibration curve data respectively to acquire corresponding values respectively corresponding to an unacquired measured value which is not acquired as the first measured value and which is any one of the measured value of the first index value and the measured value of the first biological component; it is judged whether or not a deviation amount between the respective corresponding values acquired from the respective pieces of calibration curve data exceeds a predetermined first reference value; measured value request information is transmitted to request the unacquired measured value if it is judged that the deviation amount exceeds the first reference value; the unacquired measured value, which is acquired after the transmission of the measured value request information, is designated as a second measured value to update the inherent calibration curve data on the basis of the first measured value and the second measured value; and the inherent calibration curve data after the update is transmitted to the user terminal.

When the information processing apparatus acquires the measured value of the first biological component as the first measured value, the acquired first measured value is substituted for the common calibration curve data stored in the storage device and the inherent calibration curve data inherent in the user respectively to determine the corresponding values of the first index value (unacquired measured value) corresponding to the first measured value respectively. On the other hand, when the information processing apparatus acquires the measured value of the first index value as the first measured value, the acquired first index value is substituted for the common calibration curve data and the inherent calibration curve data respectively to determine the values of the first biological component (unacquired measured value) corresponding thereto respectively. The larger deviation amount, which is provided between the corresponding values acquired from the two types of the calibration curve data as described above, means the fact that the large difference exists between the calibration curve stored in the inherent calibration curve data and the calibration curve stored in the common calibration curve data.

In view of the above, in the information processing apparatus according to the example, if the deviation amount exceeds the first reference value, for example, the measured value request information is transmitted to the user terminal. In this case, if the user terminal is separated from a measuring device (measuring instrument) for measuring the first biological component, the measured value request information may be transmitted to the measuring device. In this case, if the deviation amount between the respective corresponding values exceeds the first reference value, it is judged that the significant difference is found or acknowledged for the respective values and the significant difference is caused, for example, by the change of the physical constitution of the user. The measured value request information is the information having the contents in which the measured value (unacquired measured value) of the unacquired biological component is requested. When the information processing apparatus acquires the first biological component as the first measured value, it is requested to measure the first index value in the measured value request information. On the other hand, when the information processing apparatus acquires the first index value as the first measured value, the measured value of the first biological component is requested in the measured value request information.

When the measured value request information is transmitted, for example, to the user terminal, the user terminal informs the user of the information of the contents to request the measurement of the first index value as the unacquired measured value, for example, in the former case. The user, who receives the information, actually measures the first index value, for example, by utilizing a measuring apparatus. The information processing apparatus acquires, as the second measured value, the measured value of the first index value measured as described above. On the other hand, in the latter case, for example, the user terminal acquires the measured value of the first biological component from the measuring device for measuring the first biological component, and the measured value is transmitted to the information processing apparatus.

The second measured value, which is newly acquired by the information processing apparatus, forms one set (pair) of actual measurement data in relation to the first biological component and the first index value prescribed in the inherent calibration curve data concerning the corresponding user together with the first measured value having been already acquired. Accordingly, in the information processing apparatus, the inherent calibration curve data, which corresponds to the user who uses the user terminal, is updated by using the first measured value and the second measured value. Accordingly, for example, the correlation between the first biological component and the first index value, which is prescribed in the inherent calibration curve data, approaches the similar correlation prescribed in the common calibration curve data. The inherent calibration curve data, which is updated as described above, is transmitted to the user terminal of the corresponding user. Therefore, for example, the inherent calibration curve data can be updated by using the newly received data on the side of the user terminal. Accordingly, the user terminal can determine the first index value from the measured value of the first biological component by using the latest inherent calibration curve data. Further, according to the information processing apparatus, it is possible to judge whether or not the inherent calibration curve data, which is inherent in the user, is in an appropriate state which is to be originally provided. It is possible to update the inherent calibration curve data, if necessary.

In the information processing apparatus according to the example, it is preferable that correction request information for the first biological component is transmitted if the measured value of the first biological component is acquired as the first measured value and it is judged that the deviation amount exceeds a predetermined second reference value larger than the first reference value. The correction request information for the first biological component is the instruction information having such contents that it is requested to transmit the data of the first biological component to which the correcting process is applied, for example, with respect to the user terminal or the measuring device for the first biological component again. The user terminal, which receives the correction request information, applies the following correcting process to the measurement data of the first biological component, and a corrected measured value, which is obtained as a result thereof, is transmitted to the information processing apparatus. In this case, the information processing apparatus is preferably constructed such that the inherent calibration curve data is updated on the basis of the second measured value and the corrected measured value of the first biological component acquired after the transmission of the correction request information.

It is preferable that the measured value of the first biological component is an average value of a plurality of pieces of measurement data acquired by continuously measuring the first biological component in a predetermined reference period. For example, when the first biological component is glucose, it is preferable that the average glucose concentration, which is determined by averaging a plurality of pieces of measurement data concerning the glucose concentration measured in a certain reference period, is adopted as the measured value of the first biological component. It is also preferable that the first index value is a glycohemoglobin concentration.

In this context, when the measured value of the first biological component is the average value of the plurality of pieces of measurement data acquired by continuously measuring the first biological component in the predetermined reference period, it is preferable that the corrected measured value of the first biological component is calculated as an average value of a plurality of pieces of measurement data concerning the first biological component acquired by performing a reference period shortening process for shortening the reference period, or an average value of a plurality of pieces of measurement data concerning the first biological component acquired by performing an abnormal value removing process for removing the measurement data in which a predetermined measured value change range exceeds an allowable value in the reference period.

The information processing apparatus according to the example is preferably constructed such that it is judged that any abnormality or malfunction arises in a measuring apparatus for actually measuring the first index value from the specimen of the user if the measured value of the first index value is acquired as the first measured value and it is judged that the deviation amount exceeds a predetermined second reference value larger than the first reference value.

It is preferable that the common calibration curve data is constructed on the basis of a correlation between measured values of the first biological component measured from specimens of a plurality of examinees and measured values of the first index value measured from the specimens. It is also preferable that the information processing apparatus is incorporated into a measuring apparatus for measuring the first index value from the specimen of the user. It is of course preferable that the information processing apparatus is realized in a form of a server apparatus provided independently from the measuring apparatus. The user terminal may be provided integrally with a measuring device or instrument for measuring the first biological component from the specimen of the user, or the user terminal may be separated therefrom. In the case of the latter, the user terminal and the measuring device are capable of communicating with each other in a wired or wireless manner.

In another example a user terminal is provided which is capable of communicating with any one of the information processing apparatuses as defined above. The user terminal is preferably capable of displaying, for example, the information in relation to the measured value of the first biological component and the result of acquisition of the first index value acquired on the basis of the measured value of the first biological component and the inherent calibration curve data delivered from the information processing apparatus.

According to some examples, there is provided a user terminal which acquires a first index value for reflecting a state of a first biological component of a user and evaluating a metabolic result of the first biological component in a certain period of time on the basis of the first biological component and which provides the acquired first index value to the user. The user terminal is preferably constructed such that calibration curve data, which is provided to be used for acquiring the first index value, is updated under a predetermined condition.

For example, the user terminal is preferably constructed such that the user terminal further comprises a storage device which stores common calibration curve data and inherent calibration curve data in which a correlation inherent in the user is reflected in relation to the first biological component and the first index value; wherein any one of a measured value of the first index value actually measured from a specimen of the user and a measured value of the first biological component of the user is acquired as a first measured value; the first measured value is substituted for the common calibration curve data and the inherent calibration curve data respectively to acquire corresponding values respectively corresponding to an unacquired measured value which is not acquired as the first measured value and which is any one of the measured value of the first index value and the measured value of the first biological component; it is judged whether or not a deviation amount between the respective corresponding values acquired from the respective pieces of calibration curve data exceeds a predetermined first reference value; and the unacquired measured value is newly acquired as a second measured value and the inherent calibration curve data is updated on the basis of the newly acquired second measured value and the first measured value, if it is judged that the deviation amount exceeds the first reference value.

The user terminal is preferably constructed such that a correcting process is carried out in relation to the measured value of the first biological component if the measured value of the first biological component is acquired as the first measured value and it is judged that the deviation amount exceeds a predetermined second reference value larger than the first reference value; and the inherent calibration curve data is updated on the basis of the second measured value and a corrected measured value of the first biological component after carrying out the correcting process.

The user terminal is preferably constructed such that it is judged that any abnormality or malfunction arises in a measuring apparatus for actually measuring the first index value from the specimen of the user if the measured value of the first index value is acquired as the first measured value and it is judged that the deviation amount exceeds a predetermined second reference value larger than the first reference value.

The user terminal is preferably constructed such that the user terminal acquires a second index value in order to evaluate a metabolic ability for metabolizing the first biological component. In this case, the user terminal is preferably constructed such that an acquisition result, which relates to the first index value and the second index value, can be displayed on a display. In this case, the display may simultaneously display the first index value and the second index value on the display.

It is preferable that the user terminal further comprises a storage device which stores common calibration curve data and inherent calibration curve data in which a correlation inherent in the user is reflected in relation to the first biological component and the first index value; and a judging unit which judges a magnitude correlation with respect to a predetermined threshold value set for each of the index values in relation to the acquired first index value and the acquired second index value respectively. The user terminal is preferably constructed such that the measured value of the first biological component is substituted for the common calibration curve data and the inherent calibration curve data respectively to acquire an inherent calibration curve corresponding value and a common calibration curve corresponding value as the respective first index values corresponding to the substituted measured value of the first biological component; the magnitude correlation with respect to the predetermined threshold value set for the first index value is judged by the judging unit in relation to the acquired inherent calibration curve corresponding value and the acquired common calibration curve corresponding value respectively; and predetermined update request information, with which it is requested to update the inherent calibration curve data, is outputted if different judgment results are obtained for the inherent calibration curve corresponding value and the common calibration curve corresponding value. The update request information includes, for example, predetermined actual measurement request information with which the user is requested to actually measure the first index value.

In this context, the storage device of the user terminal is preferably constructed such that the storage device stores the actually measured value of the first index value acquired by using at least any means of an input operation performed by the user, a transportable recording medium, and communication from an external apparatus. It is preferable that the inherent calibration curve data in the storage device is updated on the basis of an added first index value, if a new actually measured value of the first index value is added to the storage device.

In still another example,
there is provided a method for providing information. It is possible to provide a method for providing information for an information processing apparatus capable of communicating with a user terminal which acquires a first index value for reflecting a state of a first biological component of a user and evaluating a metabolic result of the first biological component in a certain period of time on the basis of the first biological component and which provides the acquired first index value to the user; wherein calibration curve data is delivered by the information processing apparatus to the user terminal in order to use the calibration curve data for acquiring the first index value.

In this context, when a storage device of the information processing apparatus stores common calibration curve data and inherent calibration curve data in which a correlation inherent in the user is reflected in relation to the first biological component and the first index value; it is preferable that the method for providing information according to the present invention comprises a first step of acquiring any one of a measured value of the first index value actually measured from a specimen of the user and a measured value of the first biological component of the user as a first measured value; a second step of substituting the first measured value for the common calibration curve data and the inherent calibration curve data respectively to acquire corresponding values respectively corresponding to an unacquired measured value which is not acquired as the first measured value and which is any one of the measured value of the first index value and the measured value of the first biological component; a third step of judging whether or not a deviation amount between the respective corresponding values acquired from the respective pieces of calibration curve data exceeds a predetermined first reference value; a fourth step of transmitting measured value request information to request the unacquired measured value if it is judged that the deviation amount exceeds the first reference value; a fifth step of designating the unacquired measured value which is acquired after the transmission of the measured value request information as a second measured value to update the inherent calibration curve data on the basis of the first measured value and the second measured value; and a sixth step of transmitting the inherent calibration curve data after the update to the user terminal.

It is preferable that the method for providing information further comprises a seventh step of transmitting correction request information for the first biological component if the measured value of the first biological component is acquired as the first measured value in the first step and it is judged in the third step that the deviation amount exceeds a predetermined second reference value larger than the first reference value; and an eighth step of updating the inherent calibration curve data on the basis of the second measured value and a corrected measured value of the first biological component acquired after the transmission of the correction request information.

In still another example, there is provided a display method for displaying a measured value of a first biological component of a user on a display of a user terminal, the display method comprising an index value acquiring step of acquiring a first index value for reflecting a state of the first biological component and evaluating a metabolic result of the first biological component in a certain period of time and a second index value for evaluating a metabolic ability for the first biological component; and a display step of simultaneously displaying, on the display, results of acquisition in relation to the first index value and the second index value acquired in the index value acquiring step.

It is preferable that the display method for the display according to the present invention further comprises a judging step of judging a magnitude correlation with respect to a predetermined threshold value set for each of the index values in relation to the first index value and the second index value respectively acquired in the index value acquiring step; wherein a display area of the display is divided into a plurality of areas in the display step in order to display judgment results of the judging step in relation to the first index value and the second index value respectively so that the judgment results can be specified; and a specified area, which is included in the display area and which conforms to the judgment result in relation to each of the index values, is in such a display state that the specified area is distinguishable from the other areas.

It is preferable that the display method for the display according to the present invention further comprises substituting the measured value of the first biological component for the common calibration curve data and the inherent calibration curve data in which a correlation inherent in the user is reflected in relation to the first biological component and the first index value respectively to acquire an inherent calibration curve corresponding value and a common calibration curve corresponding value as the respective first index values corresponding to the substituted measured value of the first biological component; judging a magnitude correlation with respect to a predetermined threshold value set for the first index value in relation to the acquired inherent calibration curve corresponding value and the acquired common calibration curve corresponding value respectively; and outputting predetermined update request information with which it is requested to update the inherent calibration curve data if different judgment results are obtained for the inherent calibration curve corresponding value and the common calibration curve corresponding value.

It is noted that the means for solving the problem according to the present invention can be combined with each other as far as possible.

According to the present invention, it is possible to provide such a technique that the user terminal can accurately acquire the first index value on the basis of the first biological component in relation to the information processing apparatus capable of communicating with the user terminal for providing, to the user, the first index value which reflects the state of the first biological component of the user and which is provided to evaluate the metabolic result thereof.

BRIEF DESCRIPTION OF THE DRAWINGS

Fig. 1 shows a system arrangement of an information providing system according to a first embodiment.
Fig. 2 shows a block diagram illustrating an arrangement of a user terminal according to the first embodiment.
Fig. 3 shows a block diagram illustrating an arrangement of a server apparatus according to the first embodiment.
Fig. 4 illustrates the inherent calibration curve data.
Fig. 5 exemplifies the time-dependent transition or change of the blood sugar level by way of example.
Fig. 6 illustrates the state of connection between the server apparatus and a plurality of user terminals according to the first embodiment.
Fig. 7 illustrates the common calibration curve data SCp.
Fig. 8 illustrates the first control according to the first embodiment.
Fig. 9 shows a flow chart illustrating the procedure of a first control routine according to the first embodiment.
Fig. 10 shows a flow chart illustrating the procedure of an inherent calibration curve data update control routine according to the first embodiment.
Fig. 11 illustrates an abnormal value removing process according to the first embodiment.
Fig. 12 illustrates the second control according to the first embodiment.
Fig. 13 shows a flow chart illustrating the procedure of a second control routine according to the first embodiment.
Fig. 14 shows a flow chart illustrating the procedure of a second inherent calibration curve data update control routine according to the first embodiment.
Fig. 15 shows an exemplary arrangement of another system in relation to the information providing system according to the first embodiment.
Fig. 16 illustrates the basic display mode of the user terminal according to the first embodiment.
Fig. 17 illustrates, in the second place, the basic display mode of the user terminal according to the first embodiment.
Fig. 18 illustrates the AG display mode of the user terminal according to the first embodiment.
Fig. 19 illustrates the analysis display mode (analysis basic screen) of the user terminal according to the first embodiment.
Fig. 20 illustrates the analysis display mode (detailed information screen) of the user terminal according to the first embodiment.
Fig. 21 illustrates the second analysis display mode of the user terminal according to the first embodiment.
Fig. 22 illustrates the notice display mode of the user terminal according to the first embodiment.
Fig. 23 shows a flow chart illustrating the procedure of an FPG measurement control routine according to the first embodiment.
Fig. 24 illustrates the FPG measuring mode (FPG initial screen) of the user terminal according to the first embodiment.
Fig. 25 illustrates the FPG measuring mode (FPG continuing screen) of the user terminal according to the first embodiment.
Fig. 26 illustrates the FPG measuring mode (FPG result output screen) of the user terminal according to the first embodiment.

Fig. 27 shows a flow chart illustrating the procedure of an OGTT measurement control routine according to the first embodiment.

Fig. 28 illustrates an OGTT measuring mode (OGTT initial screen) of the user terminal according to the first embodiment.

Fig. 29 illustrates the OGTT measuring mode (OGTT continuing screen) of the user terminal according to the first embodiment.

Fig. 30 illustrates the OGTT measuring mode (OGTT result output screen) of the user terminal according to the first embodiment.

Fig. 31 shows a system arrangement of an information providing system according to a second embodiment.

Fig. 32 shows a flow chart illustrating the procedure of a third control routine according to the second embodiment.

Fig. 33 shows a flow chart illustrating the procedure of a third inherent calibration curve data update control routine according to the second embodiment.

Fig. 34 shows a flow chart illustrating the procedure of a fourth control routine according to the second embodiment.

Fig. 35 illustrates a simultaneous display mode of a user terminal according to a third embodiment.

Fig. 36 shows a flow chart illustrating the process flow adopted when the display mode of the user terminal according to the third embodiment is the simultaneous display mode.

Fig. 37 illustrates, in the second place, the simultaneous display mode according to the third embodiment.

Fig. 38 shows a flow chart illustrating the process flow of a first calibration curve update routine according to the third embodiment.

Fig. 39 shows a flow chart illustrating the process flow of a second calibration curve update routine according to the third embodiment.

Fig. 40 shows a schematic arrangement of an SMBG measuring apparatus according to the third embodiment.

Fig. 41 shows a flow chart illustrating the process flow of a first calibration curve update routine according to a fourth embodiment.

Fig. 42 shows a flow chart illustrating the process flow of a second calibration curve update routine according to the fourth embodiment.

## DESCRIPTION OF THE PREFERRED EMBODIMENTS

Embodiments according to the present invention will be exemplarily explained below on the basis of the drawings. It is not intended that the technical scope of the invention is limited, for example, to only the size, the material, the shape, and the relative arrangement of the constitutive elements described in the embodiments of the present invention, unless otherwise specifically noted. The constitutive elements, which are common to the respective drawings, are designated by the same reference numerals.

### First Embodiment

### [Schematic arrangement of system]

Fig. 1 shows a system arrangement of an information providing system S according to a first embodiment. Reference numeral 1 indicates a CGM measuring device (instrument), 2 indicates a user terminal, 3 indicates a network, 4 indicates a server apparatus (information processing apparatus), and 5 indicates a measuring apparatus. The CGM measuring device 1 and the user terminal 2 cooperate to continuously measure (actually measure) the concentration of glucose (grape sugar) contained in the intercellular fluid of a user (patient), i.e., the blood sugar level (blood glucose level). In this embodiment, the CGM measuring device 1 and the user terminal 2 are constructed as distinct devices or instruments. However, it is also allowable that they are integrally constructed.

### [User side]

#### <CGM measuring device>

The CGM measuring device 1 is provided with a glucose sensor 11 which is used while being implanted subcutaneously beneath the skin of a user, and a control computer (not shown) which is equipped with electronic parts including, for example, a processor and a memory required for the predetermined operation of the CGM measuring device 1 (for example, application of the voltage and communication with the outside). The CGM measuring device 1 can be equipped with the user by sticking the CGM measuring device 1 to the skin of the user by means of, for example, an adhesive tape or attaching the CGM measuring device 1, for example, to a belt. In this embodiment, the glucose component in the body fluid corresponds to the first biological component of the present invention. The glucose sensor 11 is an electrochemical sensor which detects the specified component by utilizing the electrochemical reaction. A sensor unit, which is composed of glucose oxidoreductase such as glucose oxidase (GOD), glucose dehydrogenase (GDH) or the like and a plurality of electrodes such as a working electrode, a counter electrode, a reference electrode and the like, is formed on the forward end side of the glucose sensor 11. As for the glucose sensor 11, the sensor unit is implanted subcutaneously, and the applied voltage applied to the electrode is controlled by the control computer. Accordingly, the continuous blood sugar measurement or continuous glucose monitoring (CGM) is carried out.

<User terminal>

Fig. 2 shows a block diagram illustrating an arrangement of the user terminal 2 according to the first embodiment. The user terminal 2 is an information processing apparatus which is disposed on the side of the user (client) and which has a display in order to display the measurement result of the CGM measuring device 1. The user terminal 2 includes the construction of a computer as explained below. That is, the user terminal 2 is provided with, for example, a processor 21, a main storage device 22, an operation unit 23, a display 24, a transportable recording medium driving device 25, an network interface 26, an auxiliary storage device 27, and a clock 28, and these components are connected to one another by means of a bus line.

The operation unit 23 is composed of, for example, operation buttons. However, the operation unit 23 may be, for example, a contact type touch panel. The data, which is inputted from the operation unit 23, is outputted to the processor 21. The network interface 26 is an interface which performs the input/output of the information with respect to the network 3. The network 3 is a communication line network which appropriately includes, for example, the internet, the telephone line network, and the satellite circuit network. The network interface 26 is provided to make connection with the wired or wireless network, which resides in, for example, the wireless circuit to make connection with the mobile phone network, wireless LAN (Local Area Network) card, and/or NIC (Network Interface Card). For example, the data, which is received by the network interface 26, is outputted to the processor 21.

The main storage device 22 provides the storage area and the working area for loading the program stored in the auxiliary storage device 27, and the main storage device 22 is used as a buffer. The main storage device 22 is, for example, a semiconductor memory such as RAM (Random Access Memory) or the like. However, there is no limitation thereto. The auxiliary storage device 27 stores various programs and the data to be used by the processor 21 when the respective programs are executed. The auxiliary storage device 27 is, for example, EPROM (Erasable Programmable ROM), a hard disk (Hard Drive Disc), or a flash memory. However, there is no limitation thereto.

The auxiliary storage device 27 has, for example, a measurement data storage unit 30, a first calibration curve storage unit 31, a second calibration curve storage unit 32, an identification information storage unit 33, an event history storage unit 34, an AG value storage unit 35, and an HbAlc value storage unit 36. The transportable recording medium driving device 25 drives the transportable recording medium such as a flash memory card or the like to read the data recorded on the transportable recording medium. The read data is outputted to the processor 21. The processor 21 is, for example, CPU (Central Processing Unit) or DSP (Digital Signal Processor). The processor 21 executes various processes by loading OS and various application programs retained in the auxiliary storage device 27 to the main storage device 22 and executing the same.

The display 24 has a first display 24A and a second display 24B. Each of the displays is, for example, a liquid crystal display device, a plasma display panel, a cathode ray tube (CRT), or an electroluminescence panel. The user terminal 2 and CGM measuring device 1 can perform data communication in a wireless or wired manner. The data communication may be performed by utilizing, for example, any wireless communication means (for example, IrDA based on the use of the infrared ray or Bluetooth based on the use of the frequency band of 2.4 GHz), or the data communication may be performed by the aid of a cable of USB (Universal Serial Bus) or the like.

[Medical facility side]

<Server apparatus>

The server apparatus 4 is a general purpose host computer which is provided on the medical facility side. The server apparatus 4 is connected so that the communication can be performed with the user terminal 2 by the aid of the network 3. Fig. 3 shows a block diagram illustrating an arrangement of the server apparatus 4 as the information processing apparatus according to the first embodiment. The server apparatus 4 is provided with, for example, a processor 41, a main storage device 42, an input device 43, an output device (monitor) 44, a transportable recording medium driving device 45, a network interface 46, an auxiliary storage device 47, and a clock 48, and these components are connected to one another by means of a bus line. In this embodiment, the information processing apparatus according to the present invention is realized by the server apparatus 4 which is provided independently from the measuring apparatus 5. However, the information processing apparatus may be incorporated into the measuring apparatus 5. In other words, the server apparatus 4 shown in Fig. 3 may be incorporated into the measuring apparatus 5 to thereby realize an apparatus which is provided with the server function and the measuring function to actually measure glycohemoglobin as described later on. An exemplary arrangement of the latter will be explained in detail later on as another embodiment.

The input device 43 is, for example, a keyboard and/or a pointing device such as a mouse or the like. The data, which is inputted from the input device 43, is outputted to the processor 41. The network interface 46 is an interface which performs the input/output of the information with respect to the network 3. The network interface 46 is equivalent to the network interface 26 of the user terminal 2. For example, the data, which is received by the network interface 46, is outputted to the processor 41.

The main storage device 42 provides the storage area and the working area for loading the program stored in the auxiliary storage device 47, and the main storage device 42 is used as a buffer. The main storage device 42 is, for example, a semiconductor memory such as RAM or the like. On the other hand, the processor 41 is, for example, CPU or DSP, which is equivalent to the processor 21 of the user terminal 2. The auxiliary storage device 47 stores, for example, various programs and the data to be used by the processor 41 when the respective programs are executed. The auxiliary storage device 47 is, for example, EPROM or a hard disk. The auxiliary storage device 47 retains, for example, the operating system (OS) and other various application programs. The auxiliary storage device 47 has, for example, a server side calibration curve storage unit 52 (second calibration curve storage unit), an AG value storage unit 53, and an HbAlc value storage unit 54. The transportable recording medium driving device 45 drives the transportable recording medium to read the data recorded on the transportable recording medium. The read data is outputted to the processor 41. The transportable recording medium is a recording medium which includes, for example, a USB (Universal Serial Bus) flash memory, a flash memory card, CD (Compact Disc), and DVD (Digital Versatile Disc).

The output device 44 is a monitor which is provided to output, for example, the processing result of the processor 41. The server apparatus 4 is capable of performing data communication with the measuring apparatus 5 in a wireless or wired manner. The data communication may be performed by utilizing, for example, any wireless communication means (for example, IrDA based on the use of the infrared ray or Bluetooth based on the use of the frequency band of 2.4 GHz), or the data communication may be performed by the aid of a cable of USB (Universal Serial Bus) or the like.

<Measuring apparatus>

The measuring apparatus 5 is provided, for example, in the medical facility (for example, a hospital or a clinic). The measuring apparatus 5 is an apparatus which measures (actually measures) the concentration of glycohemoglobin (HbA1c) contained in the blood (specimen) of a patient. As for the measuring apparatus 5, it is possible to equivalently employ, for example, a high performance liquid chromatography apparatus (HPLC apparatus) which utilizes the high performance liquid chromatography (HPLC) and which is disclosed, for example, in Patent Document 3. The measuring apparatus 5 is provided with, for example, a measuring unit (not shown) which measures the concentration of glycohemoglobin (HbA1c) (hereinafter referred to as "HbA1c concentration") from the blood of the examinee (patient) by means of an optical technique, and a control computer (not shown) which is equipped with electronic parts such as a processor, a memory and the like, the processor being provided to control the operation of the entire measuring apparatus including, for example, the measuring process in relation to HbA1c, various calculation processes, and storage of measurement results. Further, the measuring apparatus 5 is provided with various devices required to measure the glycohemoglobin concentration, including, for example, a blood collecting tube in which the blood as a sample is accommodated, a sample preparing unit which is provided to prepare the sample collected from the blood collecting tube, and an optical measuring unit.

The measurement result of the glycohemoglobin concentration, which is obtained by the measuring apparatus 5, is inputted into the server apparatus 4 in a wired or wireless manner by the aid of the transportable recording medium including, for example, a USB (Universal Serial Bus) flash memory, a flash memory card, CD (Compact Disc), and DVD (Digital Versatile Disc). Various pieces of data, which are acquired by the server apparatus 4, are stored in the auxiliary storage device 47. When various pieces of data are sent from the measuring apparatus 5 by means of the communication, the data is received by the network interface 46 of the server apparatus 4. When various pieces of data are recorded on the transportable recording medium, the data is read from the transportable recording medium by the transportable recording medium driving device 45. Various pieces of data, which are acquired from the measuring apparatus 5 as described above, are stored in the auxiliary storage device 47.

[Display mode of user terminal]

As for the CGM measuring device 1, for example, the glucose sensor 11 is retained subcutaneously over a continuous measurement period ranging from about several days to several weeks to continuously measure the concentration of glucose (hereinafter simply referred to as "glucose concentration") in the body fluid (in the blood or the intercellular fluid). The term "continuous" means the fact that the glucose concentration is measured continuously in the state in which the glucose sensor 11 is retained subcutaneously. It goes without saying that a mode, in which the glucose concentration is measured every predetermined period of time, is included therein. The measurement frequency of the glucose concentration can be arbitrarily set. However, in this embodiment, the setting is made such that the glucose concentration is measured at a frequency, i.e., once a period of several tens seconds to several minutes. Glucose in the body fluid corresponds to the first biological component.

The user terminal 2 has the first display 24A and the second display 24B. The information (hereinafter referred to as "Glu information" as well), which relates to the glucose concentration measured by the CGM measuring device 1, is displayed on the first display 24A (see Fig. 1). The Glu information, which is displayed on the first display 24A, is set so that the display mode thereof is changed, if the manual operation is accepted from the user by the aid of the operation unit 23 or if the preset conditions are completed. The number of display or displays of the user terminal 2 is not limited to any specified number. It is also allowable to adopt such a mode that a single screen is divided into a plurality of display areas. Not only the present glucose concentration but also the average glucose concentration (AG: Average Glucose concentration) which is the average value of the glucose concentrations in a certain reference period may be displayed on the first display 24A. The variation, which relates to the display mode of the display 24, will be explained in detail later on.

For example, in the case of the display mode shown in Fig. 1, the present glucose concentration is displayed on the upper part of the first display 24A, and the graph, which indicates the transition or change of the glucose concentration, is displayed on the lower part. As for the present glucose concentration, the glucose concentration measured most recently at a point in time closest to the present time, i.e., the latest glucose concentration is displayed on the first display 24A.

Next, the basic operation of the CGM measuring device 1 will be explained. In the CGM measuring device 1, the voltage, which is applied between the electrodes for forming the sensor unit of the glucose sensor 11 (between the working electrode and the counter electrode or between the working electrode and the reference electrode), is controlled to measure the response current value thereof. When the voltage is applied between the working electrode and the counter electrode or between the working electrode and the reference electrode, then the grape sugar contained in the body fluid of the patient is oxidized by oxidoreductase, and the electrons, which are taken out thereby, are supplied to the working electrode. The control computer (processor) of the CGM measuring device 1 measures, as the response current, the electric charge amount of the electrons supplied to the working electrode.

The CGM measuring device 1, which has measured the response current value correlated with the concentration of glucose contained in the body fluid of the user (patient), successively performs the data transmission of the response current value to the user terminal 2. The processor 21 of the user terminal 2 converts the transmitted response current value into the glucose concentration with reference to the first calibration curve data stored in the first calibration curve storage unit 31 of the auxiliary storage device 27. The first calibration curve data is the data in which the calibration curve (standard curve) to indicate the correlation (corresponding relationship) between the response current value measured by the glucose sensor 11 and the glucose concentration in the body fluid is stored. The first calibration curve data is stored, for example, as a numerical expression or a corresponding table in the first calibration curve storage unit 31.

The processor 21 displays the present value of the glucose concentration calculated as described above on the upper part of the first display 24A. Further, the processor 21 acquires the time and date information from the clock 28. The glucose concentration (measured value), which is correlated with the time and date information, is stored in the measured value storage unit 30 of the auxiliary storage device 27. Further, the processor 21 displays the time-dependent transition or change of the glucose concentration on the lower part of the first display 24A on the basis of the time and date information (time and date of the measurement) and the glucose concentration stored in the auxiliary storage device 27. The time axis, with which the time-dependent transition or change of the glucose concentration is displayed on the first display 24A, can be determined arbitrarily. For example, in the example shown in Fig. 1, the time-dependent transition or change of the glucose concentration is displayed in a range ranging from the past several hours to the present time. The display mode of the display 24 can be appropriately changed by accepting the manual operation of the operation unit 23 performed by the user.

The Glu information, which is displayed on the first display 24A, is appropriate to provide the information about the relatively short term diabetes care to the user, while the Glu information is not appropriate to provide the information about the long term diabetes care. Accordingly, in the user terminal 2 according to this embodiment, the information (hereinafter referred to as "HbA1c information"), which relates to glycohemoglobin (HbA1c) as the index to reflect the long term blood sugar state, is displayed on the display 24 together with the Glu information.

Glycohemoglobin is hemoglobin bonded to grape sugar (glucose), which is the index to reflect the blood sugar state, in particular, the metabolic result of grape sugar in the past 2 to 3 months on the basis of the present time. In the user terminal 2, the Glu information is displayed on the first display 24A, and the HbA1c information is simultaneously displayed on the second display 24A. Accordingly, the information about the short term diabetes care and the information about the long term diabetes care are simultaneously provided for the user. In order that the user grasps the HbAlc concentration, it is necessary to perform the measurement by using the measuring space 5 shown in Fig. 1. Conventionally, it has been difficult to conveniently provide the HbAlc concentration to the user. However, in recent years, as described in Non-Patent Document 1 described above, for example, the correlation between the average glucose concentration AG and the HbA1c concentration is progressively clarified. In this embodiment, the HbA1c concentration corresponds to the first index value. The HbA1c concentration can be used as the index value in order to evaluate the result of the metabolism of glucose in a certain period of time.

In this embodiment, the processor 21 of the user terminal 2 calculates the average glucose concentration AG by averaging the glucose concentrations acquired for a certain period of time ranging from the present time to the past by means of the CGM measuring device 1. The HbAlc concentration, which correlates with the average glucose concentration AG, is estimated on the basis of the average glucose concentration AG. The result of estimation is displayed as the HbA1c information on the second display 24B.

<Inherent calibration curve data SCu>

Fig. 4 illustrates the contents of the inherent calibration curve data SCu. The inherent calibration curve data SCu is such calibration curve data that the correlation (corresponding relationship) between the average glucose concentration AG and the HbA1c concentration inherent in the user (specified user) is prescribed or defined (reflected). The inherent calibration curve data SCu is stored in the second calibration curve storage unit 32 of the auxiliary storage device 27 of the user terminal 2. A broken line shown in the drawing indicates the correlation between the average glucose concentration AG and the HbA1c concentration inherent in the user who uses the user terminal 2. Each of the plots (quadrangles) in the drawing is provided such that each of the actually measured values of the HbAlc concentration (hereinafter referred to as "actually measured HbA1c concentrations") is plotted as one set of pair data (hereinafter referred to as "actually measured pair data") together with each of the average glucose concentrations AG corresponding thereto when the user actually measures the HbA1c concentration by means of the measuring device 5. It is preferable that at least two or more pieces of the actually measured pair data are included in the inherent calibration curve data SCu. The inherent calibration curve data SCu of the user is constructed by successively incorporating the actually measured pair data inherent in the user into the standard model equation described later on.

The standard model equation resides in the standard model to represent the standard correlation between the average glucose concentration AG and the HbA1c concentration, for which it is possible to appropriately adopt various known models. The standard model equation may be, for example, either a linear equation or a nonlinear equation. In this case, the following numerical expression (1), which is disclosed in Non-Patent Document 1, is exemplarily applied as the standard model equation by way of example. The following numerical expression (2) may be derived by deforming the numerical expression (1), which may be applied as the standard model equation.

$$\text{Average glucose concentration AG (mg/dL)} = 28.7 * \text{HbA1c (\%)} - 46.7 \quad \ldots(1)$$

$$\text{HbA1c concentration (\%)} = 0.0348 * \text{AG (mg/dL)} + 1.63 \quad \ldots(2)$$

The process, which relates to the calculation of the average glucose concentration AG and the estimation of the HbA1c concentration, is as follows. The processor 21 of the user terminal 2 calculates the average glucose concentration AG by averaging the glucose concentrations which are acquired in a certain period of time ranging from the present time to the past (hereinafter referred to as "AG calculation reference period") and which are included in the data of the glucose concentrations stored in the measured value storage unit 30. The AG calculation reference period is the period of time which serves as the reference when the average glucose concentration AG is calculated. The AG calculation reference period is not limited to any specified period of time, which can be appropriately changed. In this embodiment, the AG calculation reference period is set within a range of about several days to 3 months. For example, Fig. 5 shows the transition of the glucose concentration in past 120 hours (5 days), which shows such an example that the average glucose concentration AG in this period is 189 mg/dL. The average glucose concentration AG, which is calculated by the processor 21, is correlated with the time and date information concerning the AG calculation reference period. After that, the average glucose concentration AG is stored in the AG value storage unit 35 of the auxiliary storage device 27. Alternatively, the average glucose concentration AG, which is calculated by the user terminal 2, may be transmitted to the server apparatus 4 via the network 3, and the average glucose concentration AG may be stored in the AG value storage unit 53 of the auxiliary storage device 47.

When the HbA1c concentration is estimated, the processor 21 of the user terminal 2 reads the average glucose concentration AG stored in the AG value storage unit 35 of the auxiliary storage device 27. The processor 21 accesses the inherent calibration curve data SCu in the second calibration curve storage unit 32, and thus it is possible to obtain the estimated value of the HbAlc concentration (hereinafter referred to as "estimated HbA1c concentration").

The estimated HbA1c concentration determined as described above is displayed on the second display 24B, for example, as shown in Fig. 1. The estimated HbAlc concentration is the index which indicates the blood sugar state of the user to be provided about 2 to 3 months later. It is affirmed that the estimated HbAlc concentration is the index which reflects the metabolic result of glucose in a certain period of time. In the case of the information providing system S of this embodiment, it is possible to simultaneously display, on the user device 2, the present glucose concentration (blood sugar level) Glu, the time-dependent transition or change of the glucose concentration in a certain period of time, and the HbA1c information to represent the blood sugar state in future. Accordingly, it is easy for the user to intuitively grasp the relative relationship between the present blood sugar state and the blood sugar state in future as well. It is possible to provide the information useful to manage the blood sugar state of himself/herself to the user. In this embodiment, the HbA1c concentration, which is to be provided about 2 to 3 months later from the present time, is estimated by using the same correlation in relation to the average glucose concentration AG and the HbA1c concentration, when it is assumed that the recent average glucose concentration (for example, approximately from the past several weeks to the present time) will continue in future.

As shown in Fig. 6, the server apparatus 4, which is provided for the medical facility side, is connected so that the communication can be performed with a large number of user terminals 2 via the network 3. It is noted that only one user terminal 2 is shown in Fig. 1 for the convenience of drawing. Actually, a plurality of user terminals 2 are connected to the server apparatus 4 via the network 3 as shown in Fig. 6.

In Fig. 6, the user terminals, which are used (possessed) by users A, B, C,... respectively, are expressed as 2A, 2B, 2C, .... For example, when the CGM measuring device 1 is formulated or prescribed by the medical facility, an inherent user identification information number is imparted to each of the users, for example, by making user registration in the status providing service of the information providing system S. The user identification information, which includes the user identification information number, is stored in the identification information storage unit 33 of the auxiliary storage device 27 of the user terminal 2.

The characteristics, which include, for example, the physical constitution of each of the users, are reflected in the inherent calibration curve data SCu shown in Fig. 4. The calibration curve stored therein is modified. The inherent calibration curve data SCu, which is stored in the auxiliary storage device 27 of the user terminal 2, is updated every time when the new inherent calibration curve data SCu is successively delivered from the server apparatus 4 to the user terminal 2. The server side calibration curve storage unit 52 of the server apparatus 4 stores the common user calibration curve data SCp which is the calibration curve data common to the respective users and the inherent calibration curve data SCu which is prepared in order to be delivered to each of the user terminals 2. The inherent calibration curve data SCu has been explained with reference to Fig. 4, which is constructed as the calibration curve data inherent in each of the users. The inherent calibration curve data SCu is delivered to the corresponding user via the network 3 every time when the contents thereof are updated as described above.

In this procedure, the HbAlc concentration, which is measured by each of the users by means of the measuring apparatus 5, is correlated with the user identification information for identifying the user and the time and date of the measurement (data acquisition time and date information) by the aid of the wireless or wired communication from the measuring apparatus 5 or by the aid of the transportable recording medium. After that, the HbAlc concentration is stored in the HbAlc value storage unit 54. The average glucose concentrations AG, which are calculated in the user terminals 2 of the respective users, are transmitted to the server apparatus 4 via the network 3, and thus the average glucose concentrations AG of the respective users are collected in the server apparatus 4. The data (hereinafter referred to as "user transmission data"), which is transmitted from the user terminal 2 of each of the users, includes, for example, the user identification information and the data acquisition time and date information including the AG calculation reference period used as the basis for calculating the average glucose concentration AG, in addition to the average glucose concentration AG.

In this way, when the server apparatus 4 receives (acquires) the user transmission data from any user, the processor 41 specifies the transmission source of the data on the basis of the user identification information (transmission source indicates the user who transmitted the user transmission data or the user terminal 2 which is used by the concerning user). Subsequently, the processor 41 stores the average glucose concentration AG of the user corresponding to the user identification information in the AG value storage unit 53 while correlating the average glucose concentration AG with the user identification information and the data acquisition time and date information. For example, when the user A measures the HbAlc concentration by using the measuring apparatus 5 in the medical facility, the server apparatus 4 acquires the actually measured HbAlc concentration. The server apparatus 4, which has acquired the actually measured HbAlc concentration, stores the actually measured HbAlc concentration in the HbAlc value storage unit 54.

Subsequently, the processor 41 specifies the measurement time and date of HbA1c, and the processor 41 specifies that the newly added actually measured HbAlc concentration is the data in relation to the user A on the basis of the data acquisition time and date information and the user identification information corresponding to the actually measured HbAlc concentration added to the HbAlc value storage unit 54. Subsequently, the processor 41 accesses the AG value storage unit 53 to read the average glucose concentration AG which has the data acquisition time and date approximate to that of the actually measured HbAlc concentration, from the data in relation to the past average glucose concentrations AG received and accumulated from the user terminal 2A possessed by the user A. Accordingly, the actually measured pair data, which is composed of a pair (set) of the actually measured HbAlc concentration and the average glucose concentration AG each having the data acquisition time and date corresponding to one another, is formed. Subsequently, the processor 41 adds the actually measured pair data in relation to the user A formed as described above to the inherent calibration curve data SCu of the user A to update the inherent calibration curve data SCu stored in the server side calibration curve storage unit 52 of the auxiliary storage device 47. It is preferable that the pair of the actually measured HbAlc concentration and the average glucose concentration AG, which are used for the actually measured pair data, have the data acquisition times and dates which are close to one another. However, HbAlc is the index to indicate the long term blood sugar state, which has such a character that the value thereof is hardly changed sensitively in the short term. In view of the above, any special problem does not arise provided that the both data acquisition times and dates are not excessively deviated from each other.

In this embodiment, the explanation has been made about the update of the inherent calibration curve data SCu inherent in the user A for the purpose of convenience. However, the same procedure is also adopted for the inherent calibration curve data SCu of the other users B, C,.... That is, the server apparatus 4 receives (acquires) the user transmission data from the user terminals 2B, 2C,... used by the other users B, C,... and the actually measured HbAlc concentrations of the other users B, C,... are acquired from the measuring apparatus 5. Accordingly, the inherent calibration curve data SCu of the user corresponding to the acquired data is updated in the same manner as in the case of the user A. It is affirmed that the inherent calibration curve data SCu of each of the users constructed as described above is the calibration curve data in which the characteristics (for example, the physical constitution) of only the user himself/herself are reflected with respect to the standard model equation.

<Common calibration curve data SCp>

Next, the common calibration curve data SCp will be explained. Fig. 7 illustrates the common calibration curve data SCp stored in the auxiliary storage device 47 of the server apparatus 4 according to the first embodiment. The common calibration curve data SCp shown in the drawing is the calibration curve data in which the standard model equation is modified by incorporating the actually measured pair data (plots in the drawing) of all users A, B, C,... with respect to the standard model equation described above. The actually measured pair data referred to herein is as described above. That is, when the processor 41 of the server apparatus 4 forms the actually measured pair data concerning any one of the users, for example, the actually measured pair data concerning the user A, the actually measured pair data is plotted for both of the inherent calibration curve data SCu of the user A and the common user calibration curve data SCp. In this way, the new plot (actually measured pair data) is also added to the common calibration curve data SCp in the same manner as the inherent calibration curve data SCu. Therefore, the common calibration curve data SCp, which is stored in the server side calibration curve storage unit 52 of the auxiliary storage device 47, is updated by incorporating the newly added actually measured pair data in the same manner as the inherent calibration curve data SCu of the user A. The difference between the common calibration curve data SCp and the inherent calibration curve data SCu resides in that pieces of the actually measured pair data, which relate to a plurality of users without being limited to any specified user, are reflected. According to the common calibration curve data SCp constructed as described above, the characteristics of the individual users are averaged, and the correlation between the average glucose concentration AG and the HbAlc concentration averaged for all users is reflected.

This embodiment is illustrative of the exemplary mode in which the common calibration curve data SCp is successively updated by using the actually measured pair data of each of the users. However, it is not necessarily indispensable that the update should be performed by using the actually measured pair data of the concerning user. For example, it is also allowable that the common calibration curve data SCp is successively updated by using the measurement data in relation to the glucose concentrations and the HbAlc concentrations measured for a large number of examinees (patients) in a certain medical facility, in the medical facility such as a hospital or the like in which the measuring apparatus 5 is installed. The medical facility holds an enormous number of pieces of the measurement data, which is convenient to construct the common calibration curve data SCp in which the correlation between the glucose concentration and the HbAlc concentration of a general examinee is reflected. Further, it is also allowable to use the calibration curve data in which the correlation between the glucose concentration and the HbAlc concentration is prescribed or defined by using the data cited from any general scientific paper. For example, it is also appropriate to utilize the correlation defined by a regression curve shown in Figure. 1 of Non-Patent Document 1.

The server apparatus 4 delivers the inherent calibration curve data SCu after the update via the network 3 to the user terminal 2 used by the user corresponding to the updated inherent calibration curve data SCu every time when the inherent calibration curve data SCu, which relates to any user stored in the server side calibration curve storage unit 52 of the auxiliary storage device 47, is updated. The user terminal 2, which receives the new inherent calibration curve data SCu from the server apparatus 4, updates (overwrites) the inherent calibration curve data SCu stored in the second calibration curve storage unit 32 of the auxiliary storage device 27. In this way, the user terminal 2 of each of the users successively updates the inherent calibration curve data SCu to the latest version every time when the user terminal 2 receives the delivery of the inherent calibration curve data SCu from the server apparatus 4. Accordingly, the user terminal 2 can estimate the HbAlc concentration by using the inherent calibration curve data SCu in the state in which, for example, the latest physical constitution of the user who uses the user terminal 2 is reflected.

However, the physical constitution of the user changes in some cases as the time elapses. Such a situation sometimes arises that the inherent calibration curve data SCu is updated by the actually measured pair data which is affected by the error caused by any external factor (external factor error). Typical examples of the external factor error are exemplified, for example, by those caused by the calculation error of the average glucose concentration AG resulting from the malfunction (disorder) and/or the abnormality of the CGM measuring device 1 (including the glucose sensor 11) and/or the measurement error of the HbAlc concentration resulting from, for example, the malfunction (disorder) and/or the abnormality of the measuring apparatus 5.

In such a situation, the inherent calibration curve data SCu is sometimes greatly deviated from the line (state) on which the inherent calibration curve data SCu should originally exist. If any future HbAlc concentration is estimated by using such inherent calibration curve data SCu, it is feared that the user may erroneously recognize the blood sugar state of the user himself/herself due to the increase in the estimation error. In view of the above, the information providing system S according to this embodiment is provided with the function to check whether or not any abnormal value may be highly possibly included in the actually measured pair data included in the inherent calibration curve data SCu. The information providing system S judges whether or not the present inherent calibration curve data SCu can prescribe or define the correlation between the average glucose concentration AG and the HbAlc concentration in relation to the individual user within a certain degree of proper range.

<First control>

The first control according to this embodiment will be explained below. Fig. 8 illustrates the first control according to the first embodiment. In the first control, an explanation will be made about the exemplary control performed when the server apparatus 4 receives the user transmission data including the information in relation to the average glucose concentration AG from the user terminal 2 of any user. The first control is executed by loading the program stored in the auxiliary storage device 47 to the main storage device 42 by the processor 41 of the server apparatus 4.

In this context, Fig. 8 is equivalent to one obtained by superimposing the inherent calibration curve data SCu shown in Fig. 4 and the common calibration curve data SCp shown in Fig. 7. For example, it is assumed that the server apparatus 4 receives the user transmission data from the user terminal 2 of a certain user, and the server apparatus 4 acquires the average glucose concentration AG having a value indicated by a broken line in Fig. 8 (C in the drawing). In this situation, the processor 41 of the server apparatus 4 substitutes the acquired average glucose concentration AG (value C) for the inherent calibration curve data SCu corresponding to the user and the common calibration curve data SCp respectively to derive the corresponding values (Vd1 and Vd2 in the drawing) of glycohemoglobin (HbA1c) as the remaining biological component for constructing the actually measured pair data respectively. In this procedure, the HbAlc concentration (first index value), which corresponds to the average glucose concentration AG substituted for the inherent calibration curve data SCu, is designated as the inherent calibration curve corresponding value Vd1, and the HbAlc concentration (first index value), which corresponds to the average glucose concentration AG substituted for the common calibration curve data SCp, is designated as the common calibration curve corresponding value Vd2.

In the first control, the corresponding value deviation amount $\Delta Vd$ is calculated, which is the deviation amount between the inherent calibration curve corresponding value Vd1 and the common calibration curve corresponding value Vd2. The corresponding value deviation amount $\Delta Vd$ is rated or valuated as the index to indicate the degree of difference brought about in relation to the estimation results of the HbAlc concentrations corresponding to the identical average glucose concentration AG between the inherence calibration curve data SCu and the common calibration curve data SCp. Accordingly, in the first control, it is checked whether or not any abnormal value is highly possibly included in the actually measured pair data included in the inherent calibration curve data SCu on the basis of the magnitude of the corresponding value deviation amount $\Delta Vd$ to judge whether or not the present inherent calibration curve data SCu is in an appropriate state.

An explanation will be made below with reference to Fig. 9 about the specified process contents of the first control. Fig. 9 shows a flow chart illustrating the procedure of a first control routine according to this embodiment. This control routine is executed by loading the program stored in the auxiliary storage device 47 to the main storage device 42 by the processor 41 of the server apparatus 4. This control routine is repeatedly executed every predetermined period.

At first, in Step S101, the processor 41 accesses the AG value storage unit 53 to judge whether or not any new average glucose concentration AG is added from any user. If the affirmative judgment is made in this step, the processor 41 detects that the auxiliary storage device 47 newly acquires the average glucose concentration AG as the first measured value. In this case, the routine proceeds to Step S102. On the other hand, if the negative judgment is made in this step, this control routine is once completed.

The interval and the frequency, at which each of the user terminals 2 transmits the average glucose concentration AG to the server apparatus 4, can be arbitrarily set by the user. The AG calculation reference period is appropriately set, for example, within a range of about several days to 3 months. However, there is no limitation to this range. In Step S101, the processor 41 may specify the acquisition source user who is the acquisition source of the data on the basis of the user identification information and the data acquisition time and date information concerning the user transmission data. The processor 41 may prepare the actually measured pair data by reading the actually measured HbAlc value which has a relatively close measurement timing and which is included in the actually measured HbAlc values in relation to the concerning transmission source user stored in the HbAlc value storage unit 54.

In Step S102, the processor 41 specifies the acquisition source user (or the user terminal 2 used by the acquisition source user) as the acquisition source of the data on the basis of the user identification information and the data acquisition time and date information concerning the user transmission data, and the processor 41 accesses the server side calibration curve storage unit 52 corresponding to the acquisition source user. Subsequently, the processor 41 substitutes the first measured value as the acquired average glucose concentration AG for the inherent calibration curve data SCu corresponding to the acquisition source user and the common calibration curve data SCp respectively to derive the inherent calibration curve corresponding value Vd1 and the common calibration curve corresponding value Vd2 respectively.

In Step S103, the processor 41 judges whether or not the corresponding value deviation amount $\Delta Vd$ exceeds a predetermined first reference amount A1. The corresponding value deviation amount $\Delta Vd$ is defined as the absolute value of the difference between the inherent calibration curve corresponding value Vd1 and the common calibration curve corresponding value Vd2 ($\Delta Vd = |Vd1 - Vd2|$). If the negative judgment is made in this step ($\Delta Vd \leq A1$), it is judged that the corresponding value deviation amount $\Delta Vd$ is maintained at a sufficiently small value, and any problem does not arise in the inherent calibration curve data SCu in relation to the user terminal 2 of the acquisition source user at all. The routine proceeds to Step S104. That is, in this case, it is possible to judge that the correlation between the HbAlc concentration and the average glucose concentration AG defined by the inherent calibration curve data SCu in relation to the acquisition source user is in an appropriate state at present.

Therefore, in Step S104, the processor 41 transmits the predetermined first information to the user terminal 2 of the acquisition source user, and then this control routine is once completed. In this procedure, a message of "You are in normal state" is displayed, for example, on the second display 24B by the user terminal 2 which has received the first information. If the negative judgment is made in Step S103 as described above, it is unnecessary for the user to perform any positive action. Therefore, it is not necessarily indispensable to transmit the first information to the user terminal 2. Therefore, in this case, it is also possible to omit the process of Step S104. On the other hand, if the affirmative judgment is made in Step S103 ($\Delta Vd > A1$), the routine proceeds to Step S105. In Step S105, the processor 41 judges whether or not the corresponding value deviation amount $\Delta Vd$ exceeds a predetermined second reference value A2. The second reference value A2 is a threshold value which is set to a value larger than the first reference value A1. If the negative judgment is made in this step ($\Delta Vd \leq A2$), the routine proceeds to Step S106. If the affirmative judgment is made ($\Delta Vd > A2$), the routine proceeds to Step S107.

If the routine proceeds to Step S106, it is meant that the corresponding value deviation amount $\Delta Vd$ exceeds the first reference value A1 and the corresponding value deviation amount $\Delta Vd$ is not more than the second reference value A2. In this case, the significant difference is acknowledged between the inherent calibration curve corresponding value Vd1 and the common calibration curve corresponding value Vd2. It is judged that the significant difference is caused by the change of the physical constitution of the acquisition source user. Accordingly, in Step S106, the processor 41 transmits the second information to the user terminal 2 of the acquisition source user. Further, the inherent calibration curve data update flag is set to ON ($F \leftarrow ON$), and then this control routine is once completed. The switching of the inherent calibration curve data update flag from OFF to ON provides the trigger to perform the inherent calibration curve data update control routine as described later on.

The second information is the measured value request information to request the actual measurement of the unacquired measured value of one not added to (not acquired by) the AG value storage unit 53 in Step S101, of the average glucose concentration AG and the HbAlc concentration. In this procedure, the HbAlc concentration corresponds to the unacquired measured value. If the user terminal 2 of the acquisition source user receives the second information (measured value request information), the processor 21 allows, for example, the second display 24B to display a message of "Actually measure glycohemoglobin concentration in designated medical facility".

Next, the process in Step S107 will be explained. The situation, in which the routine proceeds to Step S107, means the fact that the corresponding value deviation amount ΔVd exceeds the second reference value A2. In this case, the significant difference is acknowledged between the inherent calibration curve corresponding value Vd1 and the common calibration curve corresponding value Vd2. It is judged that the significant difference is caused by the external factor error such as the malfunction, the abnormality or the like of the CGM measuring device 1 (including the glucose sensor 11). In this case, in Step S107, the processor 41 transmits the third information to the user terminal 2 of the acquisition source user, and then this control routine is once completed.

If the user terminal 2 of the acquisition source user receives the third information, the processor 21 of the user terminal 2 allows, for example, the second display 24B to display a message of "CGM measuring device and/or sensor may be possibly abnormal, so exchange sensor with new one or perform maintenance for CGM measuring device".

In the first control routine described above, the transmission and the notice of the first information to the third information to the user terminal 2 are not limited to those of the embodiment described above. For example, it is also allowable that the respective messages described above are outputted as voice from a speaker (not shown) of the user terminal 2.

Next, an explanation will be made about the specified process of the inherent calibration curve data update control routine. Fig. 10 shows a flow chart illustrating the procedure of the inherent calibration curve data update control routine according to this embodiment. This routine is also executed by the processor 41 of the server apparatus 4 in the same manner as the first control routine. This control routine is repeatedly executed every predetermined period if the inherent calibration curve data update flag is in a state of ON. In this procedure, if the acquisition source user, who saw the message presentation concerning the second information (measured value request information) on the second display 24B of the user terminal 2, actually measured the HbAlc concentration in the body fluid by means of the measuring apparatus 5 in the medical facility, the measurement result is newly stored in the HbAlc value storage unit 54 of the auxiliary storage device 47 of the server apparatus 4.

In Step S201, the processor 41 of the server apparatus 4 accesses the HbAlc value storage unit 54. Subsequently, the second information is transmitted to the user terminal 2 of the acquisition source user in the first control described above, and then it is judged whether or not the actually measured HbAlc concentration concerning the acquisition source user is newly added (updated). If it is judged in this step that the actually measured HbAlc concentration is not newly updated, this routine is completed. In this case, this control routine is executed again after a predetermined period elapses. On the other hand, if it is judged in this step that the actually measured HbAlc concentration is newly updated, the processor 41 detects the fact that the HbAlc value storage unit 54 newly acquires the actually measured HbAlc concentration as the second measured value. The routine proceeds to Step S202.

The newly added actually measured HbAlc concentration (second measured value) is stored in the HbAlc value storage unit 54 while being correlated with the user identification information and the data acquisition time and date information. Accordingly, in Step S202, the processor 41 of the server apparatus 4 updates the inherent calibration curve data SCu of the acquisition source user by using, as the actually measured pair data, the pair of the actually measured HbAlc concentration (second measured value) and the average glucose concentration AG (first measured value) acquired in Step S101 in the first control. Subsequently, in Step S203, the processor 41 of the server apparatus 4 delivers the inherent calibration curve data SCu updated in Step S202 to the user terminal 2 of the corresponding acquisition source user. After that, the processor 41 of the server apparatus 4 sets the inherent calibration curve data update flag to OFF, and this routine is completed.

Next, a modified embodiment of this embodiment will be explained. In this modified embodiment, the correction request information, which is directed to the average glucose concentration AG, is transmitted to the user terminal 2 of the acquisition source user in addition to the third information in Step S107 of the first control routine shown in Fig. 9. The correction request information is the instruction information to request that the corrected average glucose concentration AG', which is recalculated for the average glucose concentration AG after carrying out the abnormal value removing process concerning a first modified embodiment or the reference period shortening process concerning a second modified embodiment, should be transmitted, with respect to the user terminal 2.

Fig. 11 illustrates the abnormal value removing process according to this embodiment. The abnormal value removing process refers to the process in which any abnormal value is removed from the measurement data of the glucose concentration acquired in the AG calculation reference period. The graph shown in the drawing indicates the time-dependent transition or change of the glucose concentration measured by the CGM measuring device 1. In Fig. 11, the setting is made such that the glucose concentration is measured every 1 minute. In the abnormal value removing process, if the change range of the glucose concentration per unit time (hereinafter referred to as "glucose concentration unit change amount") exceeds a predetermined allowable value, the concerning plot is removed as one which indicates the abnormal value (detached value). In Fig. 11, for example, the pieces of measurement data (plots) A, B are removed as abnormal values. Accordingly, the average glucose concentration AG is recalculated by using the measurement data after removing the abnormal values (measurement data A, B) in the AG calculation reference period. It is preferable that a proper value is determined beforehand, for example, on the basis of an empirical rule such as an experiment or the like for the allowable value in relation to the glucose concentration unit change amount.

The abnormal value removing process explained with reference to Fig. 11 is executed by loading the program stored in the auxiliary storage device 27 to the main storage device 22 by the processor 21 of the user terminal 2. The processor 21 obtains the corrected average glucose concentration AG' by calculating the average value of the measurement data of the glucose concentration acquired by performing the abnormal value removing process (hereinafter referred to as "abnormal value-removed measurement data"), and the corrected average glucose concentration AG' is transmitted to the server apparatus 4 again. The corrected average glucose concentration AG' corresponds to the corrected measured value of the first biological component according to the present invention.

If the corrected average glucose concentration AG' is transmitted to the server apparatus 4 by the processor 21 of the user terminal 2 as described above, the transmission is used as the trigger to restart the first control routine explained with reference to Fig. 9. As a result, the respective processes shown in Fig. 9 are performed by using the corrected average glucose concentration AG'. Any abnormal value, which was included in the measurement data of the glucose concentration, is removed from the corrected average glucose concentration AG'. Therefore, the corresponding value deviation amount ΔVd, which is calculated in the first control, is reduced. As a result, it is considered that the corresponding value deviation amount ΔVd hardly exceeds the second reference value A2.

Next, an explanation will be made about the reference period shortening process according to the second modified embodiment. The reference period shortening process is the process to shorten the AG calculation reference period. The processor 21 of the user terminal 2 calculates the corrected average glucose concentration AG' on the basis of the measurement data of the glucose concentration acquired by performing the reference period shortening process. For example, if the AG calculation reference period is set to about 3 months in the initial setting, the AG calculation reference period may be shortened to about 1 month by carrying out the reference period shortening process. Another variation concerning the reference period shortening process is also available. That is, the period (hereinafter referred to as "abnormal value frequent appearance period"), in which the frequency state of the glucose concentration unit change amount to exceed an allowable value frequently arises while exceeding a predetermined threshold value, is acquired in relation to the measurement data of the glucose concentration in the AG calculation reference period. The AG calculation reference period is shortened by excluding the abnormal value frequent appearance period from the AG calculation reference period.

In this way, the processor 21 of the user terminal 2 transmits the corrected average glucose concentration AG' acquired by performing the reference period shortening process to the server apparatus 4 again. Subsequently, this is used as the trigger to start the first control routine as explained with reference to Fig. 9. In this procedure, the respective processes shown in Fig. 9 are performed on the basis of the corrected average glucose concentration AG'. In this procedure, the corrected average glucose concentration AG' is calculated on the basis of the measurement data of the glucose concentration acquired in the remaining period of the AG calculation reference period from which the abnormal value frequent appearance period is excluded. Therefore, the corresponding value deviation amount ΔVd, which is calculated in the first control, is reduced. As a result, the corresponding value deviation amount ΔVd hardly exceeds the second reference value A2.

Any one of the abnormal value removing process according to the first modified embodiment and the reference period shortening process according to the second modified embodiment may be selectively carried out, or the both may be carried out. For example, the following procedure is also available. That is, even when the abnormal value removing process (reference period shortening process) is firstly carried out, if the corresponding value deviation amount ΔVd, which is acquired by carrying out the first control routine thereafter, exceeds the second reference value A2, then the reference period shortening process (abnormal value removing process) is performed.

<Second control>

Next, the second control according to this embodiment will be explained. Fig. 12 illustrates the second control according to the first embodiment. Fig. 12 shows a graph in which the inherent calibration curve data SCu and the common calibration curve data SCp are superimposed in the same manner as in Fig. 8. In the second control, an example of the control is explained, which is provided when the server apparatus 4 acquires the actually measured HbAlc concentration in relation to any user from the measuring apparatus 5. The second control is also executed by loading the program stored in the auxiliary storage device 47 to the main storage device 42 by the processor 41 of the server apparatus 4.

For example, it is assumed that the server apparatus 4 acquires the actually measured HbAlc concentration of a certain user (D in Fig. 12) from the measuring apparatus 5. In this case, the processor 41 of the server apparatus 4 substitutes the acquired HbAlc concentration (value D) for the inherent calibration curve data SCu corresponding to the user and the common calibration curve data SCp respectively. As a result, the corresponding values (Vd1' and Vd2' in Fig. 12), which correspond to the average glucose concentration AG as the remaining biological component for constructing the actually measured pair data together with the HbAlc concentration, are derived respectively. In this procedure, the average glucose concentration AG, which corresponds to the actually measured HbAlc concentration substituted for the inherent calibration curve data SCu, is designated as the inherent calibration curve corresponding value Vd1'. Further, the average glucose concentration AG, which corresponds to the actually measured HbAlc concentration substituted for the common calibration curve data SCp, is designated as the common calibration curve corresponding value Vd2'. Subsequently, also in the second control, the corresponding value deviation amount ΔVd' is acquired, which is the deviation amount between the inherent calibration curve corresponding value Vd1' and the common calibration curve corresponding value Vd2' in the same manner as in the first control. It is checked whether or not any abnormal value is highly possibly included in the actually measured pair data included in the inherent calibration curve data SCu on the basis of the magnitude of the determined corresponding value deviation amount ΔVd' to judge whether or not the present inherent calibration curve data SCu is in an appropriate state.

An explanation will be made below with reference to Fig. 13 about the specified process contents of the second control. Fig. 13 shows a flow chart illustrating the procedure of a second control routine according to this embodiment. This control routine is executed by loading the program stored in the auxiliary storage device 47 to the main storage device 42 by the processor 41 of the server apparatus 4. This control routine is repeatedly executed every predetermined period.

At first, in Step S301, the processor 41 accesses the HbAlc value storage unit 54 to judge whether or not the actually measured HbAlc concentration is newly added from any user. If the affirmative judgment is made in this step, the processor 41 detects the fact that the HbAlc value storage unit 54 newly acquires the actually measured HbAlc concentration as the first measured value. In this case, the routine proceeds to Step S302. On the other hand, if the negative judgment is made in this step, this control routine is once completed. In Step S302, the processor 41 specifies the acquisition source user (or the user terminal 2 of the acquisition source user) as the acquisition source of the actually measured HbAlc concentration on the basis of the user identification information stored while being correlated with the actually measured HbAlc concentration in the HbAlc value storage unit 54. The processor 41 accesses the server side calibration curve storage unit 52 corresponding to the acquisition source user, and the acquired actually measured HbAlc concentration is substituted as the first measured value for the inherent calibration curve data SCu corresponding to the acquisition source user and the common calibration curve data SCp respectively to thereby derive the inherent calibration curve corresponding value Vd1' and the common calibration curve corresponding value Vd2' respectively. In Step S302, the processor 41 may prepare the actually measured pair data by reading the average glucose concentration AG having the approximate measurement timing from the AG value storage unit 53 on the basis of the data acquisition time and date information stored while being correlated with the actually measured HbAlc concentration in the HbAlc value storage unit 54.

In Step S303, the processor 41 judges whether or not the corresponding value deviation amount ΔVd' exceeds a predetermined first reference amount A1'. The corresponding value deviation amount ΔVd' is defined as the absolute value of the difference between the inherent calibration curve corresponding value Vd1' and the common calibration curve corresponding value Vd2' ($\Delta Vd' = |Vd1' - Vd2'|$). If the negative judgment is made in this step ($\Delta Vd' \leq A1'$), it is judged that the corresponding value deviation amount ΔVd' is maintained at a sufficiently small value, and any problem does not arise in the inherent calibration curve data SCu in the user terminal 2 of the acquisition source user. The routine proceeds to Step S304. In this case, it is possible to judge that the correlation between the HbAlc concentration and the average glucose concentration AG defined by the inherent calibration curve data SCu in relation to the acquisition source user is in an appropriate state at present.

Therefore, in Step S304, the processor 41 transmits the first information to the user terminal 2 of the acquisition source user, and then this control routine is once completed. In this procedure, a message of "Normal state is confirmed" is displayed, for example, on the second display 24B by the user terminal 2 which has received the first information.

In this step, it is unnecessary for the user to perform any positive action in the same manner as in the first control. Therefore, it is also allowable that this control routine is completed as it is, while omitting the process in Step S304 in which the first information is transmitted to the user terminal 2. The transmission and the notice of the first information with respect to the user terminal 2 are not limited to those of the embodiment described above. For example, the message as described above may be outputted as voice from a speaker (not shown) of the user terminal 2.

On the other hand, if the affirmative judgment is made in Step S303 ($\Delta Vd' > A1'$), the routine proceeds to Step S305. In Step S305, it is further judged whether or not the corresponding value deviation amount ΔVd' exceeds a predetermined second reference value A2'. The second reference value A2' is a threshold value which is set to a value larger than the first reference value A1'. If the negative judgment is made in this step ($\Delta Vd' \leq A2'$), the routine proceeds to Step S306. On the other hand, if the affirmative judgment is made in this step ($\Delta Vd' > A2'$), the routine proceeds to Step S307.

If the routine proceeds to Step S306, it is meant that the corresponding value deviation amount ΔVd' exceeds the first reference value A1' and the corresponding value deviation amount ΔVd' is not more than the second reference value A2'. In this case, the significant difference is acknowledged between the inherent calibration curve corresponding value Vd1' and the common calibration curve corresponding value Vd2'. It is judged that the significant difference is caused by the change of the physical constitution of the acquisition source user. Accordingly, in Step S306, the processor 41 transmits the fourth information to the user terminal 2 of the acquisition source user. Further, the second inherent calibration curve data update flag is set to ON (F ← ON), and then this control routine is once completed.

The switching of the second inherent calibration curve data update flag from OFF to ON provides the trigger to perform the second inherent calibration curve data update control routine as described later on. The fourth information is the instruction information having the contents to request that the average glucose concentration AG, which is not acquired by the HbAlc value storage unit 54 in Step S301 and which is any one of the average glucose concentration AG and the HbAlc concentration, should be calculated and transmitted.

Next, the process in Step S307 will be explained. The situation, in which the routine proceeds to Step S307, means the fact that the corresponding value deviation amount ΔVd' exceeds the second reference value A2'. In this case, the significant difference is acknowledged between the inherent calibration curve corresponding value Vd1' and the common calibration curve corresponding value Vd2'. It is judged that the significant difference is caused by the external factor error such as the malfunction or the abnormality of the measuring apparatus 5. In this case, the processor 41 of the server apparatus 4 outputs and displays, for example, a message of "Inspect whether or not measuring apparatus has abnormality or malfunction" on a monitor (display) in relation to the output device 44. Alternatively, the message described above may be outputted as voice from a speaker (not shown) of the output device 44. When the process of this step is completed, this control routine is once completed. In this control, if it is confirmed that the measuring apparatus 5 involves no abnormality and no malfunction, it is also allowable to perform the process of Step S306.

Next, an explanation will be made about the second inherent calibration curve data update control routine. Fig. 14 shows a flow chart illustrating the procedure of the second inherent calibration curve data update control routine according to the first embodiment. This routine is also executed by the processor 41 of the server apparatus 4 in the same manner as the second control routine. This control routine is repeatedly executed every predetermined period if the second inherent calibration curve data update flag is in a state of ON.

At first, an explanation will be made about the process performed by the user terminal 2 of the acquisition source user which receives the fourth information transmitted by the server apparatus 4 in Step S306 of the second control routine. As described above, the fourth information is the instruction information having the contents to request the calculation of the average glucose concentration AG and the transmission of the calculation result as described above. The fourth information includes the data acquisition time and date information of the actually measured HbAlc concentration acquired by the server apparatus 4 in Step S301 of the second control routine. The processor 21 of the user terminal 2, which receives the fourth information, executes the process for calculating the average glucose concentration AG so that the measurement timing of the actually measured HbAlc concentration is included in the AG calculation reference period. In this procedure, the AG calculation reference period can be set to an appropriate period. The calculated average glucose concentration AG is transmitted as the second measured value to the server apparatus 4.

Subsequently, when the second inherent calibration curve data update control routine is started, the processor 41 of the server apparatus 4 firstly accesses the AG value storage unit 53 of the auxiliary storage device 47 in Step S401. Subsequently, the processor 41 judges whether or not the average glucose concentration AG concerning the acquisition source user is newly added (updated) as the second measured value after the fourth information is transmitted to the user terminal 2 of the acquisition source user in the second control as described above. Accordingly, the processor 41 judges whether or not the AG value storage unit 53 newly acquires the average glucose concentration AG as the second measured value. In this connection, if the average glucose concentration AG, which is calculated by the user terminal 2 of the acquisition source user that receives the instruction concerning the fourth information, is transmitted to the server apparatus 4, the calculation result is newly stored in the AG value storage unit 53 of the auxiliary storage device 47 of the server apparatus 4.

If it is judged in this step that the average glucose concentration AG is not newly updated as the second measured value, this routine is completed as it is. In this case, this control routine is executed again after a predetermined period of time elapses. On the other hand, if it is judged in this step that the average glucose concentration AG is newly updated as the second measured value, the routine proceeds to Step S402.

The average glucose concentration AG (second measured value), which is newly added to the AG value storage unit 53, is stored while being correlated with the user identification information and the data acquisition time and date information. Accordingly, in Step S402, the processor 41 of the server apparatus 4 updates the inherent calibration curve data SCu of the acquisition source user by using, as the actually measured pair data, the pair of the average glucose concentration AG (second measured value) and the actually measured HbAlc concentration (first measured value) acquired in Step S301 in the second control routine.

Subsequently, in Step S403, the processor 41 of the server apparatus 4 transmits the inherent calibration curve data SCu updated in Step S402 to the user terminal 2 of the corresponding acquisition source user. After that, the processor 41 of the server apparatus 4 sets the second inherent calibration curve data update flag to OFF, and this routine is completed.

As described above, according to the information providing system S of this embodiment, both of the Glu information which contributes to the short term diabetes care and the HbAlc information which contributes to the recognition or grasp of the long term metabolic result in relation to glucose contained in the body fluid are simultaneously displayed on the display 24 of the user terminal 2. Accordingly, the information terminal, which is excellent in convenience of use, can be provided for the user. In this case, the inherent calibration curve data SCu, which is provided to be used for the estimation of the HbAlc concentration in the user terminal 2, is delivered from the server apparatus 4 at any time. Therefore, the inherent calibration curve data SCu in the user terminal 2 can be always maintained to be in a satisfactory state. When the HbAlc concentration is estimated on the basis of the average glucose concentration AG, it is possible to judge whether or not the inherent calibration curve data SCu is proper or reasonable as the calibration curve which is in conformity with the present physical constitution of the user on the basis of the relative relationship between the common calibration curve data SCp which is common to the respective users and the inherent calibration curve data SCu which is individual for the individual users, while it is premised that the inherent calibration curve data SCu, which is inherent in each of the users, is used. That is, it is possible to judge whether or not the present inherent calibration curve data SCu for each of the users is in the originally expected state. It is possible to update the inherent calibration curve data SCu, if necessary. It is possible to arose a caution if there is a possibility of the external factor error.

In this embodiment, the control contents of the first control and the second control may be executed while being appropriately combined with each other. For example, it is also allowable to adopt the following procedure. That is, when the server apparatus 4 acquires any one of the average glucose concentration AG and the actually measured HbAlc concentration as the first measured value from the user terminal 2, if the corresponding value deviation amount exceeds the first reference amount, then the other measured value is acquired as the second measured value, and the inherent calibration curve data SCu of the corresponding user is updated by using the second measured value and the first measured value having been already acquired.

[Other exemplary system construction]

Next, an explanation will be made about variation of the exemplary system construction (arrangement) concerning the information providing system S. It is possible to adopt various forms or modes within a range without deviating from the gist or essential characteristics of the present invention for the information providing system S according to this embodiment.

Fig. 15 shows an exemplary arrangement of another system in relation to the information providing system S according to the first embodiment. In the exemplary arrangement of the system described above, such an exemplary arrangement of the system is adopted that the information processing apparatus according to the present invention is provided independently as the server apparatus 4 of the general purpose computer. However, as shown in Fig. 15, the information processing apparatus may be incorporated into a measuring apparatus 5A. In this case, the block diagram illustrating the arrangement shown in Fig. 3 can be equivalently employed as a block diagram illustrating a part of the arrangement provided for the measuring apparatus 5A. As described above, the information processing apparatus according to the present invention may be realized as the server apparatus 4 which is independent from the measuring apparatus for measuring the HbAlc concentration. Alternatively, the information processing apparatus according to the present invention may be realized as the computer which is incorporated into the measuring apparatus. In the case of the latter, it is also possible to apply various types of the control as described above.

[Display mode of user terminal]

Next, an explanation will be made about the display mode of the user terminal 2 concerning the information providing system S. The user terminal 2 has the display 24 (first display 24A and second display 24B) as described above. The Glu information is displayed on the first display 24A, and the HbAlc information is displayed on the second display 24B. Various modes are prepared for the display mode in relation to the Glu information and the HbAlc information to be displayed on the display 24 of the user terminal 2. Specifically, the display mode is realized by executing various programs by the processor 21 by using various pieces of data stored in the auxiliary storage device 27.

<<Basic display mode>>

Figs. 16 and 17 illustrate the basic display mode of the user terminal 2. In the basic display mode, the transition curve (history curve) of the glucose concentration provided in a relatively short period (past 2 hours in the example shown in Fig. 16) and the present glucose concentration are displayed on the first display 24A. The range of the time axis for displaying the transition curve of the glucose concentration is not specifically limited, which can be also changed appropriately by accepting the manual operation with the operation unit 23 performed by the user.

The user terminal 2 according to this embodiment determines, for example, the estimated HbAlc concentration which reflects the future blood sugar state to be provided several months later, on the basis of the average glucose concentration AG calculated from the measurement data of the CGM measuring device 1. In other words, the present estimated HbAlc concentration is estimated, for example, on the basis of the measurement result of the glucose concentration measured several months ago.

The display is provided on the second display 24B in such a mode that the plot which indicates the past estimated HbAlc concentration and the plot which indicates the present estimated HbAlc concentration are connected by a solid line, and the plot which indicates the present estimated HbAlc concentration and the plot which indicates the future estimated HbAlc concentration are connected by a broken line. Further, in the example shown in Fig. 17, the plots which indicate the past and present estimated HbAlc concentrations are in a lighting display mode, and the plot which indicates the future estimated HbAlc concentration is in a flashing display mode. Accordingly, the display mode differs between the both. This is useful to intuitively and quickly grasp whether each of the plots displayed on the second display 24B represents the estimated HbAlc concentration until the present time or each of the plots represents the estimated HbAlc concentration in future.

The following diagnostic criteria are used for the diabetes in Japan. That is, if any one of the conditions of (1) the fasting blood glucose (fasting plasma glucose) level (FPG) is not less than 126 mg/dL, (2) the blood glucose level based on the 75 g oral glucose tolerance test (OGTT) is not less than 200 mg/dL, (3) any time blood glucose level is not less than 200 mg/dL, and (4) the hemoglobin A1c concentration is not less than 6.5% is fulfilled, it is judged that the disease is of the "diabetes type". If the reexamination is performed on another day, and any one of the conditions (1) to (4) described above is fulfilled in the reexamination as well, then it is diagnosed that the disease is "diabetes". If any one of the conditions (1) to (3) and any one of the conditions of (5) the typical symptoms of diabetes (for example, thirst or dry mouth, polydipsia, polyuria) and (6) the presence of diabetic retinopathy are fulfilled, it is diagnosed that the disease is "diabetes" without performing the reexamination. If the conditions of (1) the fasting blood glucose (fasting plasma glucose) level (FPG) is less than 110 mg/dL and (2) the blood glucose level based on the 75 g oral glucose tolerance test (OGTT) is less than 140 mg/dL are fulfilled, the disease is classified into the "normal type". Even in the case of the "diabetes type", the disease, which is not of the "normal type", is classified into the "borderline type". The "borderline type", which is considered to be at risk of diabetes, is further classified into the following three types (kinds). In general, the "borderline type" is also referred to as "borderline type diabetes" (which is not the formal disease), which has a high ratio to become "diabetes type". The "borderline type" is considered to reside in such a group that the future risk to cause diabetes is high even when the diabetes does not appear at present.

<IFG type (fasting blood glucose abnormal type or impaired fasting glycaemia type)>

As for the IFG type, if the fasting blood glucose (fasting plasma glucose) level (FPG) is within a range of not less than 110 mg/dL and less than 126 mg/dL, it is judged that the disease falls under this type. FPG can be defined as the blood sugar level (blood glucose level) in the fasting state in which the time elapses after the meal. For example, the blood sugar level, which is obtained after the elapse of 8 to 12 hours after the meal, is adopted.

<IGT type (loading glucose tolerance ability abnormal type or impaired glucose tolerance type)>

As for the IGT type, if the blood sugar level (blood glucose level) based on the 75 g oral glucose tolerance test (OGTT) is within a range of not less than 140 mg/dL and less than 200 mg/dL, it is judged that the disease falls under this type. The 75 g oral glucose tolerance test is such a test that the blood sugar level is measured, for example, 30 minutes, 60 minutes, 90 minutes, and 120 minutes after the ingestion of 75 g of grape sugar (glucose) (for example, aqueous grape sugar solution). For example, the measuring timings and the number of times of measurement of the blood sugar level after the ingestion of grape sugar are different from those described above in some cases.

<IFG + IGT type (complication type)>

The IFG + IGT type is the type which falls under both of the IFG type and the IGT type described above. If the fasting blood glucose (fasting plasma glucose) level (FPG) is within a range of not less than 110 mg/dL and less than 126 mg/dL and the blood sugar level (blood glucose level) based on the 75 g oral glucose tolerance test (OGTT) is within a range of not less than 140 mg/dL and less than 200 mg/dL, the disease falls under this complication type. As for the description concerning the diagnostic criteria of the diabetes as described above, the diagnostic criteria for the diabetes diagnosis are exemplified by way of example, which had been established by Japan Diabetes Society in Japan when the invention was created by the present inventors. It is sufficiently assumed that the diagnostic criteria may be revised as the time elapses and/or the diagnostic criteria may differ depending on each of countries. However, the specified contents of the diagnostic criteria exert no influence on the application of the present invention and the technical scope thereof at all.

As described above, as for the HbAlc concentration, the numerical value of 6.5% has the important significance in relation to the diabetes care. Therefore, if the estimated HbAlc concentration, which is obtained by the processor 21, is not less than a predetermined reference concentration Vsh, the output display mode of the estimated HbAlc concentration is displayed in a highlighting manner on the second display 24B of the user terminal 2.

In this embodiment, the reference concentration Vsh is set to 6.5%. However, the reference concentration Vsh may be appropriately changed. The mode of highlighting, which is provided when the estimated HbAlc concentration is not less than Vsh, is preferably exemplified by the use of any conspicuous color (for example, red) as compared with the color (for example, black) used in the ordinary display mode. In this embodiment, for example, the plot is provided with the black color as the ordinary display mode if the estimated HbAlc concentration, which is obtained by substituting the average glucose concentration AG for the inherent calibration curve data SCu, is less than the reference concentration Vsh (6.5%). If the estimated HbAlc concentration is not less than the reference concentration Vsh (6.5%), the plot is provided with the red color as the highlighting display mode. In this way, the user can clearly grasp whether or not the estimated result of the HbAlc concentration is not less than the reference concentration Vsh (6.5%) by glancing at the second display 24B. However, the mode of highlighting display is described by way of example. It is also allowable to adopt any other mode.

The frequency, at which the processor 21 of the user terminal 2 obtains the estimated HbAlc concentration, is a matter of design capable of being changed depending on the specification of the user terminal 2. The processor 21 of the user terminal 2 updates the HbAlc information on the second display 24B every time when the estimated HbAlc concentration is newly obtained.

<<AG display mode>>

Fig. 18 illustrates the AG display mode of the user terminal 2. The AG display mode is such a mode that the average glucose concentration AG and the transition curve (history curve) of the glucose concentration corresponding to the AG calculation reference period are displayed on the first display 24A. In this case, the AG display mode is automatically switched from the basic display mode by using the trigger of the fact that the estimated HbAlc concentration has a value of not less than the reference concentration Vsh. Specifically, the processor 21 of the user terminal 2 judges whether or not the value of the estimated HbAlc concentration is not less than the reference concentration Vsh every time when the estimated HbAlc concentration is calculated. In this procedure, if the negative judgment is made (estimated HbAlc concentration < Vsh), the display mode is maintained to be the basic display mode. On the other hand, if the affirmative judgment is made (estimated HbAlc concentration ≥ Vsh), then the instruction is outputted from the processor 21 to the display 24, and the display mode is switched from the basic display mode to the AG display mode. Accordingly, it is easy to grasp the average glucose concentration AG as the basis for determining the estimated HbAlc concentration and the transition of the glucose concentration corresponding to the AG calculation reference period, when the estimated HbAlc concentration is not less than the reference concentration Vsh, which is convenient.

The respective displays 24A, 24B of the user terminal 2 are contact type touch panels. The processor 21 of the user terminal 2 displays an icon A, an icon B, and an icon C in a predetermined area of the second display 24B in the AG display mode. The icons A, B, C are icons corresponding to the analysis display mode, the second analysis display mode, and the notice display mode respectively as described later on. If the processor 21 detects that the user touches any one of the icons, the control is performed to switch the display mode of the user terminal 2 to the display mode corresponding to the icon selected by the user. It is not necessarily indispensable to provide the touch panel as described above. It is also allowable to switch the display mode by using the trigger of the acceptance of the input operation of the operation unit 23 by the user, for example, such that the user depresses a decision button while adjusting a cursor to any one of the icons by operating a cursor button.

<<Analysis display mode>>

Figs. 19 and 20 illustrate the analysis display mode of the user terminal 2. Fig. 19 is referred to as "analysis basic screen" in the analysis display mode, and Fig. 20 is referred to as "detailed information screen" in the analysis display mode. As described above, if the processor 21 detects that the user touches the icon A in the AG display mode, the first display 24A is switched into the screen shown in Fig. 19. The display mode of the second display 24B, which is provided in the analysis display mode, is the same as or equivalent to, for example, that provided in the basic display mode. In the analysis basic screen in the analysis display mode shown in Fig. 19, the area (hereinafter referred to as "high blood sugar area"), in which the glucose concentration is higher than a predetermined threshold value Vsh2 in relation to the glucose concentration corresponding to the AG calculation reference period displayed on the first display 24A in the AG display mode, is displayed, for example, by being painted out so that the area is subjected to the highlighting display thereby. Accordingly, the period, in which the glucose concentration was especially high in the past, can be grasped at a glance. The specified numerical value of the threshold value Vsh2 for defining the high blood sugar area can be appropriately set depending on, for example, the specification of the user terminal 2.

Further, in the analysis display mode, if the processor 21 detects that the user touches any portion of the high blood sugar area on the first display 24A, the detailed information screen of the timing (period) corresponding to the portion touched by the user is displayed on the first display 24A. The timing corresponding to the portion touched by the user means the timing on the time axis of the transition graph of the glucose concentration. The detailed information screen will be explained below with reference to Fig. 20.

As shown in Fig. 20, the time and date, the event type, and the glucose concentration are displayed as a set of information in time series on the detailed information screen. In the drawing, the event of "MEAL" means the "meal", and the event of "EXER" means the "exercise". The indication of "11/20 MEAL 280" means the fact that the glucose concentration after the meal on November 20 was 280 mg/dL. The indication of "11/20 EXER 110" means the fact that the glucose concentration after the exercise on November 20 was 110 mg/dL. The event type is not limited to "MEAL" (meal) and "EXER" (exercise) as described above. For example, it is possible to appropriately add any event such as "BATH" (bath) and the like.

Every time when the user experiences (executes) various events, the user operates the operation unit 23 so that the operation corresponds to each of the event types. For example, the user terminal 2 may be provided with event buttons (for example, meal button and exercise button) corresponding to the respective events, and the user may depress the button corresponding to the event. Alternatively, every time when the user experiences various events, the button operation based on the use of a cursor button and a decision button may be accepted from the user. If the processor 21 of the user terminal 2 detects the button operation performed by the user in relation to the event, then the event history information is prepared such that the time and date information concerning the execution of the button operation, the glucose concentration at that time, and the event type are correlated with each other, and the event history information is stored in the event history storage unit 34 of the auxiliary storage device 27.

On the other hand, if the switching request of the user to switch the analysis basic screen in the analysis display mode to the detailed information screen is accepted (i.e., if the user touches any portion of the high blood sugar area), the processor 21 of the user terminal 2 reads the event history information in a predetermined period including the timing corresponding to the portion touched by the user from the event history storage unit 34 of the auxiliary storage device 27. Subsequently, the processor 21 allows the first display 24A to display the event history information read from the event history storage unit 34 in time series.

In general, the glucose concentration rises by ingesting the meal, and the glucose concentration lowers by performing the exercise. The various types of invents which remarkably cause the fluctuation of the glucose concentration as described above and the detailed data of the glucose concentration which corresponds to the event can be provided for the user in a comparable manner on the detailed information screen of the analysis display mode. Therefore, the user can analyze the cause of the increase in the speculated HbAlc value by himself/herself on the basis of the detailed information data corresponding to the past high blood sugar area. Accordingly, it is possible to provide the information useful for the diabetes care of the user in a convenient manner.

<<Second analysis display mode>>

Fig. 21 illustrates the second analysis display mode of the user terminal 2. If the processor 21 of the user terminal 2 detects that the user touches the icon B in the AG display mode, the first display 24A is switched into the screen shown in Fig. 21. In the second analysis display mode shown in Fig. 21, the measurement data of the glucose concentration which can be judged as FPG (fasting blood glucose (fasting plasma glucose) level) in the AG calculation reference period or the measurement data of the glucose concentration in a state close to OGTT is displayed by plots. Specifically, the processor 21 specifies the measurement data of the glucose concentration which can be judged to fall under FPG or OGTT, on the basis of, for example, whether or not the transition pattern of the glucose concentration is approximate or coincident with the previously prescribed transition pattern corresponding to FPG or OGTT, and the elapsed time after the meal button is depressed by the user. The processor 21 allows the specified measurement data to be displayed by plots on the graph of the glucose concentration on the first display 24A. Accordingly, the user can easily grasp FPG and OGTT in the past.

<<Notice display mode>>

Fig. 22 illustrates the notice display mode of the user terminal 2. In the notice display mode, the information to be noticed to the user is displayed. If the processor 21 detects that the user touches the icon C in the AG display mode, the switching is performed to the display mode shown in Fig. 22. In the notice display mode, the transition curve of the glucose concentration and the present glucose concentration are displayed on the first display 24A in the same manner as in the basic display mode, and the second display 24B is switched into the notice screen shown in the drawing. In this situation, the estimated HbAlc concentration is not less than 6.5% which is the reference concentration Vsh. Therefore, for example, a message of "Recommend you to carry out measurement of fasting blood sugar level or oral glucose tolerance test in medical facility" is displayed on the notice screen to hasten the user to carry out the examination. The message described above may be outputted as voice from a speaker (not shown) of the user terminal 2 together with the screen display or in place of the screen display as described above. Further, the processor 21 of the user terminal 21 allows the letters of "FPG measuring mode" and "OGTT measuring mode" to be displayed under the notice message directed to the user as described above, on the notice screen displayed on the second display 24B. Subsequently, when the operation button concerning the operation unit 23 is operated by the user, or the display portion of "FPG measuring mode" or "OGTT measuring mode" of the touch panel is touched by the user, then the processor 21 starts the execution of the control concerning the measuring mode selected by the user.

<FPG measuring mode>

If the user selects the "FPC measuring mode" in the notice display mode, the processor 21 of the user terminal 2 starts the execution of the FPG measurement control by using the selection as the trigger. Fig. 23 shows a flow chart illustrating the procedure of an FPG measurement control routine according to this embodiment. This control routine is executed by loading the program stored in the auxiliary storage device 27 to the main storage device 22 by the processor 21 of the user terminal 2.

The FPG measurement control is the control in which FPG (fasting blood glucose level) is measured by using the CGM measuring device 1. When this control routine is started, the processor 21 allows the second display 24B to display the FPG initial screen in Step S501. For example, as shown in Fig. 24, messages of, for example, "Measurement of FPG (fasting blood glucose level) is started", "Did you finish your meal?", "Yes", and "No" are displayed on the second display 24B (FPG initial screen).

The user selects (determines) "Yes" or "No" by operating the operation unit 23 by using the operation button or the touch panel. In Step S502, if the processor 21 detects that "No" is selected by the user, the processor 21 allows the second display 24B to display a message of "Take your meal" for a certain period of time. The processor 21 accepts the operation of the operation unit 23 performed by the user by displaying, for example, the FPG initial screen shown in Fig. 24 after the elapse of the certain period of time.

In Step S502, if the processor 21 detects that "Yes" is selected by the user, the routine proceeds to Step S503. If the operation of the operation unit 23 by the user is not accepted even when the predetermined period elapses in Step S502, then this routine may be once completed to return to the basic display mode described above.

In Step S503, the processor 21 receives the present time and date information from the clock 28 to calculate the test finish time of FPG (hereinafter referred to as "FPG test finish time"). The test finish time is the time after the elapse of a period of time (hereinafter referred to as "FPG time required (duration)") required for the examination of FPG after the start of FPG. The FPG test finish time can be calculated, for example, as the time after the elapse of the FPG time required on the basis of the present time. In this embodiment, the FPG time required is set within a range of, for example, about 8 to 12 hours. However, the present invention is not limited to this range. The following procedure is also available as a modified embodiment. That is, for example, a message of "Input time at which you finally finished your meal" may be displayed on the FPG initial screen on the second display 24B to accept the time input by the operation of the operation unit 23 by the user. In this procedure, it is appropriate that the scheduled test finish time of FPG is determined by adding the FPG time required to the time inputted by the user (latest meal time). In this modified embodiment, if the time input operation is not accepted from the user even when the routine waits for not less than a certain period of time with the FPG initial screen, then this control routine may be once completed.

Subsequently, in Step S504, the processor 21 allows the second display 24B to display the FPG continuing screen. As shown in Fig. 25, for example, messages of "FPG (fasting blood glucose level) is being measured" and "Test will finish at time of xx o'clock xx minutes. Do not take your meal until finish" are displayed on the FPG continuing screen. In this procedure, the time, which is calculated in Step S503, is displayed as the test finish time. Subsequently, after the elapse of a certain period of time, the routine proceeds to the process of Step S505.

In Step S505, the processor 21 receives the present time and date information from the clock 28 to judge whether or not the FPG test finish time comes. If it is judged in this step that the FPG test finish time does not come yet, the routine returns to Step S504. Subsequently, after a certain period of time elapses, the routine proceeds to the judging process in Step S505 again. If it is judged in Step S505 that the FPG test finish time has already come, the routine proceeds to Step S506.

In Step S506, the processor 21 reads the latest measurement data of the glucose concentration from the measurement data storage unit 30 of the auxiliary storage device 27, and the FPG result output screen to output as FPG is displayed on the second display 24B. For example, as shown in Fig. 26, the processor 21 allows the second display 24B to display messages of "Measurement of FPG (fasting blood glucose level) is completed" and "FPG is xx mg/dL" (FPG result output screen). In this step, if obtained FPG (fasting blood glucose level) is less than 110 mg/dL, it is also allowable to display a message of "No problem is found in value of FPG (fasting blood glucose level)". If FPG is within a range of not less than 110 mg/dL to less than 126 mg/dL, it is also allowable to display a message of "Recommend you to reexamine FPG in medical facility, because you highly possibly fall under borderline diabetes". If FPG is not less than 126 mg/dL, it is also allowable to display a message of "Strongly recommend you to reexamine FPG in medical facility, because you highly possibly fall under diabetes".

When the process of this step is completed, this control routine is once completed. The processor 21 may restore the display mode of the user terminal 2 to the basic display mode upon the completion of this control routine. The voice output may be simultaneously performed from a speaker (not shown) of the user terminal 2 for the display messages to be displayed on the display 24 as explained in relation to this control routine.

<OGTT measuring mode>

Next, the "OGTT measuring mode" will be explained. If the "OGTT measuring mode" is selected by the user in the notice display mode, the processor 21 starts the execution of the OGTT measurement control. Fig. 27 shows a flow chart illustrating the procedure of an OGTT measurement control routine according to this embodiment. This control routine is executed by loading the program stored in the auxiliary storage device 27 to the main storage device 22 by the processor 21 of the user terminal 2. The OGTT measurement control is the control to carry out the measurement of OGTT by using the CGM measuring device 1. When this control routine is started, the processor 21 of the user terminal 2 allows the second display 24B to display the OGTT initial screen in Step S601. As shown in Fig. 28, for example, the processor 21 allows the second display 24B to display messages of, for example, "Oral glucose tolerance test is started", "Yes", and "No" (OGTT initial screen).

The user selects (determines) "Yes" or "No" by operating the operation unit 23 by means of the operation button or the touch panel. If the processor 21 detects that "No" is selected by the user in Step S602, the routine returns to Step S601. In this case, the OGTT initial screen shown in Fig. 28 is displayed to accept the operation of the operation unit 23 by the user. If the processor 21 detects that "Yes" is selected by the user in Step S602, the routine proceeds to Step S603. If the operation of the operation unit 23 by the user is not accepted even when a predetermined period elapses in Step S602, then this routine may be once completed to return to the basic display mode described above.

In Step S603, it is judged whether or not glucose (grape sugar) has been already orally administered. Specifically, the processor 21 allows the second display 24B to display a message of "Glucose already orally administered?". The user selects (determines) "Yes" or "No" by operating the operation unit 23 by means of the operation button or the touch panel. If the processor 21 detects that "No" is selected by the user in Step S603, then the processor 21 allows the second display 24B to display a message of "Orally administer glucose" for a certain period of time, and then the message of "Glucose already orally administered?" is displayed again in Step S603. After that, the judging process is performed as described above. If the processor 21 detects that "Yes" is selected by the user, then it is judged that glucose has been already orally administered, and the routine proceeds to Step S604.

In Step S604, the processor 21 gives the instruction to the clock 28 to start the measurement of the elapsed time $\Delta Tm$. The processor 21 receives the present time and date information from the clock 28 to calculate the time after the elapse of 2 hours (120 minutes) therefrom as the scheduled finish time of OGTT. Subsequently, in Step S605, the processor 21 allows the second display 24B to display the OGTT continuing screen. As shown in Fig. 29, for example, messages of "Oral glucose tolerance test is being continued" and "Test will finish at time of xx o'clock xx minutes. Do not take your meal until finish" are displayed on the OGTT continuing screen. In this procedure, the time, which is calculated in Step S604, is displayed as the test finish time. Subsequently, after a certain period of time elapses, the processor 21 proceeds to the process of Step S606.

In Step S606, the processor 21 judges whether or not the elapsed time $\Delta Tm$ elapses the OGTT time required (duration) $\Delta Tmb$. The OGTT time required $\Delta Tmb$ is the time required for the oral glucose tolerance test. In this case, the OGTT time required $\Delta Tmb$ is set to 2 hours (120 minutes). However, the present invention is not necessarily limited thereto. If the negative judgment is made in this step ($\Delta Tm < \Delta Tmb$), the routine returns to Step S605. After a certain period elapses, the routine proceeds to the judging process in Step S606 again. If the affirmative judgment is made in Step S606 ($\Delta Tm \geq \Delta Tmb$), i.e., if it is judged that the elapsed time $\Delta Tm$ elapses the OGTT time required $\Delta Tmb$ (120 minutes), then the routine proceeds to Step S607.

In Step S607, the processor 21 reads the measurement data of the glucose concentration as obtained when 30 minutes, 60 minutes, 90 minutes, and 120 minutes elapse after the start of the oral glucose tolerance test, from the measurement data storage unit 30 of the auxiliary storage device 27. The processor 21 allows the read measurement data to be displayed as the OGTT result output screen on the second display 24B. As shown in Fig. 30, as for the OGTT result output screen, for example, a message of "Oral glucose tolerance test is completed" and measurement results of "30 minutes after loading: xx mg/dL, 60 minutes after loading: xx mg/dL, 90 minutes after loading: xx mg/dL, 120 minutes after loading: xx mg/dL" are displayed on the second display 24B.

In this step, if the glucose concentration at 120 minutes after the loading (2h-PG value) is less than 140 mg/dL, the processor 21 may allow the second display 24B to display a message of "No problem is found in result of oral glucose tolerance test". If the 2h-PG value is within a range of not less than 140 mg/dL to less than 200 mg/dL, the processor 21 may allow the second display 24B to display a message of "Recommend you to reexamine oral glucose tolerance test in medical facility, because you highly possibly fall under borderline diabetes". If the 2h-PG value is not less than 200 mg/dL, the processor 21 may allow the second display 24B to display a message of "Strongly recommend you to reexamine oral glucose tolerance test in medical facility, because you highly possibly fall under diabetes". In another mode in this step, for example, only the glucose concentration at 120 minutes after the loading (2h-PG value) may be displayed.

When the process of this step is completed, this control routine is once completed. The processor 21 may restore the display mode of the user terminal 2 to the basic display mode upon the completion of this control routine. The voice output may be simultaneously performed from a speaker (not shown) of the user terminal 2 for the display messages to be displayed on the display 24 as explained in relation to this control routine.

Second Embodiment

Next, a second embodiment according to the present invention will be explained. The first embodiment described above is illustrative of such a mode that the concerning calibration curve data, which is provided to update the inherent calibration curve data SCu stored in the second calibration curve storage unit 32 of the auxiliary storage device 27 of the user terminal 2, is incorporated into the measuring apparatus 5, or the concerning calibration curve data is delivered from the server apparatus 4 provided independently therefrom. In contrast thereto, in the second embodiment, the processor 21 of the user terminal 2 judges whether or not the inherent calibration curve data SCu, which is stored in the auxiliary storage device 27 of itself, is proper on the basis of the glucose concentration (measured value of the first biological component) acquired from the CGM measuring device 1 by the user terminal 2 or the actually measured HbAlc concentration acquired, for example, by the actual measurement data input operation performed by the user. If it is judged that the inherent calibration curve data SCu is not proper, the inherent calibration curve data SCu is updated.

Fig. 31 shows a system arrangement of an information providing system S according to the second embodiment. The CGM measuring device 1 and the measuring apparatus 5, which are shown in the drawing, are equivalent to those described in the first embodiment. The basic form or mode of the user terminal 2 according to the second embodiment is common to that of the first embodiment. The common feature is provided such that the Glu information and the HbAlc information can be displayed on the display 24. The display form or mode of the user terminal 2, which has been explained in the first embodiment, is also applicable to the second embodiment.

In the user terminal 2 of this embodiment, the common calibration curve data SCp (see Fig. 7) is stored in the second calibration curve storage unit 32 of the auxiliary storage device 27 in addition to the inherent calibration curve data SCu (see Fig. 4). The inherent calibration curve data SCu and the common calibration curve data SCp are as explained in the first embodiment. In this embodiment, the common calibration curve data SCp is constructed by a large number of pieces of actually measured pair data composed of combinations of blood sugar levels measured from specimens of a plurality of examinees (for example, patients in the medical facility) and measured values of glycohemoglobin (HbA1c) which are actually measured.

<Third control>

Fig. 32 shows a flow chart illustrating the procedure of a third control routine. The third control routine is executed by loading the program stored in the auxiliary storage device 27 to the main storage device 22 every predetermined period by the processor 21 of the user terminal 2.

In Step S701, the processor 21 of the user terminal 2 accesses the AG value storage unit 53 of the auxiliary storage device 27 to judge whether or not any new average glucose concentration AG is added to the AG value storage unit 35. If the affirmative judgment is made in this step, the processor 21 detects that the AG value storage unit 35 newly acquires the average glucose concentration AG as the first measured value, and the routine proceeds to Step S702. On the other hand, if the negative judgment is made in this step, this control routine is once completed. In Step S702, the processor 21 accesses the second calibration curve storage unit 32. The processor 21 substitutes the average glucose concentration AG acquired as described above as the first measured value for the inherent calibration curve data SCu and the common calibration curve data SCp stored in the second calibration curve storage unit 32 respectively. Accordingly, the processor 21 derives the inherent calibration curve corresponding value Vd1 and the common calibration curve corresponding value Vd2 respectively. The inherent calibration curve corresponding value Vd1 and the common calibration curve corresponding value Vd2 are as explained in the first embodiment.

In Step S703, the processor 21 judges whether or not the corresponding value deviation amount $\Delta Vd$ exceeds a predetermined first reference amount A1. The corresponding value deviation amount $\Delta Vd$ is defined as the absolute value of the difference between the inherent calibration curve corresponding value Vd1 and the common calibration curve corresponding value Vd2 ($\Delta Vd = |Vd1 - Vd2|$). If the negative judgment is made in this step ($\Delta Vd \leq A1$), it is judged that the corresponding value deviation amount $\Delta Vd$ is maintained at a sufficiently small value, and any problem does not arise in the inherent calibration curve data SCu stored in the second calibration curve storage unit 32 of the user terminal 2. In this case, this control routine is completed as it is. Alternatively, the processor 21 may perform the following procedure. That is, a message of "Normal state is confirmed" is displayed, for example, on the second display 24B, and/or the message is outputted as voice from a speaker (not shown) of the user terminal 2. After that, this control routine is completed.

On the other hand, if the affirmative judgment is made in Step S703 ($\Delta Vd > A1$), the routine proceeds to Step S704. In Step S704, it is further judged whether or not the corresponding value deviation amount $\Delta Vd$ exceeds a predetermined second reference value A2. The second reference value A2 is a threshold value which is set to a value larger than the first reference value A1. If the negative judgment is made in this step ($\Delta Vd \leq A2$), the routine proceeds to Step S705. If the affirmative judgment is made ($\Delta Vd > A2$), the routine proceeds to Step S706. If the routine proceeds to Step S705, it is meant that the corresponding value deviation amount $\Delta Vd$ exceeds the first reference value A1 and the corresponding value deviation amount $\Delta Vd$ is not more than the second reference value A2. In this case, the significant difference is acknowledged between the inherent calibration curve corresponding value Vd1 and the common calibration curve corresponding value Vd2. It is judged that the significant difference is caused by the change of the physical constitution of the user who uses the user terminal 2.

Accordingly, in Step S705, the processor 21 outputs the measured value request signal to require the input of the measurement result of the biological component, i.e., the HbAlc concentration in this case which is either the average glucose concentration and the HbAlc concentration and which is not acquired by the AG value storage unit 35 in Step S701. When the measured value request signal is outputted by the processor 21, then the first notice information is outputted from the user terminal 2 to the user, and the third inherent calibration curve data update flag is set to ON ($F \leftarrow ON$) .

The first notice information is, for example, a message of "Actually measure glycohemoglobin concentration in designated medical facility and input measurement result" displayed on the second display 24A. The switching of the third inherent calibration curve data update flag from OFF to ON provides the trigger to perform the third inherent calibration curve data update control routine as described later on. If the process of this step is completed, this control routine is once completed. On the other hand, if the routine proceeds to the process of Step S706, it is meant that the corresponding value deviation amount $\Delta Vd$ exceeds the second reference value A2. In this case, the significant difference is acknowledged between the inherent calibration curve corresponding value Vd1 and the common calibration curve corresponding value Vd2. It is judged that the significant difference is caused by the external factor error such as the malfunction, the abnormality or the like of the CGM measuring device 1 (including the glucose sensor 11). In this case, in Step S706, the second notice information is outputted from the user terminal 2 to the user. The second notice information is, for example, a message of "CGM measuring device and/or sensor may be possibly abnormal, so exchange sensor with new one or perform maintenance for CGM measuring device" displayed on the second display 24B. When the process of this step is completed, this control routine is once completed. As for the first notice information and the second notice information as described above, the respective messages as described above may be outputted as voice, for example, from a speaker (not shown) of the user terminal 2.

Next, an explanation will be made about the specified process of the third inherent calibration curve data update control routine. Fig. 33 shows a flow chart illustrating the procedure of the third inherent calibration curve data update control routine according to this embodiment. This routine is also executed by the processor 21 of the user terminal 2 in the same manner as the third control routine. This control routine is repeatedly executed every predetermined period if the third inherent calibration curve data update flag is in a state of ON. The user, who sees the message of the first notice information displayed on the second display 24B of the user terminal 2, measures the HbAlc concentration of himself/herself by using the measuring apparatus 5 in the medical facility. The actually measured HbAlc concentration, which is obtained as described above, is inputted by means of the button input operation by using the operation unit 23, and the actually measured HbAlc concentration is stored as the second measured value in the HbAlc value storage unit 36. The HbAlc value storage unit 36 stores the actually measured HbAlc concentration acquired by at least any means of the input operation of the operation unit 23 by the user, the transportable recording medium, and the communication from any external apparatus. The external apparatus is herein exemplified, for example, by the server apparatus 4 and the measuring apparatus 5. The transportable recording medium can be exemplified, for example, by a USB (Universal Serial Bus) flash memory and a flash memory card.

In Step S801, the processor 21 of the user terminal 2 accesses the HbAlc value storage unit 36. Subsequently, the measured value request signal (first notice information) is outputted in the third control described above, and then it is judged whether or not the actually measured HbAlc concentration is newly added (updated) in the HbAlc value storage unit 36. If it is judged in this step that the actually measured HbAlc concentration is not newly updated, this routine is completed as it is. In this case, this control routine is executed again after a predetermined period elapses. On the other hand, if it is judged in this step that the actually measured HbAlc concentration is newly updated, the processor 21 detects the fact that the HbAlc value storage unit 36 newly acquires the actually measured HbAlc concentration as the second measured value. In this case, the processor 21 proceeds to Step S802. In Step S802, the processor 21 of the user terminal 2 updates the inherent calibration curve data SCu stored in the second calibration curve storage unit 32 by using, as the actually measured pair data, the pair of the actually measured HbAlc concentration (second measured value) and the average glucose concentration AG (first measured value) acquired in Step S701 in the third control shown in Fig. 32. When the process of this step is completed, this control routine is once completed.

If it is judged in Step S704 of the third control described above that the corresponding value deviation amount ΔVd exceeds the second reference value A2, the processor 21 may carry out the correcting process in relation to the average glucose concentration AG acquired as the first measured value by the AG value storage unit 35 of the auxiliary storage device 27. The correcting process is exemplified, for example, by the abnormal value removing process (first modified embodiment) and the reference period shortening process (second modified embodiment) as explained in the first embodiment. The contents of the respective processes are the same as or equivalent to the process contents described in the first embodiment, any detailed explanation of which is omitted. Subsequently, the processor 21 of the user terminal 2 carries out the correcting process such as the abnormal value removing process, the reference period shortening process or the like to calculate the corrected average blood sugar level AG' (corrected measured value of the first biological component). Based on the calculated result, the processor 21 updates the inherent calibration curve data SCu on the basis of the corrected measured value of the first biological component (first measured value) and the actually measured HbAlc concentration (second measured value). Any one of the abnormal value removing process and the reference period shortening process may be selectively carried out, or both of them may be sequentially carried out.

<Fourth control>

Next, the fourth control in the second embodiment will be explained. The fourth control is the control which corresponds to the second control in the first embodiment. Fig. 34 shows a flow chart illustrating the procedure of a fourth control routine. The fourth control routine is executed by loading the program stored in the auxiliary storage device 27 to the main storage device 22 every predetermined period by the processor 21 of the user terminal 2.

When this control routine is started, the processor 21 of the user terminal 2 firstly accesses the HbAlc value storage unit 36 of the auxiliary storage device 27 in Step S901 to judge whether or not any actually measured HbAlc concentration is newly added (updated). If the affirmative judgment is made in this step, it is detected that the HbAlc value storage unit 36 newly acquires the actually measured HbAlc concentration as the first measured value, and the routine proceeds to Step S902. On the other hand, if the negative judgment is made in this step, this control routine is once completed.

Subsequently, in Step S902, the processor 21 of the user terminal 2 accesses the second calibration curve storage unit 32. The processor 21 substitutes the actually measured HbAlc concentration acquired as described above as the first measured value for the common calibration curve data SCp and the inherent calibration curve data SCu stored in the second calibration curve storage unit 32 respectively to derive the inherent calibration curve corresponding value Vd1' and the common calibration curve corresponding value Vd2' respectively.

Subsequently, in Step S903, the processor 21 judges whether or not the corresponding value deviation amount ΔVd' exceeds a predetermined first reference amount A1'. The corresponding value deviation amount ΔVd' is defined as the absolute value of the difference between the inherent calibration curve corresponding value Vd1' and the common calibration curve corresponding value Vd2' ($\Delta Vd' = |Vd1' - Vd2'|$). If the negative judgment is made in this step ($\Delta Vd' \leq A1'$), it is judged that the corresponding value deviation amount ΔVd' is maintained at a sufficiently small value, and any problem does not arise in the inherent calibration curve data SCu stored in the second calibration curve storage unit 32 of the user terminal 2. In this case, this control routine is completed as it is. Alternatively, the processor 21 may perform the following procedure. That is, a message of "Normal state is confirmed" is displayed, for example, on the second display 24B, and/or the message is outputted as voice from a speaker (not shown) of the user terminal 2. After that, this control routine is completed.

On the other hand, if the affirmative judgment is made in Step S903 ($\Delta Vd' > A1'$), the routine proceeds to Step S904. In Step S904, it is further judged whether or not the corresponding value deviation amount ΔVd' exceeds a predetermined second reference value A2'. The second reference value A2' is a threshold value which is set to a value larger than the first reference value A1'. If the negative judgment is made in this step ($\Delta Vd' \leq A2'$), the routine proceeds to Step S905. On the other hand, if the affirmative judgment is made in this step ($\Delta Vd' > A2'$), the routine proceeds to Step S906. If the routine proceeds from Step S904 to Step S905, it is judged that the significant difference is acknowledged between the inherent calibration curve corresponding value Vd1' and the common calibration curve corresponding value Vd2'. It is judged that the significant difference is caused, for example, by the change of the physical constitution of the user. Accordingly, the processor 21 reads the average glucose concentration AG (unacquired measured value) as the second measured value which is any one of the average glucose concentration AG and the HbAlc concentration and which is not acquired as the first measured value by the HbAlc value storage unit 36 in Step S901, from the AG value storage unit 35 of the auxiliary storage device 27, and the average glucose concentration AG (unacquired measured value) is acquired as the second measured value. In this procedure, it is preferable that the AG calculation reference period, which is provided for the average glucose concentration AG acquired from the AG value storage unit 35, includes the measurement time and date of the actually measured HbAlc concentration detected in Step S901. When the process of this step is completed, the routine proceeds to Step S907.

On the other hand, if the routine proceeds from Step S904 to Step S906, it is judged that the significant difference is acknowledged between the inherent calibration curve corresponding value Vd1' and the common calibration curve corresponding value Vd2'. It is judged that the significant difference is caused by the external factor error such as the malfunction or the abnormality of the measuring apparatus 5. Accordingly, in this step, the third notice information is outputted from the user terminal 2 to the user. The third notice information is, for example, a message of "Measuring apparatus may possibly involve abnormality or malfunction. Recommend you to measure glycohemoglobin again" displayed on the second display 24B and/or a voice output of the message outputted from a speaker (not shown) of the user terminal 2.

In Step S907, the processor 21 of the user terminal 2 updates the inherent calibration curve data SCu stored in the auxiliary storage device 27 of the user terminal 2 on the basis of the pair of the average glucose concentration AG which is read as the second measured value from the AG value storage unit 35 and the actually measured HbAlc concentration which is acquired as the first measured value by the HbAlc value storage unit 36 in Step S901. When the process of this step is completed, this control routine is once completed.

In the second embodiment described above, it is also allowable that the control contents of the third control and the fourth control are appropriately combined and executed. For example, the following procedure may be adopted. That is, if any one of the average glucose concentration AG and the actually measured HbAlc concentration is acquired as the first measured value, and the corresponding value deviation amount exceeds the first reference amount, then the user terminal 2 acquires the other measured value as the second measured value, and the inherent calibration curve data SCu is updated by using the pair of the second measured value and the first measured value having been already acquired.

## Third Embodiment

Next, a third embodiment according to the present invention will be explained. The system arrangement of an information providing system S according to this embodiment is the same as or equivalent to that of the first embodiment (see Fig. 1). In this embodiment, an explanation will be made about the "simultaneous display mode" prepared as one display mode of the user terminal 2.

When the user terminal 2 accepts the operation of the user, for example, via the operation unit 23, the display mode is changed to the simultaneous display mode. For example, any one of the buttons of the operation unit 23 may be allotted to a simultaneous display mode button for selecting the simultaneous display mode. The simultaneous display mode is such a display mode that a plurality of index values, which reflect the blood sugar states of the user, are simultaneously displayed on the display 24. The plurality of index values are, for example, the first index value which is provided to evaluate the metabolic result (result of the metabolism) of glucose for a certain period of time and the second index value which is provided to evaluate the ability of glucose metabolism. The index value which reflects the long term blood sugar state, for example, the HbAlc concentration which is the index value for reflecting the metabolic result of glucose for past several months is adopted as the first index value. On the other hand, the second index value is exemplified, for example, by FPG and OGTT. In this embodiment, at least any one of FPG and OGTT is adopted as the second index value. However, it is not excluded that any index other than FPG and OGTT is adopted as the second index value.

In recent years, it is suggested that the HbAlc concentration should be considered when it is judged whether or not the disease is the borderline diabetes in the same manner as FPG and OGTT, and the progress in further from the "borderline type" to the "diabetes type" can be accurately predicted by combining the second index value such as FPG, OGTT or the like and the first index value such as the HbAlc concentration. For example, in relation to the condition to fulfill whether or not FPG as the second index value is within a range of not less than 110 mg/dL and less than 126 mg/dL and the condition to fulfill whether or not the HbAlc concentration as the first index value is within a relatively high range (5.7 to 6.4%), it is reported that the probability of appearance of diabetes in future is raised when both of the conditions are fulfilled as compared with when any one of the conditions is fulfilled. Therefore, it is possible to accurately predict whether or not the risk of progress in further from the "borderline type" to the "diabetes type", i.e., the appearance risk of diabetes is high, by combining the first index value and the second index value in relation to the blood sugar state.

In view of the above, the user terminal 2 allows the second display 24B to simultaneously display both of the first index value and the second index value in relation to the blood sugar state when the "simultaneous display mode" is selected. Fig. 35 illustrates the simultaneous display mode of the user terminal 2. Fig. 36 shows a flow chart illustrating the process flow adopted when the display mode of the user terminal 2 is the simultaneous display mode. The processes of the respective steps of this flow chart are realized by loading the program stored in the auxiliary storage device 27 to the main storage device 22 by the processor 21 of the user terminal 2. As for this flow chart, an explanation will be made as exemplified by a case in which FPG is adopted as the second index value.

In Step S1001, the processor 21 reads the latest value of the average glucose concentration AG stored in the AG value storage unit 53 of the auxiliary storage device 27. Subsequently, in Step S1002, the HbAlc concentration as the first index value is acquired on the basis of the average glucose concentration AG read in Step S1001. Specifically, the processor 21 acquires the estimated HbAlc concentration by substituting the average glucose concentration AG read in Step S1001 for the inherent calibration curve data SCu stored in the second calibration curve storage unit 32 of the auxiliary storage device 27. Further, the processor 21 accesses the measured value storage unit 30 of the auxiliary storage device 27 to read FPG. The CGM measuring device 1 measures the blood sugar level continuously over a measurement period of about several days to several weeks. Therefore, FPG is also stored in the measured value storage unit 30. Accordingly, the processor 21 reads the measurement data of the glucose concentration corresponding to FPG from the measured value storage unit 30. Accordingly, the processor 21 can acquire the HbAlc concentration and FPG.

FPG is the glucose concentration measured at fasting before the meal (for example, 8 to 12 hours after the last meal). A meal button, which is to be depressed when the meal is ingested, is allotted to any one of the buttons of the operation unit 23 of the user terminal 2. Every time when the meal button is depressed, the processor 21 allows the event history storage unit 34 to store the event information of "meal" as the event history information while being correlated with the time and date information. Accordingly, the processor 21 can acquire the time and date at which the user ingested the meal, by accessing the event history storage unit 34. Therefore, it is possible to acquire FPG, for example, by reading, from the measured value storage unit 30, the blood sugar level Glu after the elapse of a predetermined period of time (for example, about 8 to 12 hours) after the time and date at which the meal was ingested. Alternatively, the user terminal 2 may be provided with a function to automatically detect FPG. For example, when the glucose concentration is lowered to a value lower than a predetermined threshold value after the glucose concentration, which changes in a time-dependent manner, arrives at a peak value (maximum value), and this state is thereafter maintained for not less than a predetermined period of time (for example, about 8 to 12 hours), then the measured value of the glucose concentration, which is obtained during the concerning period, may be adopted as FPG. For example, the processor 21 can acquire FPG by accessing the measured value storage unit 30 of the auxiliary storage device 27 and reading the measurement data in which the condition as described above holds. Specifically, the processor 21 specifies, as the FPG candidate data, the measurement data which exhibits the value lower than the predetermined threshold value after the peak value (maximum value), from the measurement data of the glucose concentration stored in the measured value storage unit 30. The processor 21 judges whether or not the values of the respective pieces of measurement data measured during the predetermined period of time are less than the threshold value, on the basis of the measurement time and date of the FPG candidate data. If the values of the respective pieces of measurement data are less than the threshold value, the value of the FPG candidate data may be formally acquired as FPG.

Subsequently, in Step S1003, the processor 21 judges the magnitude correlation with respect to a predetermined threshold value set for each of the index values of the HbAlc concentration (first index value) and FPG (second index value) acquired in Step S1002. In this case, for example, the threshold value with respect to the HbAlc concentration is set to 5.7% and 6.4%, and the threshold value with respect to FPG is set to 110 mg/dL and 126 mg/dL. The processor 21, which executes this step, functions as the judging unit 39. However, the threshold values can be appropriately changed.

In Step S1004, in order that the judgment results, which relate to the estimated HbAlc concentration as the first index value and FPG as the second index value respectively, are displayed so that they can be specified, the display area of the second display 24B is divided into a plurality of areas to provide such a display state that a specified area, which is included in the display area and which conforms to the judgment result for each of the index values, can be distinguished from the other area. This simultaneously means that the acquisition results, which relate to the estimated HbAlc concentration (first index value) and FPG (second index value) acquired in Step S1002, are simultaneously displayed on the second display 24B.

As shown in Fig. 35, the display area of the second display 24B is divided into four areas of areas A to D. In this case, the area A is the area in which FPG is less than 110 mg/dL and the estimated HbAlc concentration is less than 5.7%. The area B is the area in which FPG is less than 110 mg/dL and the estimated HbAlc concentration is not less than 5.7% and less than 6.4%. The area C is the area in which FPG is not less than 110 mg/dL and less than 126 mg/dL and the estimated HbAlc concentration is less than 5.7%. The area D is the area in which FPG is not less than 110 mg/dL and less than 126 mg/dL and the estimated HbAlc concentration is not less than 5.7% and less than 6.4%.

A specified example will be described. For example, it is assumed that FPG acquired in Step S1002 is 105 mg/dL and the estimated HbAlc concentration is 6.0%. In this case, the area B falls under the specified area which conforms to the judgment results with respect to the estimated HbAlc concentration and FPG respectively. Accordingly, the processor 21 controls the display mode of the second display 24B so that the specified area B is in a display state capable of being distinguished from the other areas A, C, D. For example, only the area B may be displayed brightly, and the other areas A, C, D may be displayed darkly. However, it is also allowable to adopt any other distinguishing method provided that the method resides in such a mode that the specified area, which conforms to each of the judgment results for the HbAlc concentration and FPG and which is included in the display area of the second display 24B, can be distinguished from the other areas. When the process of Step S1004 is completed, this process flow is completed.

As described above, according to the display method for the display concerning this control, it is possible to simultaneously display, on the display, the HbAlc concentration (first index value) which is useful to grasp the long term metabolic result in relation to glucose in the body fluid and FPG (second index value) which is useful to grasp the metabolic ability for glucose in the body fluid. Therefore, the user can easily grasp in what state the both index values are placed at present. The display area of the display 24 is divided into the plurality of areas, and the specified area, which conforms to the judgment result for each of the index values, is displayed in such a mode that the specified area can be distinguished from the other areas. Therefore, the user can visually and intuitively grasp the degree of the risk of the change from the borderline type to the diabetes in future. In other words, the user can easily predict the probability of the appearance of diabetes. Therefore, the information terminal apparatus (user terminal), which is excellent in convenience of use, can be provided for the user.

<Modified embodiment of simultaneous display mode>

Fig. 37 illustrates, in the second place, the simultaneous display mode of the user terminal 2. In a modified embodiment shown in Fig. 37, FPG and OGTT are adopted as the second index values. This modified embodiment is different from the embodiment described above in that the plurality of index values are adopted as the second index values. When this modified embodiment is applied, it is appropriate that the processor 21 of the user terminal 2 acquires OGTT in addition to the HbAlc concentration and FPG in Step S1002 shown in Fig. 36. More specifically, the processor 21 appropriately reads OGTT stored most recently, from the measured value storage unit 30 of the auxiliary storage device 27.

In this modified embodiment, an OGTT button, which is to be depressed when the oral glucose tolerance test is started, is allotted to any one of the buttons of the operation unit 23 of the user terminal 2. The user depresses the OGTT button at the point in time at which 75 g of grape sugar (glucose) is orally administered. When the processor 21 detects the depression of the OGTT button by the user, the event information of "OGTT" is stored in the event history storage unit 34 as the event history information correlated with the time and date information. The processor 21 of the user terminal 2 accesses the event history storage unit 34 to acquire the time and date information at which the OGTT button was depressed most recently. OGTT can be acquired by reading, from the measured value storage unit 30, the measurement data of the glucose concentration corresponding to the elapse of 120 minutes after the time and date at which the OGTT button was depressed.

In the case of this modified embodiment, in Step S1003 shown in Fig. 36, the processor 21 judges whether or not OGTT is not less than a previously determined threshold value. The threshold value for OGTT is exemplified, for example, by 140 mg/dL and 200 mg/dL. The processor 21 also judges the magnitude correlation between the acquired value and the threshold value for OGTT in addition to the HbAlc concentration and FPG. In Step S1004, the acquired results concerning the acquired HbAlc concentration, FPG, and OGTT are simultaneously displayed on the second display 24B. In the example shown in Fig. 37, the Venn diagram form is adopted as the display mode for the respective indexes on the second display 24B.

At first, the display area of the second display 24B is divided into areas of areas X, Y, Z. The area X is the area corresponding to the judgment result of each of the index values in which FPG is not less than 110 mg/dL and less than 126 mg/dL. The area Y is the area corresponding to the judgment result of each of the index values in which OGTT is not less than 140 mg/dL and less than 200 mg/dL. The area Z is the area corresponding to the judgment result of each of the index values in which the HbAlc concentration is not less than 5.7% and less than 6.4%.

Further, areas xy, yz, zx, xyz are provided at portions at which the areas X to Z are overlapped with each other. The area xy corresponds to the portion in which only the areas X and Y are overlapped with each other. Therefore, the area xy is the area corresponding to the judgment result of each of the index values in which FPG is not less than 110 mg/dL and less than 126 mg/dL, OGTT is not less than 140 mg/dL and less than 200 mg/dL, and the HbAlc concentration is less than 5.7%. The area yz corresponds to the portion in which only the areas Y and Z are overlapped with each other. Therefore, the area yz is the area corresponding to the judgment result of each of the index values in which OGTT is not less than 140 mg/dL and less than 200 mg/dL, the HbAlc concentration is not less than 5.7% and less than 6.4%, and FPG is less than 110 mg/dL. The area zx corresponds to the portion in which only the areas Z and X are overlapped with each other. Therefore, the area zx is the area corresponding to the judgment result of each of the index values in which the HbAlc concentration is not less than 5.7% and less than 6.4%, FPG is not less than 110 mg/dL and less than 126 mg/dL, and OGTT is less than 140 mg/dL. The area xyz corresponds to the portion in which all of the areas X, Y, Z are overlapped with each other. Therefore, the area xyz is the area corresponding to the judgment result of each of the index values in which FPG is not less than 110 mg/dL and less than 126 mg/dL, OGTT is not less than 140 mg/dL and less than 200 mg/dL, and the HbAlc concentration is not less than 5.7% and less than 6.4%.

A specified example will now be described. For example, it is assumed that FPG, which is acquired in Step S1002, is 115 mg/dL, OGTT is 150 mg/dL, and the HbAlc concentration is 6.0%. In this case, the area xyz falls under the specified area which conforms to the judgment results in Step S1003 for the respective index values. Accordingly, in Step S1004, the processor 21 controls the display mode of the second display 24B so that the area xyz, which is included in the display area of the second display 24B, is in such a display state that the area xyz can be distinguished from the other areas. For example, in the example shown in Fig. 37, the plot display is made in the area xyz, and thus the area xyz can be distinguished from the other areas. Accordingly, the user can grasp, visually at a glance, that the present state of each of the index values is the state corresponding to the area xyz. According to this modified embodiment, the plurality of types of index values are adopted as the second index values for reflecting the ability to metabolize glucose in the body fluid. Therefore, it is possible to more accurately provide, to the user, the information in relation to the appearance risk of diabetes in future. In other words, the information, which is useful for the diabetes care for the user, can be provided in a such a form that the convenience is more enhanced for the user.

<Update of inherent calibration curve data SCu>

Next, an explanation will be made about the update of the inherent calibration curve data SCu stored in the user terminal 2 in this embodiment. Fig. 38 shows a flow chart illustrating the process flow of a first calibration curve update routine according to this embodiment. An explanation will now be made about the exemplary control to be performed when the server apparatus 4 receives the user transmission data including the information in relation to the average glucose concentration AG from the user terminal 2 concerning any user. The first calibration curve update routine is repeatedly executed very predetermined period by loading the program stored in the auxiliary storage device 47 to the main storage device 42 by the processor 41 of the server apparatus 4.

In Step S1101, the processor 41 of the server apparatus 4 accesses the AG value storage unit 53 to judge whether or not any new average glucose concentration AG is added from any user. If the affirmative judgment is made in this step, the routine proceeds to Step S1102. If the negative judgment is made, this routine is completed.

In Step S1102, the processor 41 specifies the acquisition source user as the acquisition source of the data on the basis of the user identification information concerning the user transmission data and the data acquisition time and date information. The processor 41 accesses the server side calibration curve storage unit 52 corresponding to the user terminal 2 used by the specified acquisition source user. The processor 41 substitutes the acquired average glucose concentration AG for the inherent calibration curve data SCu corresponding to the acquisition source user and the common calibration curve data SCp respectively. The HbAlc concentrations (first index values), which correspond to the substituted average glucose concentration AG in relation to the inherent calibration curve data SCu and the common calibration curve data SCp respectively, are derived as the inherent calibration curve corresponding value Vd1 and the common calibration curve corresponding value Vd2 respectively (see Fig. 8). The inherent calibration curve corresponding value Vd1 and the common calibration curve corresponding value Vd2 are as described in the first embodiment.

In Step S1103, the processor 41 judges the magnitude correlation with respect to the threshold values set for the HbAlc concentration in relation to the inherent calibration curve corresponding value Vd1 and the common calibration curve corresponding value Vd2 respectively. In this case, as explained with reference to Fig. 36, the threshold value is set, for example, to 5.7% and 6.4%. Subsequently, in Step S1104, the processor 41 judges whether or not the judgment results for the inherent calibration curve corresponding value Vd1 and the common calibration curve corresponding value Vd2 respectively are different from each other. If the judgment results for the inherent calibration curve corresponding value Vd1 and the common calibration curve corresponding value Vd2 respectively are different from each other, the routine proceeds to Step S1105. If the judgment results are not different from each other, this routine is completed.

An explanation will be made about a situation in which the respective judgment results for the inherent calibration curve corresponding value Vd1 and the common calibration curve corresponding value Vd2 are different from each other in Step S1103 described above. For example, if the inherent calibration curve corresponding value Vd1 is acquired as 5.5%, and the common calibration curve corresponding value Vd2 is acquired as 5.9%, then the judgment results for the respective corresponding values Vd1, Vd2 are different from each other. In this context, a consideration is made about the combined data A (Vd1, FPG) and the combined data B (Vd2, FPG) prepared by combining the inherent calibration curve corresponding value Vd1 and the common calibration curve corresponding value Vd2 respectively with FPG as the second index value. If the user terminal 2 is controlled in the simultaneous display mode in accordance with the process flow concerning Fig. 36 by using the combined data A and the combined data B as described above, the areas, which are included in the display area divided into the plurality of areas on the second display 24B and to which the combined data A and the combined data B belong respectively, are different from each other. Therefore, in this case, it is possible to acknowledge that the significant difference is provided between the inherent calibration curve data SCu and the common calibration curve data SCp. It is possible to judge that the significant difference is caused, for example, by the change of the physical constitution of the user or the external factor error such as the malfunction, the abnormality or the like of the CGM measuring device 1 (including the glucose sensor 11).

Accordingly, in Step S1105, the processor 41 transmits the predetermined update request information to the user terminal 2 of the acquisition source user. The update request information is the actual measurement request information to request the actual measurement of the first index value, i.e., the HbAlc concentration with respect to the acquisition source user. If the user terminal 2 of the acquisition source user receives the update request information (actual measurement request information), the processor 21 allows, for example, the second display 24B to display a message M1 of "Actually measure glycohemoglobin concentration in designated medical facility". It is also allowable to display a message M2 of "CGM measuring device and/or sensor may be possibly abnormal, so exchange sensor with new one or perform maintenance for CGM measuring device" in combination with the foregoing message. Alternatively, the type of the message may be selected depending on whether or not the difference X between the inherent calibration curve corresponding value Vd1 and the common calibration curve corresponding value Vd2 is not less than a threshold value $\alpha$. For example, the message M1 may be outputted if the difference X is less than the threshold value $\alpha$, while the message M2 may be outputted if the difference X is not less than the threshold value $\alpha$. The messages may be outputted as voice from a speaker (not shown) of the user terminal 2, in place of the display presentation on the display. Further, in this step, the inherent calibration curve data update flag is set to ON ($F \leftarrow ON$). The switching of the inherent calibration curve data update flag from OFF to ON provides the trigger to execute the second calibration curve update routine as described later on.

This exemplary control is illustrative of the exemplary case in which FPG as the second index value is coincident between the combined data A (Vd1, FPG) and the combined data B (Vd2, FPG). However, it is also allowable that the values, which are indicated by FPG of the combined data A and FPG of the combined data B, are different from each other. In this case, it is appropriate in Step S1103 that the processor 41 judges the magnitude correlation between the predetermined threshold value determined for FPG (second index value) and FPG included in each of the pieces of combined data. Further, it is appropriate to adopt the following procedure. That is, in Step S1104, if the processor 41 judges that the judgment result in relation to the magnitude correlation between the first index value included in each of the pieces of combined data A (Vd1, FPG1) and combined data B (Vd2, FPG2) and the threshold value corresponding to the first index value and the judgment result in relation to the magnitude correlation between the second index value included in each of the pieces of combined data A (Vd1, FPG1) and combined data B (Vd2, FPG2) and the threshold value corresponding to the second index value are different from each other even partially, the routine proceeds to Step S1105 to perform the process for transmitting the update request information to the user terminal 2. The contents of the series of processes described above can be also applied to the first calibration curve update routine (see Fig. 41) executed by the processor 21 of the user terminal 2 in the fourth embodiment described later on. That is, the exemplary modified processes in relation to Steps S1103 to S1105 shown in Fig. 38 described above can be employed equivalently as exemplary modified processes in relation to Steps S1303 to S1305 shown in Fig. 41 described later on.

The common user calibration curve data SCp may be classified depending on, for example, the conditions of sex and age group. For example, the common user calibration curve data SCp may be dealt with as follows. That is, the auxiliary storage device 47 of the server apparatus 4 may store a plurality of types of the common user calibration curve data SCp including, for example, the calibration curve data SCpA constructed by using the actually measured pair data of only males, the calibration curve data SCpB constructed by using the actually measured pair data of only females, the calibration curve data SCpC constructed by using the actually measured pair data of examinees in thirties, the calibration curve data SCpD constructed by using the actually measured pair data of examinees in forties and so forth. In this case, it is appropriate that a number of the common calibration curve corresponding values Vd2 (Vd2A, Vd2B, Vd2C, Vd2D,...) are derived in Step S1103 of the control described above, the number corresponding to the plurality of types of the common user calibration curve data SCp. The following procedure is appropriately adopted. That is, in Step S1104, if the judgment result, which relates to any one of Vd2A, Vd2B, Vd2C, Vd2D,..., is different from the judgment result in relation to the inherent calibration curve corresponding value Vd1, the update request information is transmitted to the user terminal 2 in Step S1105 to arouse the user in relation to the update of the inherent calibration curve data SCu.

Next, an explanation will be made about a specified process of the second calibration curve update routine. Fig. 39 shows a flow chart illustrating the process flow of the second calibration curve update routine according to this embodiment. This routine is also executed by the processor 41 of the server apparatus 4 in the same manner as the first calibration curve update routine. This control routine is repeatedly executed every predetermined period when the inherent calibration curve data update flag is in a state of ON. In this procedure, when the user, who confirmed the message presentation concerning the update request information (measurement request information) on the second display 24B of the user terminal 2, actually measured the HbAlc concentration by using the measuring apparatus 5 in the medical facility, the actual measurement result is newly stored in the HbAlc value storage unit 54 of the auxiliary storage device 47 of the server apparatus 4.

In Step S1201, the processor 41 of the server apparatus 4 accesses the HbAlc value storage unit 54. The processor 41 judges whether or not the data of the actually measured HbAlc concentration, which is stored in the HbAlc value storage unit 54, is newly added (updated). If it is judged in this step that the data of the actually measured HbAlc concentration is not added (updated), this routine is completed. On the other hand, if the data of the actually measured HbAlc concentration is added (updated), the routine proceeds to Step S1202.

In this case, the data of the actually measured HbAlc concentration, which is added to the HbAlc value storage unit 54, is stored while being correlated with the user identification information and the data acquisition time and date information. Accordingly, in Step S1202, the processor 41 updates the inherent calibration curve data SCu of the acquisition source user stored in the server side calibration curve storage unit 52 by using, as the actually measured pair data, the pair of the actually measured HbAlc concentration and the average glucose concentration AG acquired in Step S1101 shown in Fig. 38. Subsequently, in Step S1203, the inherent calibration curve data SCu, which is updated in Step S1202, is delivered by the processor 41 to the user terminal 2 of the corresponding acquisition source user. After that, the processor 41 sets the inherent calibration curve data update flag to OFF, and this routine is completed.

An explanation will now be made about a modified embodiment of the second calibration curve update routine described above. In Step S1202, if any new data of the actually measured HbAlc concentration is added to the HbAlc value storage unit 54, the processor 41 of the server apparatus 4 transmits the newly added data of the actually measured HbAlc concentration to the user terminal 2 of the acquisition source user.

The user terminal 2, which receives the data of the actually measured HbAlc concentration via the network interface 26 from the server apparatus 4, stores the data of the actually measured HbAlc concentration in the actually measured value storage unit 36 of the auxiliary storage device 27. The processor 21 of the user terminal 2 can update the inherent calibration curve data SCu stored in the second calibration curve storage unit 32 of the auxiliary storage device 27, on the basis of the actually measured pair data of the actually measured HbAlc concentration received from the server apparatus 4 and the average glucose concentration AG corresponding thereto.

As described above, according to the method for updating the inherent calibration curve data SCu concerning the information providing system S, it is possible to judge whether or not the inherent calibration curve data SCu is in a state to be originally provided, on the basis of the relative relationship between the common calibration curve data SCp and the inherent calibration curve data SCu, while basically using the inherent calibration curve data SCu inherent in each of the users for estimating the HbAlc concentration. Further, it is possible to update the inherent calibration curve data SCu of the user terminal 2, if necessary.

## Other exemplary system arrangement

The user terminal 2 according to this embodiment may be an SMBG measuring apparatus for performing the self-monitoring of blood glucose (SMBG). Fig. 40 shows a schematic arrangement of the SMBG measuring apparatus 200. The SMBG measuring apparatus 200 performs the measurement of blood in accordance with an electrochemical technique by using a biosensor 205. The SMBG measuring apparatus 200 is provided with a casing 201, a display 202, operation buttons 203, and a sensor insertion port 204. Although not shown in the drawing, the SMBG measuring apparatus 200 has a circuit board which is equipped with electronic parts including, for example, CPU, RAM, and ROM required for predetermined operations of the SMBG measuring apparatus 200 (for example, application of voltage and communication with outside).

As shown in Fig. 40, the casing 201 is provided with the display 202 and the plurality of operation buttons 203. The plurality of operation buttons 203 are used to perform various types of setting and operations including, for example, the start and the completion of the measurement. The plurality of operation buttons 203 may be a contact type touch panel. The display 202 displays the measurement result and the error, and the display 202 displays, for example, the operation procedure in the setting and the operation situation. The SMBG measuring apparatus 200 can measure the glucose concentration by installing the biosensor 205 which retains blood as a sample to the sensor insertion port 204. The average glucose concentration AG can be calculated, for example, such that the glucose concentration is measured by using the biosensor 205 every several hours to select and average pieces of measurement data obtained at least at two points. Therefore, the HbAlc concentration can be appropriately estimated from the measured glucose concentration by storing the inherent calibration curve data SCu inherent in the user in a storage device of the SMBG measuring apparatus 200 in the same manner as the user terminal 2 described above. The simultaneous display mode as shown in Figs. 35 and 37 can be prepared as one of the display modes of the display 202. The control, which relates to the update of the inherent calibration curve data SCu described above, can be also applied to the SMBG measuring apparatus 200.

## Fourth Embodiment

Next, a fourth embodiment will be explained. The fourth embodiment is illustrative of such an arrangement that the processor 21 of the user terminal 2 judges whether or not the inherent calibration curve data SCu stored in the auxiliary storage device 27 of the user terminal 2 is proper, and the inherent calibration curve data SCu is updated if it is judged that the inherent calibration curve data SCu is not proper. The system arrangement of the information providing system S according to this embodiment is the same as or equivalent to that of the second embodiment (see Fig. 31).

Fig. 41 shows a flow chart illustrating the process flow of a first calibration curve update routine according to this embodiment. The first calibration curve update routine is executed by loading the program stored in the auxiliary storage device 27 to the main storage device 22 every predetermined period by the processor 21 of the user terminal 2. In Step S1301, the processor 21 of the user terminal 2 accesses the AG value storage unit 35 of the auxiliary storage device 27 to judge whether or not any new average glucose concentration AG is added to the AG value storage unit 35. If the affirmative judgment is made in this step, the routine proceeds to Step S1302. If the negative judgment is made in this step, this control routine is completed. In Step S1302, the processor 21 accesses the second calibration curve storage unit 32, and the average glucose concentration AG acquired as described above is substituted for the inherent calibration curve data SCu and the common calibration curve data SCp stored in the second calibration curve storage unit 32 respectively to acquire the inherent calibration curve corresponding value Vd1 and the common calibration curve corresponding value Vd2.

In Step S1303, the processor 21 judges the magnitude correlation with respect to predetermined threshold values concerning the HbAlc concentration for the inherent calibration curve corresponding value Vd1 and the common calibration curve corresponding value Vd2 respectively. In this case, the threshold value for the HbAlc concentration is set, for example, to 5.7% and 6.4%. In Step S1304, the processor 21 judges whether or not the judgment results for the inherent calibration curve corresponding value Vd1 and the common calibration curve corresponding value Vd2 are different from each other. In this case, the processor 21 of the user terminal 2 executes the program stored in the auxiliary storage device 27 to perform the judgment in this step as described above, and thus the processor 21 functions as the judging unit. If the judgment results for the inherent calibration curve corresponding value Vd1 and the common calibration curve corresponding value Vd2 are different from each other in this step, the routine proceeds to Step S1305. If the judgment results are not different from each other, this routine is completed.

In Step S1305, the processor 21 outputs the update request information to request the update of the inherent calibration curve data. The update request information is the actual measurement request information to request the actual measurement of the first index value, i.e., the HbAlc concentration with respect to the user. In this step, the processor 21 allows the second display 24B to display the message M1 explained in the third embodiment. Further, the message M2 described above may be displayed in combination with the foregoing message. The type of the message may be selected depending on whether or not the difference X between the inherent calibration curve corresponding value Vd1 and the common calibration curve corresponding value Vd2 is not less than the threshold value $\alpha$, in the same manner as in the third embodiment. The messages may be outputted as voice from a speaker (not shown) of the user terminal 2 in place of the display presentation on the display. Further, in this step, the inherent calibration curve data update flag is set to ON (F $\leftarrow$ ON). The switching of the inherent calibration curve data update flag from OFF to ON provides the trigger to execute a second calibration curve update routine as described below.

Next, an explanation will be made about the second calibration curve update routine. Fig. 42 shows a flow chart illustrating the process flow of the second calibration curve update routine according to this embodiment. This routine is also executed by the processor 21 in the same manner as the first calibration curve update routine. This control routine is repeatedly executed every predetermined period when the inherent calibration curve data update flag is in a state of ON. In this procedure, when the user, who confirmed the message presentation concerning the update request information (measurement request information) on the second display 24B of the user terminal 2, actually measured the HbAlc concentration by using the measuring apparatus 5 in the medical facility, the actual measurement result is newly stored in the HbAlc value storage unit 54 (actually measured value acquiring unit) of the auxiliary storage device 47 of the server apparatus 4. The HbAlc value storage unit 36 stores the actually measured HbAlc concentration acquired by at least any means of the input operation performed by the user with the operation unit 23, any transportable recording medium, and the communication from an external apparatus such as the server apparatus 4, the measuring apparatus 5 or the like.

In Step S1401, the processor 21 accesses the HbAlc value storage unit 36. The processor 21 judges whether or not the data concerning the actually measured HbAlc concentration, which is stored in the HbAlc value storage unit 36, is newly added. If it is judged in this step that the data of the actually measured HbAlc concentration is not added (updated), this routine is completed. On the other hand, if the data of the actually measured HbAlc concentration is added (updated), the routine proceeds to Step S1402. In this case, the data of the actually measured HbAlc concentration, which is added to the HbAlc value storage unit 36, is stored while being correlated with the user identification information and the data acquisition time and date information. Accordingly, in Step S1402, the processor 21 updates the inherent calibration curve data SCu stored in the auxiliary storage device 27 by providing, as the actually measured pair data, the combination of the actually measured HbAlc concentration and the average glucose concentration AG corresponding to the data acquisition time and date information. After that, the processor 21 sets the inherent calibration curve data update flag to OFF, and this routine is completed.

As described above, it is also possible to judge whether or not the inherent calibration curve data SCu is in a state to be originally provided, on the basis of the relative relationship between the common calibration curve data SCp and the inherent calibration curve data SCu in the same manner as in the third embodiment, in relation to the method for updating the inherent calibration curve data SCu concerning the fourth embodiment. It is possible to update the inherent calibration curve data SCu of the user terminal 2, if necessary.

Explanation about computer readable medium

Any one of the functions provided in this embodiment explained above may be stored in a storage area of a computer readable medium by being coded. In this case, the program, which is usable to realize the function, may be provided via the computer readable medium to a computer or a computer which is incorporated into any machine or any apparatus. The computer or the computer which is incorporated into any machine or any apparatus can realize the function by reading the program from the storage area of the computer readable medium and executing the program.

The computer readable medium herein refers to a recording medium which accumulates the information including, for example, programs and data in accordance with any electrical, magnetic, optical, chemical, physical, or mechanical action and which retains the information in a state of being readable by the computer. Those included in the recording medium as described above, which are removable from the computer, include, for example, flexible disk, magneto-optical disk, CD-ROM, CD-R/W, DVD, DAT, 8 mm tape, and memory card. The recording medium, which is fixed in the computer, includes, for example, hard disk and ROM. The form or mode for carrying out the present invention can include combinations of the respective embodiments as far as possible. Various modifications may be applied to the embodiments described above within a range without deviating from the gist or essential characteristics of the present invention.

## Claims

**1.** An information processing apparatus (4) capable of communicating with a user terminal (2) which is configured to acquire a HbAlc concentration for reflecting a state of an average glucose concentration of a user in a certain period of time, and to provide the acquired HbAlc concentration to the user:
the information processing apparatus (4) comprising:
a storage device (47) which is configured to store common calibration curve data in which a correlation between the average glucose concentration and the HbAlc concentration averaged for multiple users is reflected, and inherent calibration curve data in which a correlation inherent in the user is reflected in relation to the average glucose concentration and the HbAlc concentration, wherein:
the information processing apparatus (4) is configured to:

deliver the inherent calibration curve data to the user terminal (2) for acquiring the HbAlc concentration;
acquire a measured value of the average glucose concentration of the user;
apply the acquired measured value of the average glucose concentration to the common calibration curve data and the inherent calibration curve data respectively, to obtain a common calibration curve corresponding value and an inherent calibration curve corresponding value of the HbAlc concentration;
judge whether or not a deviation amount between the common calibration curve corresponding value and the inherent calibration curve corresponding value exceeds a predetermined first reference value;
transmit measured value request information to request the actual measurement of the HbAlc concentration if it is judged that the deviation amount exceeds the first reference value;
update the inherent calibration curve data when the deviation exceeds the first reference value by using both the measured concentration of HbAlc and the measured value of the average glucose concentration; and
transmit the updated inherent calibration curve data to the user terminal (2).

**2.** The information processing apparatus (4) according to claim 1, wherein the information processing apparatus (4) is further configured to:

transmit correction request information for the average glucose concentration if it is judged that the deviation amount exceeds a predetermined second reference value larger than the first reference value; and
update the inherent calibration curve data on the basis of the measured HbAlc concentration and a corrected measured value of the average glucose concentration acquired after the transmission of the correction request information.

**3.** The information processing apparatus (4) according to claim 2, wherein:

the measured value of the average glucose concentration is an average value of a plurality of pieces of measurement data acquired by continuously measuring the average glucose concentration in a predetermined reference period; and
the corrected measured value of the average glucose concentration is calculated as an average value of a plurality of pieces of measurement data concerning the average glucose concentration acquired by performing a reference period shortening process for shortening the reference period, or an average value of a plurality of pieces of measurement data concerning the average glucose concentration acquired by performing an abnormal value removing process for removing the measurement data in which a predetermined measured value change range exceeds an allowable value in the reference period.

**4.** An information processing apparatus (4) capable of communicating with a user terminal (2) which is configured to acquire a HbAlc concentration for reflecting a state of an average glucose concentration of a user in a certain period of time, and to provide the acquired HbAlc concentration to the user:
the information processing apparatus (4) comprising:
a storage device (47) which is configured to store common calibration curve data in which a correlation between the average glucose concentration and the HbAlc concentration averaged for multiple users is reflected, and inherent calibration curve data in which a correlation inherent in the user is reflected in relation to the average glucose concentration and the HbAlc concentration, wherein:
the information processing apparatus (4) is configured to:

deliver the inherent calibration curve data to the user terminal (2) for acquiring the HbAlc concentration;
acquire a measured value of the HbAlc concentration actually measured from a specimen of the user;
apply the acquired measured value of the HbAlc concentration to the common calibration curve data and the inherent calibration curve data, respectively, to obtain a common calibration curve corresponding value and an inherent calibration curve corresponding value of the average glucose concentration;
judge whether or not a deviation amount between the common calibration curve corresponding value and the inherent calibration curve corresponding value exceeds a predetermined first reference value;
transmit measured value request information to request the actual measurement of the average glucose concentration if it is judged that the deviation amount exceeds the first reference value;
update the inherent calibration curve data when the deviation exceeds the first reference value by using both the measured value of HbAlc concentration and the measured value of the average glucose concentration; and
transmit the updated inherent calibration curve data to the user terminal (2).

**5.** The information processing apparatus (4) according to claim 4, wherein the information processing apparatus (4) is further configured to judge that any abnormality or malfunction arises in a measuring apparatus for actually measuring the HbAlc concentration from the specimen of the user if it is judged that the deviation amount exceeds a predetermined second reference value larger than the first reference value.

**6.** The information processing apparatus (4) according to any one of claims 1 to 5, wherein the common calibration curve data is constructed on the basis of a correlation between measured values of the average glucose concentration measured from specimens of a plurality of examinees and measured values of the HbAlc concentration measured from the specimens.

**7.** A system comprising:

a user terminal (2) which is configured to acquire a HbAlc concentration for reflecting a state of an average glucose concentration of a user in a certain period of time and to provide the acquired HbAlc concentration to the user; and
an information processing apparatus (4) according to any of the preceding claims.

## Patentansprüche

**1.** Informationsverarbeitungsvorrichtung (4), die mit einem Benutzerendgerät (2) kommunizieren kann, das zum Erfassen einer HbA1c-Konzentration konfiguriert ist, um einen Zustand einer durchschnittlichen Glucosekonzentration eines Benutzers in einer bestimmten Zeitperiode zu reflektieren und die erfasste HbA1c-Konzentration dem Benutzer anzuzeigen;
wobei die Informationsverarbeitungsvorrichtung (4) Folgendes umfasst:

ein Speichergerät (47), konfiguriert zum Speichern von gemeinsamen Kalibrationskurvendaten, in denen eine Korrelation zwischen der durchschnittlichen Glucosekonzentration und der über mehrere Benutzer gemittelten HbA1c Konzentration reflektiert wird, und von inhärenten Kalibrationskurvendaten, in denen eine in dem Benutzer inhärente Korrelation in Bezug auf die durchschnittliche Glucosekonzentration und die HbA1c-Konzentration reflektiert wird, wobei:
die Informationsverarbeitungsvorrichtung (4) konfiguriert ist zum:

Liefern der inhärenten Kalibrationskurvendaten zum Benutzerendgerät (2) zum Erfassen der HbA1c-Konzentration;
Erfassen eines Messwertes der durchschnittlichen Glucosekonzentration des Benutzers;
Anwenden des erfassten Messwertes der durchschnittlichen Glucosekonzentration auf die gemeinsamen Kalibrationskurvendaten bzw. die inhärenten Kalibrationskurvendaten, um einen gemeinsamer Kalibrationskurve entsprechenden Wert und einen inhärenter Kalibrationskurve entsprechenden Wert der HbA1c-Konzentration zu erhalten;
Beurteilen, ob ein Abweichungsbetrag zwischen dem gemeinsamer Kalibrationskurve entsprechenden Wert und dem inhärenter Kalibrationskurve entsprechenden Wert einen vorbestimmten ersten Referenzwert übersteigt oder nicht;
Senden von Messwert-Anforderungsinformationen zum Anfordern des eigentlichen Messwertes der HbA1c-Konzentration, wenn beurteilt wird, dass der Abweichungsbetrag den ersten Referenzwert übersteigt;
Aktualisieren der inhärenten Kalibrationskurvendaten, wenn die Abweichung den ersten Referenzwert übersteigt, anhand der gemessenen Konzentration von HbA1c und des Messwertes der durchschnittlichen Glucosekonzentration; und
Senden der aktualisierten inhärenten Kalibrationskurvendaten zu dem Benutzerendgerät (2).

**2.** Informationsverarbeitungsvorrichtung (4) nach Anspruch 1, wobei die Informationsverarbeitungsvorrichtung (4) ferner konfiguriert ist zum:

Senden von Korrekturanforderungsinformationen für die durchschnittliche Glucosekonzentration, wenn beurteilt wird, dass der Abweichungsbetrag einen vorbestimmten zweiten Referenzwert übersteigt, der größer ist als der erste Referenzwert; und
Aktualisieren der inhärenten Kalibrationskurvendaten auf der Basis der gemessenen HbA1c-Konzentration und eines korrigierten Messwerts der durchschnittlichen Glucosekonzentration, erfasst nach dem Senden der Korrekturanforderungsinformationen.

**3.** Informationsverarbeitungsvorrichtung (4) nach Anspruch 2, wobei:

der Messwert der durchschnittlichen Glucosekonzentration ein Durchschnittswert von mehreren Messdaten ist, erfasst durch kontinuierliches Messen der durchschnittlichen Glucosekonzentration in einer vorbestimmten Referenzperiode; und
der korrigierte Messwert der durchschnittlichen Glucosekonzentration als ein Durchschnittswert von mehreren Messdaten über die durchschnittliche Glucosekonzentration berechnet wird, erfasst durch Durchführen eines Referenzperioden-Verkürzungsprozesses zum Verkürzen der Referenzperiode, oder als Durchschnittswert von mehreren Messdaten in Bezug auf die durchschnittliche Glucosekonzentration, erfasst durch Durchführen eines Prozesses zum Entfernen von anormalen Werten, um die Messdaten zu entfernen, in denen ein vorbestimmter Messwertänderungsbereich einen zulässigen Wert in der Referenzperiode übersteigt.

**4.** Informationsverarbeitungsvorrichtung (4), die mit einem Benutzerendgerät (2) kommunizieren kann, das zum Erfassen einer HbA1c-Konzentration konfiguriert ist, um einen Zustand einer durchschnittlichen Glucosekonzentration eines Benutzers in einer bestimmten Zeitperiode zu reflektieren und um dem Benutzer die erfasste HbA1c-Konzentration anzuzeigen:
wobei die Informationsverarbeitungsvorrichtung (4) Folgendes umfasst:
ein Speichergerät (47), konfiguriert zum Speichern von gemeinsamen Kalibrationsdaten, in denen eine Korrelation zwischen der durchschnittlichen Glucosekonzentration und der HbA1c-Konzentration gemittelt für mehrere Benutzer reflektiert wird, und inhärenten Kalibrationskurvendaten, in denen eine in dem Benutzer inhärente Korrelation in Bezug auf die durchschnittliche Glucosekonzentration und die HbA1c-Konzentration reflektiert wird, wobei:
die Informationsverarbeitungsvorrichtung (4) konfiguriert ist zum:

Liefern der inhärenten Kalibrationskurvendaten an das Benutzerendgerät (2) zum Erfassen der HbA1c-Konzentration;
Erfassen eines Messwertes der tatsächlich von einer Probe des Benutzers gemessenen HbA1c-Konzentration;
Anwenden des erfassten Messwertes der HbA1c-Konzentration auf die gemeinsamen Kalibrationskurvendaten bzw. die inhärenten Kalibrationskurvendaten, um einen gemeinsamer Kalibrationskurve entsprechenden Wert und einen inhärenter Kalibrationskurve entsprechenden Wert der durchschnittlichen Glucosekonzentration zu erhalten;
Beurteilen, ob ein Abweichungsbetrag zwischen dem gemeinsamer Kalibrationskurve entsprechenden Wert und dem inhärenter Kalibrationskurve entsprechenden Wert einen vorbestimmten ersten Referenzwert übersteigt oder nicht;
Senden von Messwertanforderungsinformationen zum Anfordern des eigentlichen Messwerts der durchschnittlichen Glucose-Konzentration, wenn beurteilt wird, dass der Abweichungsbetrag den ersten Referenzwert übersteigt;
Aktualisieren der inhärenten Kalibrationskurvendaten, wenn die Abweichung den ersten Referenzwert übersteigt, anhand des Messwertes der HbA1c-Konzentration und des Messwertes der durchschnittlichen Glucosekonzentration; und
Senden der aktualisierten inhärenten Kalibrationskurvendaten zum Benutzerendgerät (2).

**5.** Informationsverarbeitungsvorrichtung (4) nach Anspruch 4, wobei die Informationsverarbeitungsvorrichtung (4) ferner konfiguriert ist zum Beurteilen, dass eine Anormalität oder Fehlfunktion in einer Messvorrichtung entsteht, um tatsächlich die HbA1c-Konzentration von der Probe des Benutzers zu messen, wenn beurteilt wird, dass der Abweichungsbetrag einen vorbestimmten zweiten Referenzwert übersteigt, der größer ist als der erste Referenzwert.

**6.** Informationsverarbeitungsvorrichtung (4) nach einem der Ansprüche 1 bis 5, wobei die gemeinsamen Kalibrationskurvendaten auf der Basis einer Korrelation zwischen Messwerten der durchschnittlichen von Proben einer Mehrzahl von Prüflingen gemessenen Glucosekonzentration und Messwerten der von den Proben gemessenen HbA1c-Konzentration konstruiert sind.

**7.** System, das Folgendes umfasst:

ein Benutzerendgerät (2), das zum Erfassen einer HbA1c-Konzentration konfiguriert ist, um einen Zustand einer durchschnittlichen Glucosekonzentration eines Benutzers in einer bestimmten Zeitperiode zu reflektieren und um dem Benutzer die erfasste HbA1c-Konzentration anzugeben; und
eine Informationsverarbeitungsvorrichtung (4) nach einem der vorherigen Ansprüche.

## Revendications

**1.** Appareil de traitement d'informations (4) capable de communiquer avec un terminal utilisateur (2) qui est configuré pour acquérir une concentration d'HbA1c afin de refléter une concentration de glucose moyenne d'un utilisateur sur une certaine période de temps, et pour fournir la concentration d'HbA1c acquise à l'utilisateur :
l'appareil de traitement d'informations (4) comprenant :
un dispositif de stockage (47) qui est configuré pour stocker des données de courbe d'étalonnage commune dans laquelle une corrélation entre la concentration de glucose moyenne et la concentration d'HbA1c moyenne calculée pour plusieurs utilisateurs est reflétée, et des données de courbe d'étalonnage inhérente dans laquelle une corrélation inhérente chez l'utilisateur est reflétée par rapport à la concentration de glucose moyenne et la concentration d'HbA1c, dans lequel :
l'appareil de traitement d'informations (4) est configuré pour :

délivrer les données de courbe d'étalonnage inhérente au terminal utilisateur (2) afin d'acquérir la concentration d'HbA1c ;
acquérir une valeur mesurée de la concentration de glucose moyenne de l'utilisateur ;
appliquer la valeur mesurée acquise de la concentration de glucose moyenne aux données de courbe d'étalonnage commune et aux données de courbe d'étalonnage inhérente respectivement, pour obtenir une valeur correspondant à la courbe d'étalonnage commune et une valeur correspondant à la courbe d'étalonnage inhérente de la concentration d'HbA1c ;
juger si une grandeur d'écart entre la valeur correspondant à la courbe d'étalonnage commune et la valeur correspondant à la courbe d'étalonnage inhérente dépasse ou non une première valeur de référence prédéterminée ;

transmettre des informations de demande de valeur mesurée pour demander la mesure réelle de la concentration d'HbA1c s'il est jugé que la grandeur d'écart dépasse la première valeur de référence ;
mettre à jour les données de courbe d'étalonnage inhérente quand l'écart dépasse la première valeur de référence en utilisant à la fois la concentration mesurée d'HbA1c et la valeur mesurée de la concentration de glucose moyenne ; et
transmettre les données de courbe d'étalonnage inhérente mises à jour au terminal utilisateur (2).

**2.** Appareil de traitement d'informations (4) selon la revendication 1, dans lequel l'appareil de traitement d'informations (4) est configuré en outre pour :

transmettre des informations de demande de correction pour la concentration de glucose moyenne s'il est jugé que la grandeur d'écart dépasse une deuxième valeur de référence prédéterminée supérieure à la première valeur de référence ; et
mettre à jour les données de courbe d'étalonnage inhérente sur la base de la concentration d'HbA1c mesurée et une valeur mesurée corrigée de la concentration de glucose moyenne acquise après la transmission des informations de demande de correction.

**3.** Appareil de traitement d'informations (4) selon la revendication 2, dans lequel :

la valeur mesurée de la concentration de glucose moyenne est une valeur moyenne d'une pluralité d'éléments de données de mesure acquises par la mesure continue de la concentration de glucose moyenne sur une période de référence prédéterminée ; et
la valeur mesurée corrigée de la concentration de glucose moyenne est calculée comme une valeur moyenne d'une pluralité d'éléments de données de mesure concernant la concentration de glucose moyenne acquises par la mise en oeuvre d'un procédé de raccourcissement de la période de référence afin de raccourcir la période de référence, ou une valeur moyenne d'une pluralité d'éléments de données de mesure concernant la concentration de glucose moyenne acquises par la mise en oeuvre d'un procédé d'élimination des valeurs anormales afin d'éliminer les données de mesure dans lesquelles une plage de changement de valeur mesurée prédéterminée dépasse une valeur attribuable sur la période de référence.

**4.** Appareil de traitement d'informations (4) capable de communiquer avec un terminal utilisateur (2) qui est configuré pour acquérir une concentration d'HbA1c afin de refléter un état d'une concentration de glucose moyenne d'un utilisateur sur une certaine période de temps, et pour fournir la concentration d'HbA1c acquise à l'utilisateur :
l'appareil de traitement d'informations (4) comprenant :
un dispositif de stockage (47) qui est configuré pour stocker des données de courbe d'étalonnage commune dans laquelle une corrélation entre la concentration de glucose moyenne et la concentration d'HbA1c moyenne calculée pour plusieurs utilisateurs est reflétée, et des données de courbe d'étalonnage inhérente dans laquelle une corrélation inhérente chez l'utilisateur est reflétée par rapport à la concentration de glucose moyenne et la concentration d'HbA1c, dans lequel :
l'appareil de traitement d'informations (4) est configuré pour :

délivrer les données de courbe d'étalonnage inhérente au terminal utilisateur (2) afin d'acquérir la concentration d'HbA1c ;
acquérir une valeur mesurée de la concentration d'HbA1c réellement mesurée à partir d'un échantillon de l'utilisateur ;
appliquer la valeur mesurée acquise de la concentration d'HbA1c aux données de courbe d'étalonnage commune et aux données de courbe d'étalonnage inhérente respectivement, pour obtenir une valeur correspondant à la courbe d'étalonnage commune et une valeur correspondant à la courbe d'étalonnage inhérente de la concentration de glucose moyenne ;
juger si une grandeur d'écart entre la valeur correspondant à la courbe d'étalonnage commune et la valeur correspondant à la courbe d'étalonnage inhérente dépasse ou non une première valeur de référence prédéterminée ;
transmettre des informations de demande de valeur mesurée pour demander la mesure réelle de la concentration de glucose moyenne s'il est jugé que la grandeur d'écart dépasse la première valeur de référence ;
mettre à jour les données de courbe d'étalonnage inhérente quand l'écart dépasse la première valeur de référence en utilisant à la fois la valeur mesurée de la concentration d'HbA1c et la valeur mesurée de la concentration de glucose moyenne ; et
transmettre les données de courbe d'étalonnage inhérente mises à jour au terminal utilisateur (2).

**5.** Appareil de traitement d'informations (4) selon la revendication 4, dans lequel l'appareil de traitement d'informations (4) est configuré en outre pour juger si toute anomalie ou tout dysfonctionnement survient dans un appareil de mesure pour mesurer réellement la concentration d'HbA1c à partir de l'échantillon de l'utilisateur s'il est jugé que la grandeur d'écart dépasse une deuxième valeur de référence prédéterminée supérieure à la première valeur de référence.

**6.** Appareil de traitement d'informations (4) selon l'une quelconque des revendications 1 à 5, dans lequel les données de courbe d'étalonnage commune sont construites sur la base d'une corrélation entre des valeurs mesurées de la concentration de glucose moyenne mesurée à partir d'échantillons d'une pluralité de personnes examinées et de valeurs mesurées de la concentration d'HbA1c mesurée à partir des échantillons.

**7.** Système comprenant :

un terminal utilisateur (2) qui est configuré pour acquérir une concentration d'HbA1c pour refléter un état d'une concentration de glucose moyenne d'un utilisateur sur une certaine période de temps et pour fournir la concentration d'HbA1c acquise à l'utilisateur ; et
un appareil de traitement d'informations (4) selon l'une quelconque des revendications précédentes.

FIG. 1

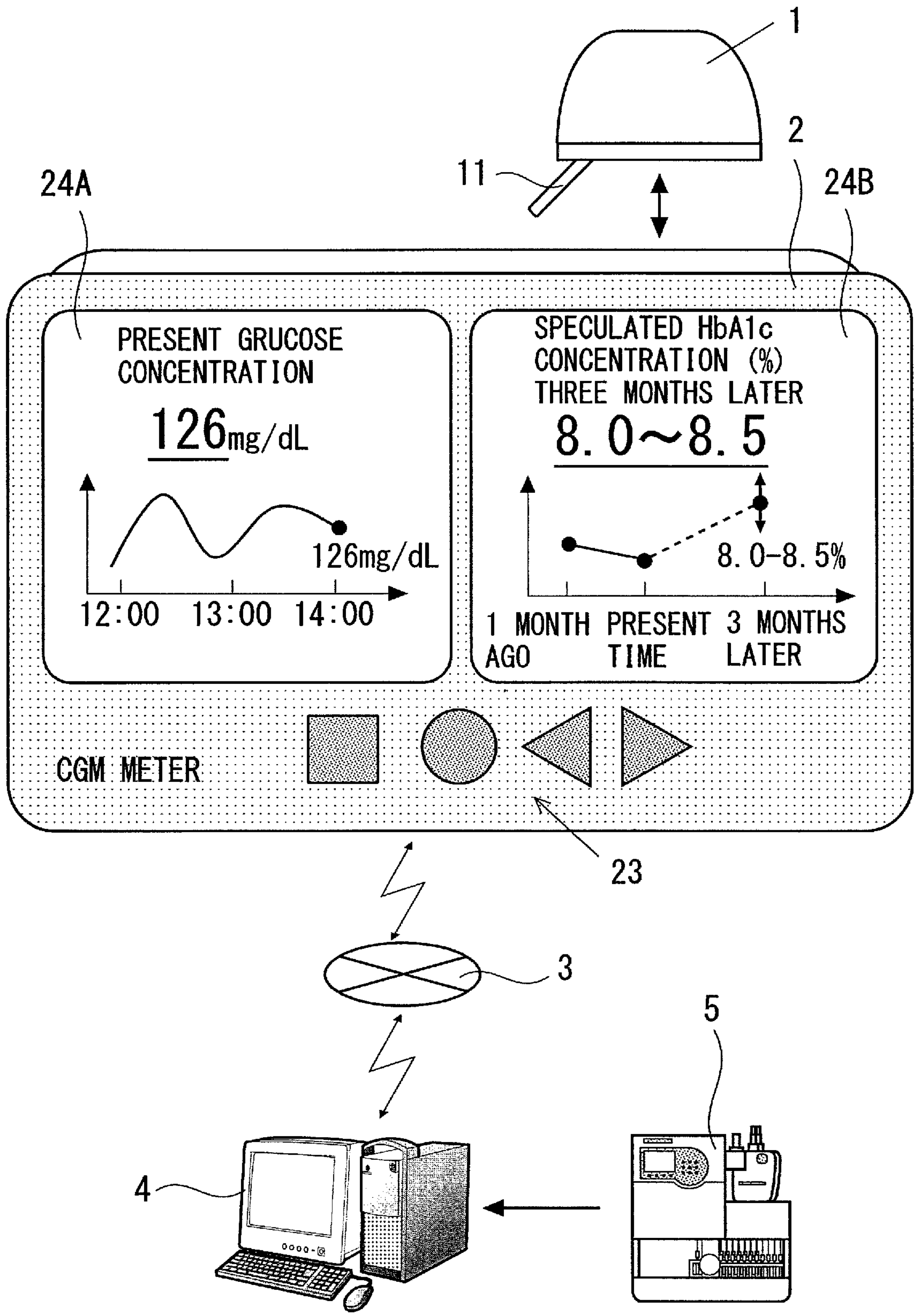
1
2
11
24A
24B
PRESENT GRUCOSE
CONCENTRATION
126mg/dL
126mg/dL
12:00 13:00 14:00
SPECULATED HbA1c
CONCENTRATION (%)
THREE MONTHS LATER
8.0~8.5
8.0-8.5%
1 MONTH AGO
PRESENT TIME
3 MONTHS LATER
CGM METER
23
3
4
5

FIG. 2

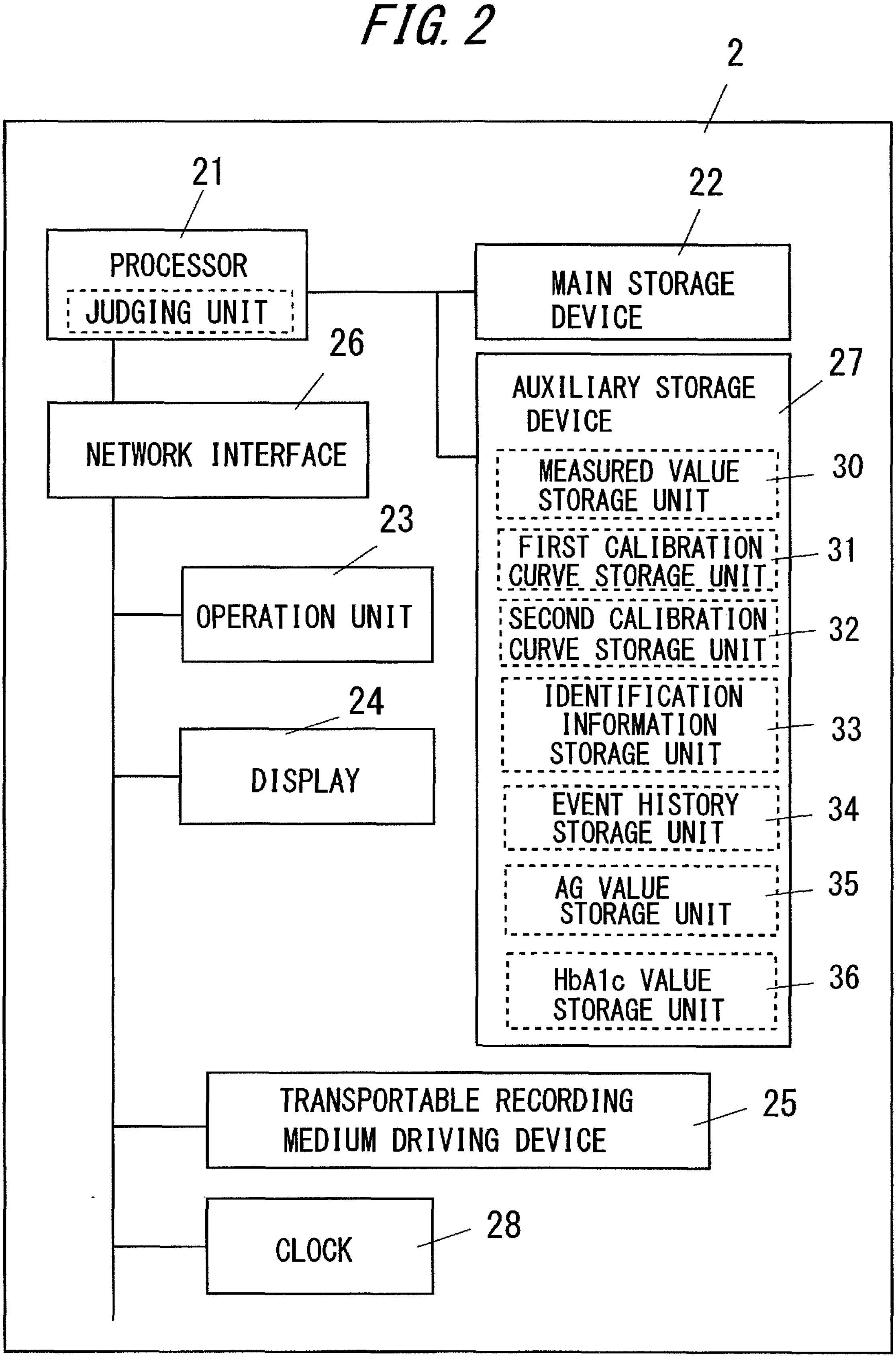
2
21
PROCESSOR
JUDGING UNIT
22
MAIN STORAGE DEVICE
26
NETWORK INTERFACE
27
AUXILIARY STORAGE DEVICE
MEASURED VALUE STORAGE UNIT
30
FIRST CALIBRATION CURVE STORAGE UNIT
31
SECOND CALIBRATION CURVE STORAGE UNIT
32
IDENTIFICATION INFORMATION STORAGE UNIT
33
EVENT HISTORY STORAGE UNIT
34
AG VALUE STORAGE UNIT
35
HbA1c VALUE STORAGE UNIT
36
23
OPERATION UNIT
24
DISPLAY
TRANSPORTABLE RECORDING MEDIUM DRIVING DEVICE
25
CLOCK
28

FIG. 3

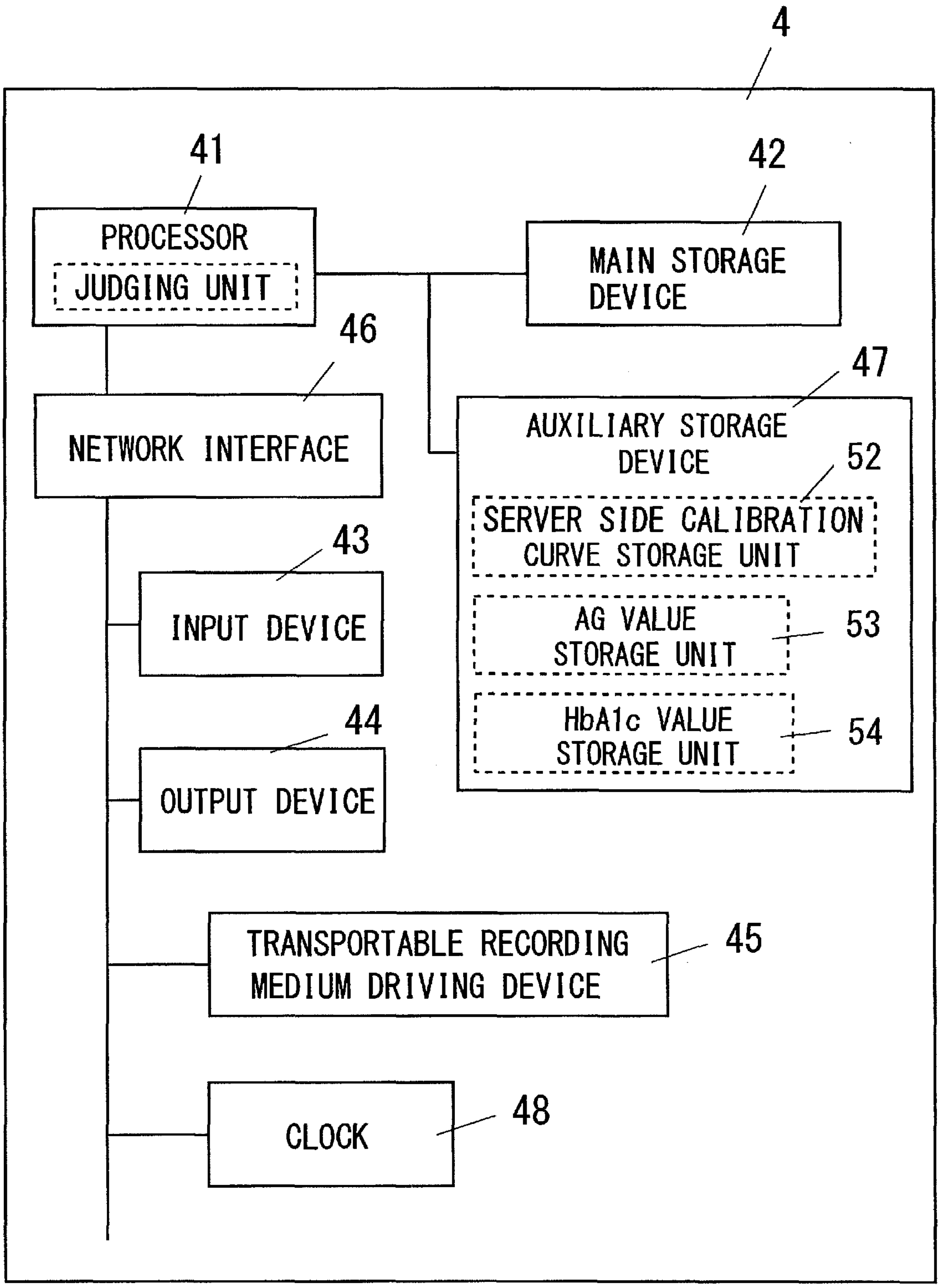
4
41
PROCESSOR
JUDGING UNIT
42
MAIN STORAGE DEVICE
46
NETWORK INTERFACE
47
AUXILIARY STORAGE DEVICE
52
SERVER SIDE CALIBRATION CURVE STORAGE UNIT
43
INPUT DEVICE
AG VALUE STORAGE UNIT
53
44
HbA1c VALUE STORAGE UNIT
54
OUTPUT DEVICE
TRANSPORTABLE RECORDING MEDIUM DRIVING DEVICE
45
CLOCK
48

FIG. 4

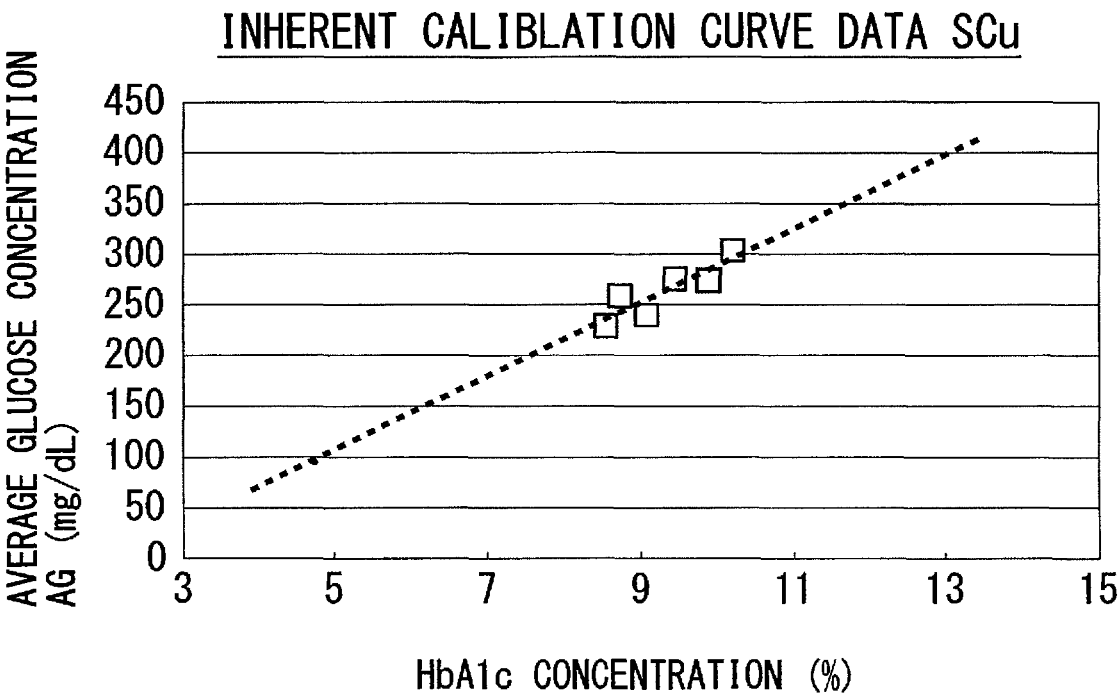
INHERENT CALIBLATION CURVE DATA SCu
AVERAGE GLUCOSE CONCENTRATION
AG (mg/dL)
450
400
350
300
250
200
150
100
50
0
3
5
7
9
11
13
15
HbA1c CONCENTRATION (%)

FIG. 5

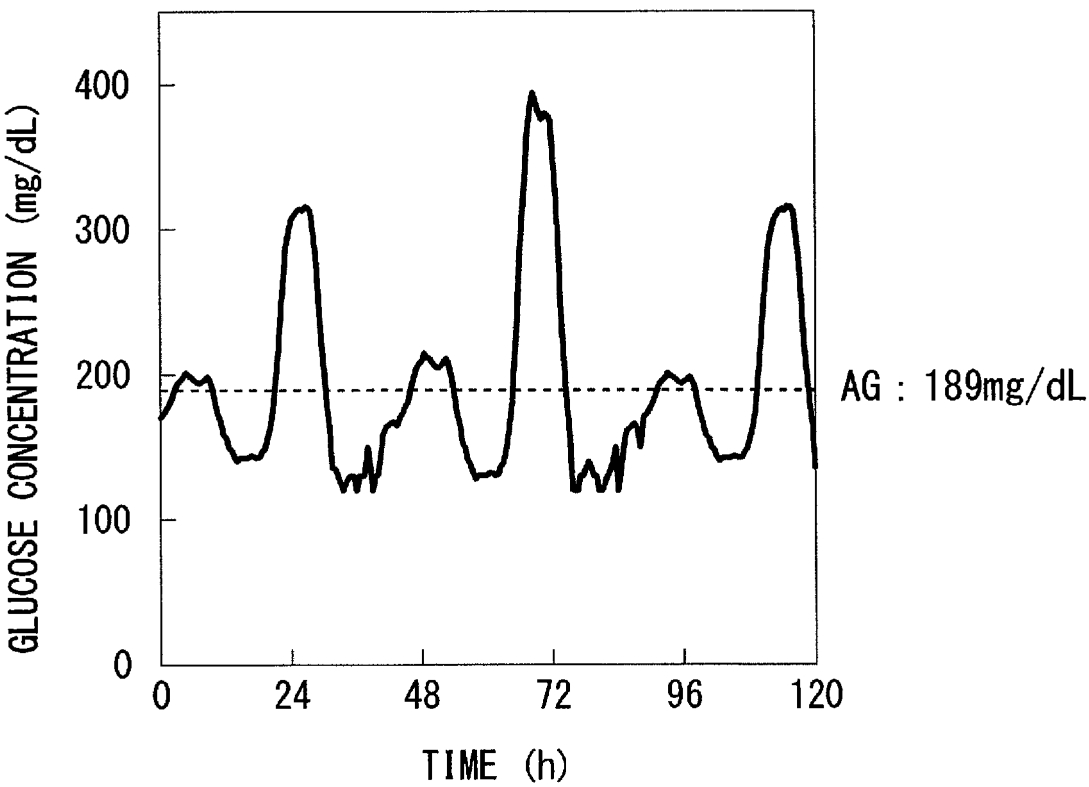
GLUCOSE CONCENTRATION (mg/dL)
400
300
200
100
0
0
24
48
72
96
120
TIME (h)
AG : 189mg/dL

FIG. 6

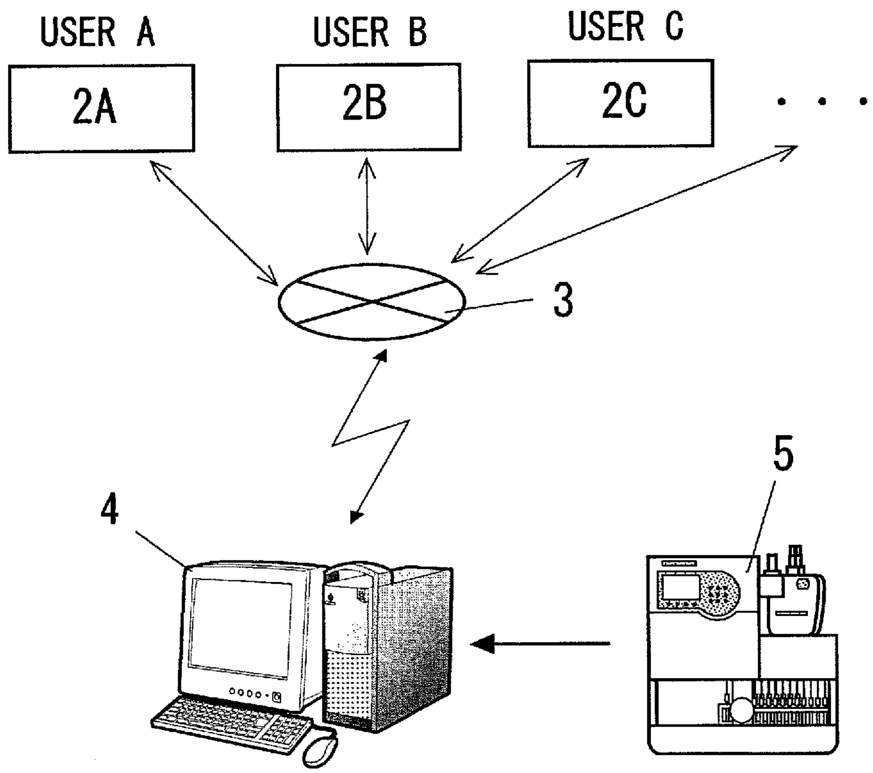

FIG. 7

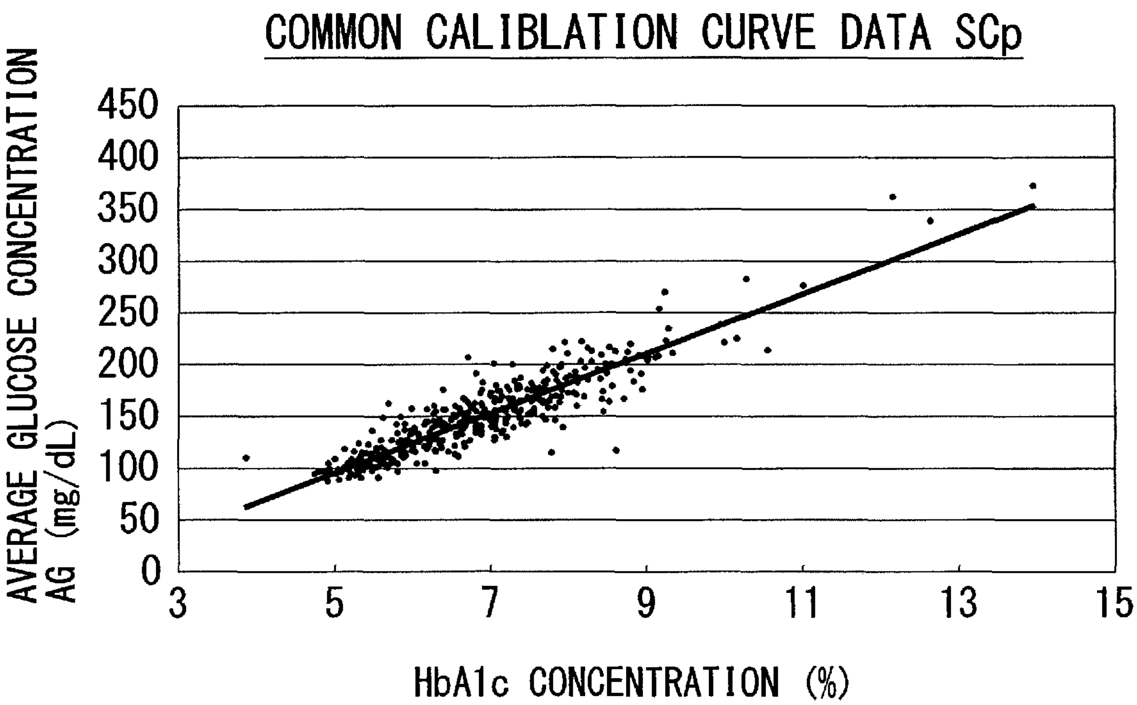
COMMON CALIBLATION CURVE DATA SCp
AVERAGE GLUCOSE CONCENTRATION AG (mg/dL)
450
400
350
300
250
200
150
100
50
0
3
5
7
9
11
13
15
HbA1c CONCENTRATION (%)

FIG. 8

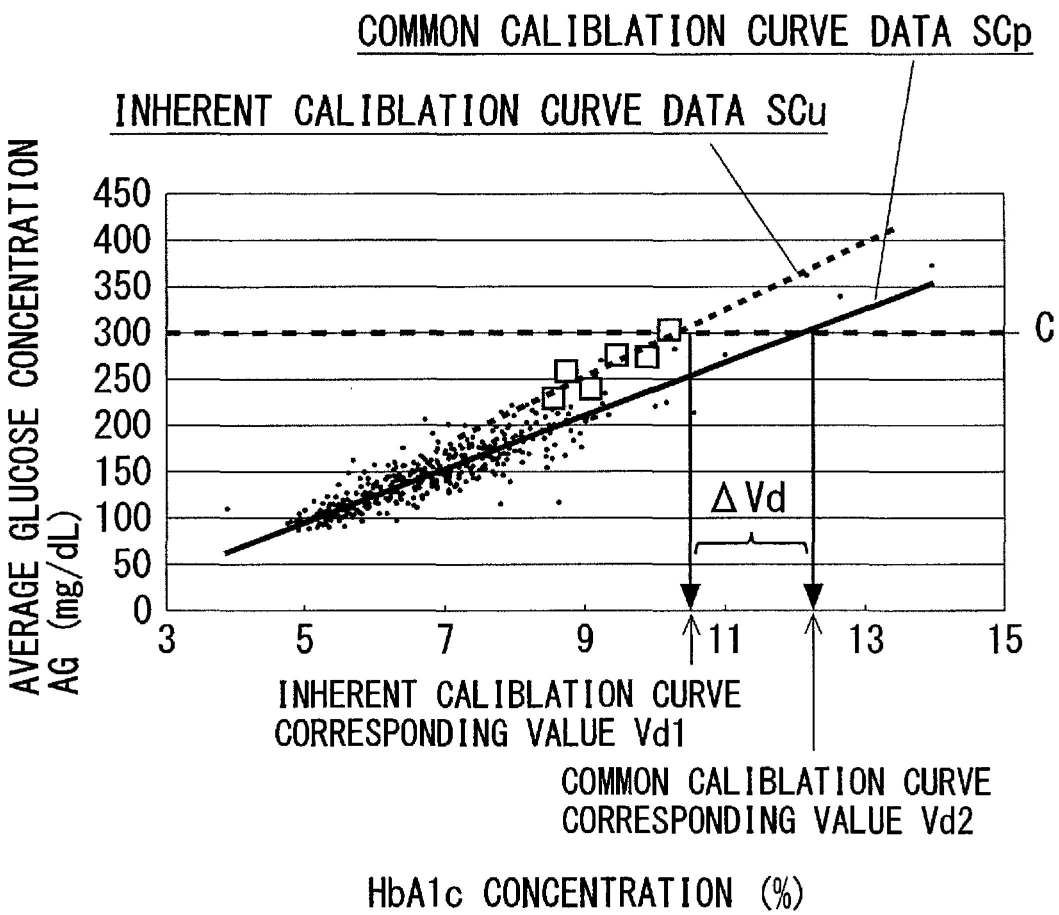
COMMON CALIBLATION CURVE DATA SCp
INHERENT CALIBLATION CURVE DATA SCu
AVERAGE GLUCOSE CONCENTRATION
AG (mg/dL)
450
400
350
300
250
200
150
100
50
0
3
5
7
9
11
13
15
C
ΔVd
INHERENT CALIBLATION CURVE
CORRESPONDING VALUE Vd1
COMMON CALIBLATION CURVE
CORRESPONDING VALUE Vd2
HbA1c CONCENTRATION (%)

FIG. 9

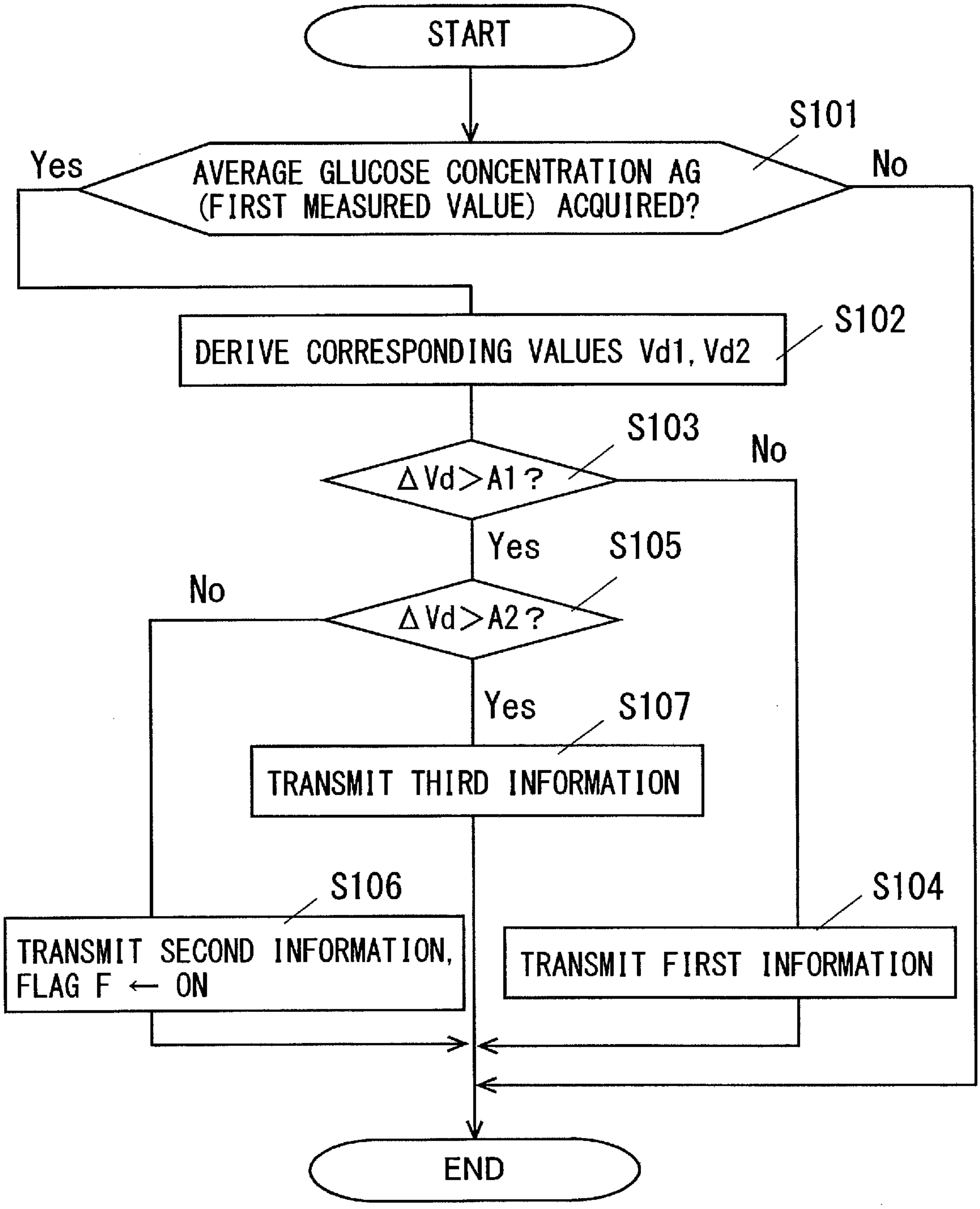
START
S101
Yes
AVERAGE GLUCOSE CONCENTRATION AG
(FIRST MEASURED VALUE) ACQUIRED?
No
DERIVE CORRESPONDING VALUES Vd1, Vd2
S102
S103
No
ΔVd>A1?
Yes
S105
No
ΔVd>A2?
Yes
S107
TRANSMIT THIRD INFORMATION
S106
TRANSMIT SECOND INFORMATION,
FLAG F ← ON
S104
TRANSMIT FIRST INFORMATION
END

FIG. 10

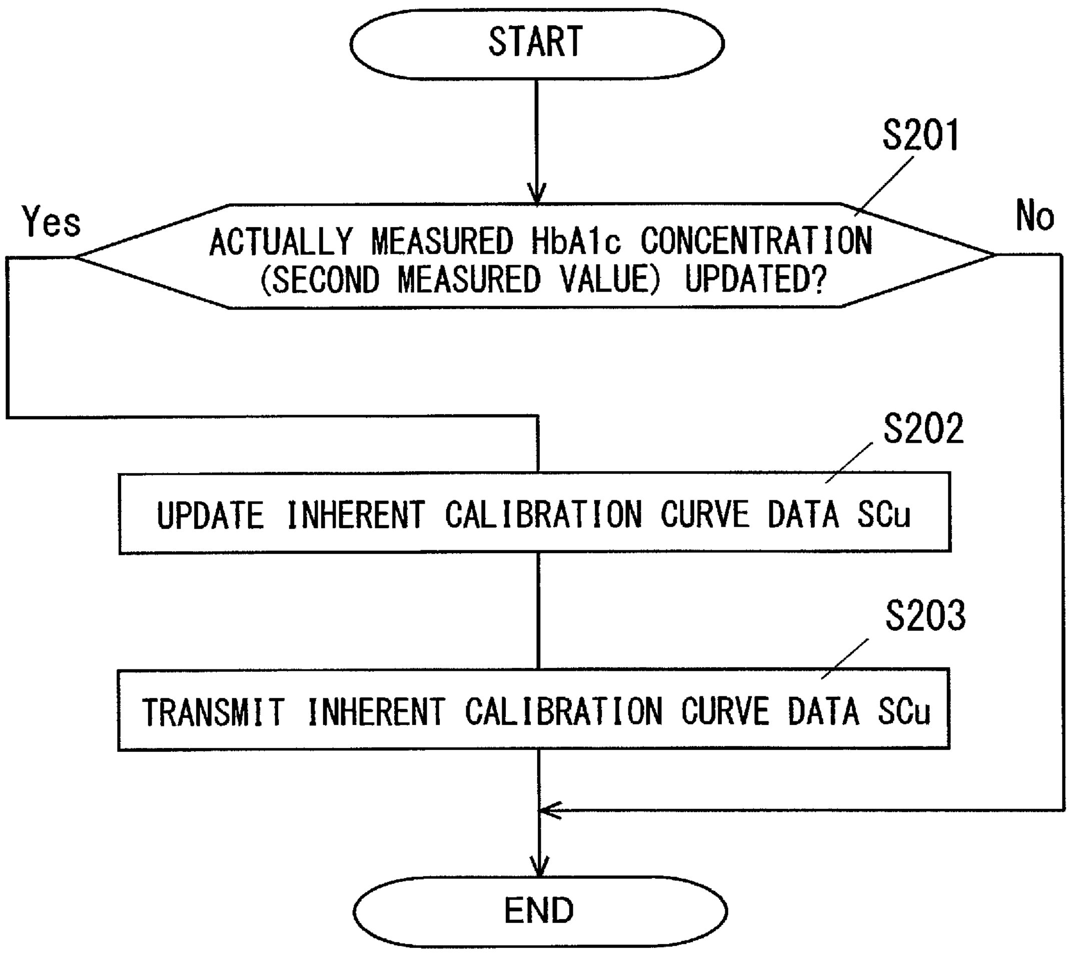
START
S201
ACTUALLY MEASURED HbA1c CONCENTRATION (SECOND MEASURED VALUE) UPDATED?
Yes
No
S202
UPDATE INHERENT CALIBRATION CURVE DATA SCu
S203
TRANSMIT INHERENT CALIBRATION CURVE DATA SCu
END

FIG. 11

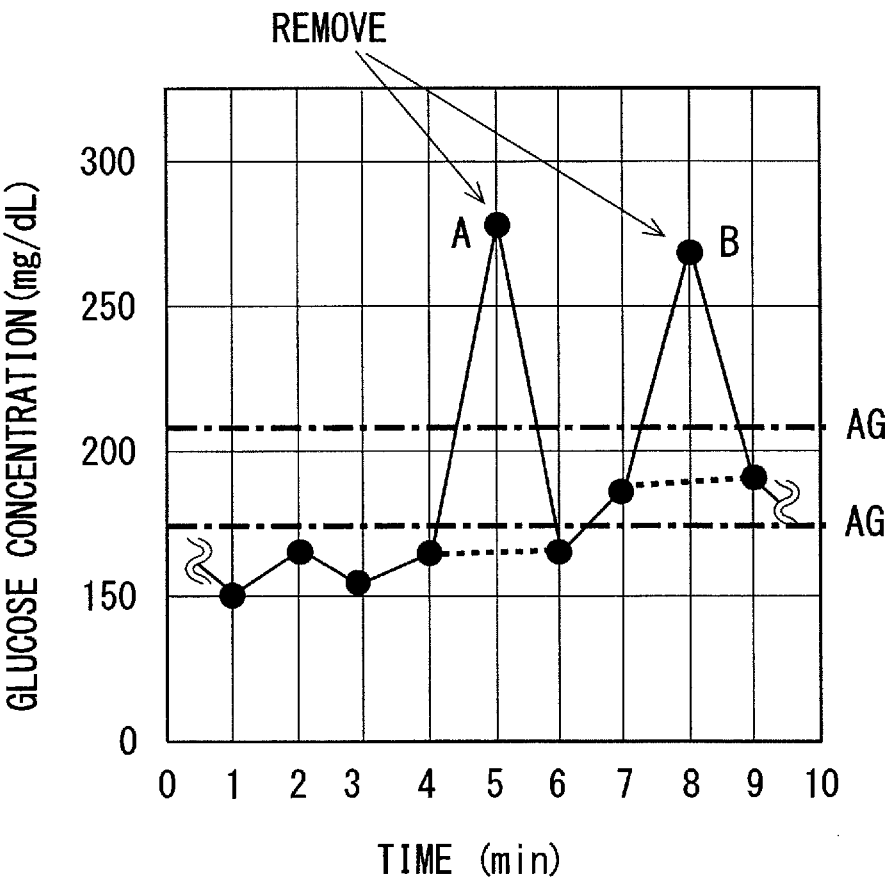
REMOVE
A
B
AG
AG
GLUCOSE CONCENTRATION (mg/dL)
300
250
200
150
0
0
1
2
3
4
5
6
7
8
9
10
TIME (min)

FIG. 12

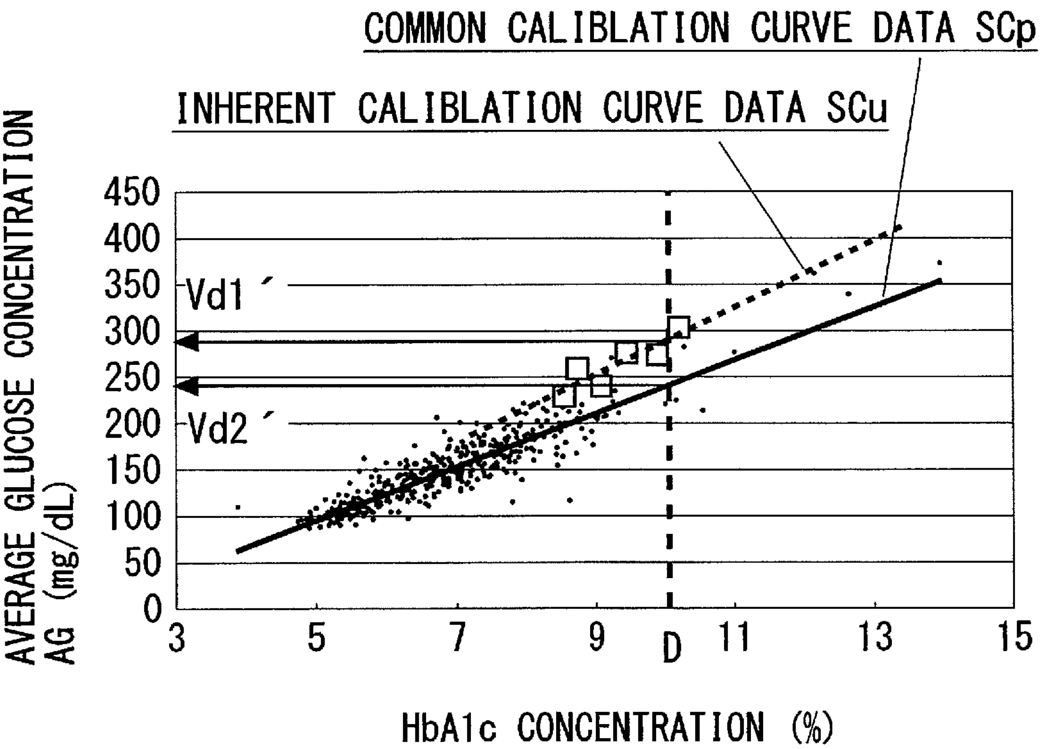
COMMON CALIBLATION CURVE DATA SCp
INHERENT CALIBLATION CURVE DATA SCu
AVERAGE GLUCOSE CONCENTRATION
AG (mg/dL)
450
400
350
300
250
200
150
100
50
0
Vd1 ′
Vd2 ′
3
5
7
9
D
11
13
15
HbA1c CONCENTRATION (%)

FIG. 13

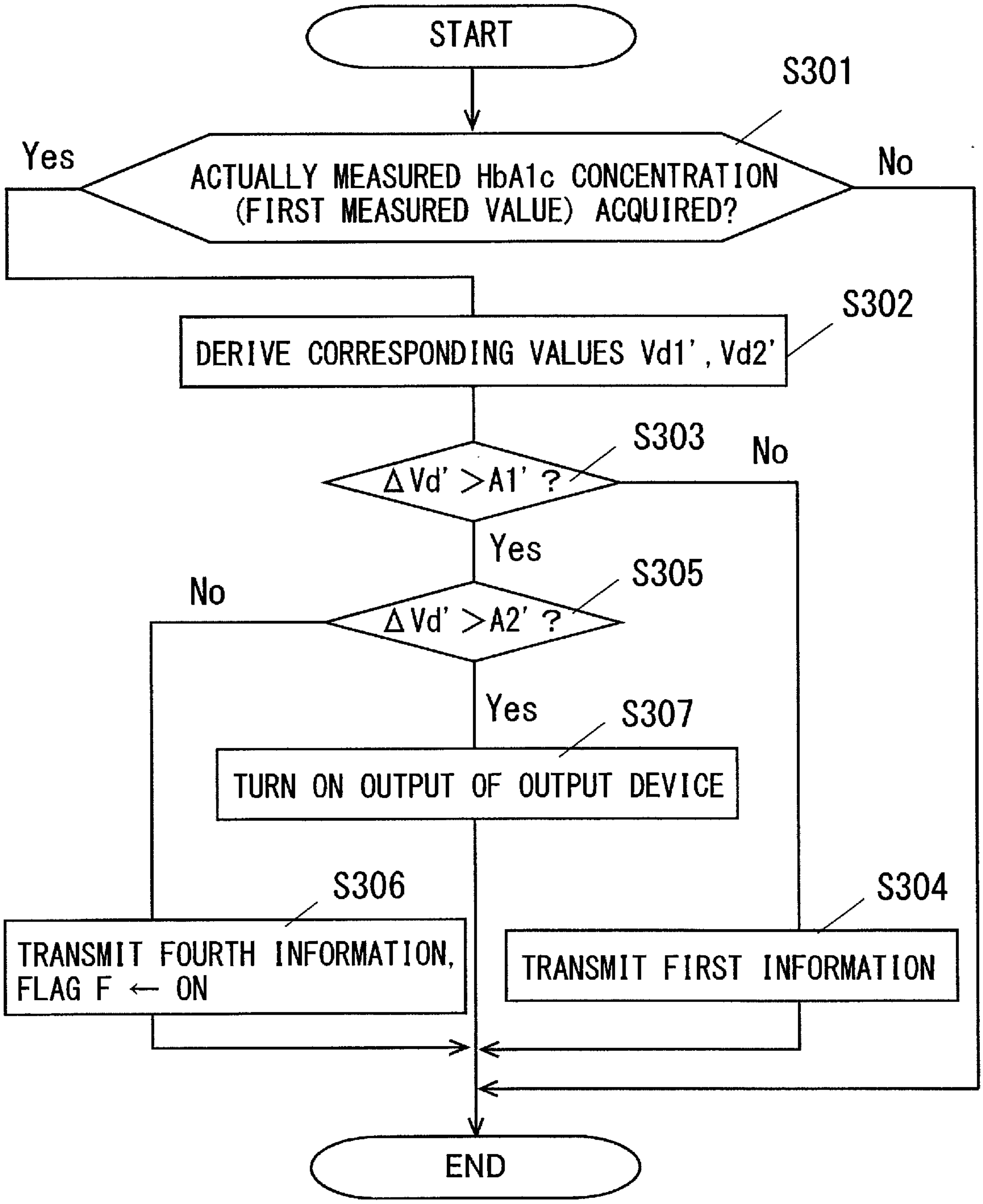
START
S301
ACTUALLY MEASURED HbA1c CONCENTRATION (FIRST MEASURED VALUE) ACQUIRED?
Yes
No
S302
DERIVE CORRESPONDING VALUES Vd1', Vd2'
S303
ΔVd' >A1' ?
No
Yes
S305
ΔVd' >A2' ?
No
Yes
S307
TURN ON OUTPUT OF OUTPUT DEVICE
S306
TRANSMIT FOURTH INFORMATION, FLAG F ← ON
S304
TRANSMIT FIRST INFORMATION
END

FIG. 14

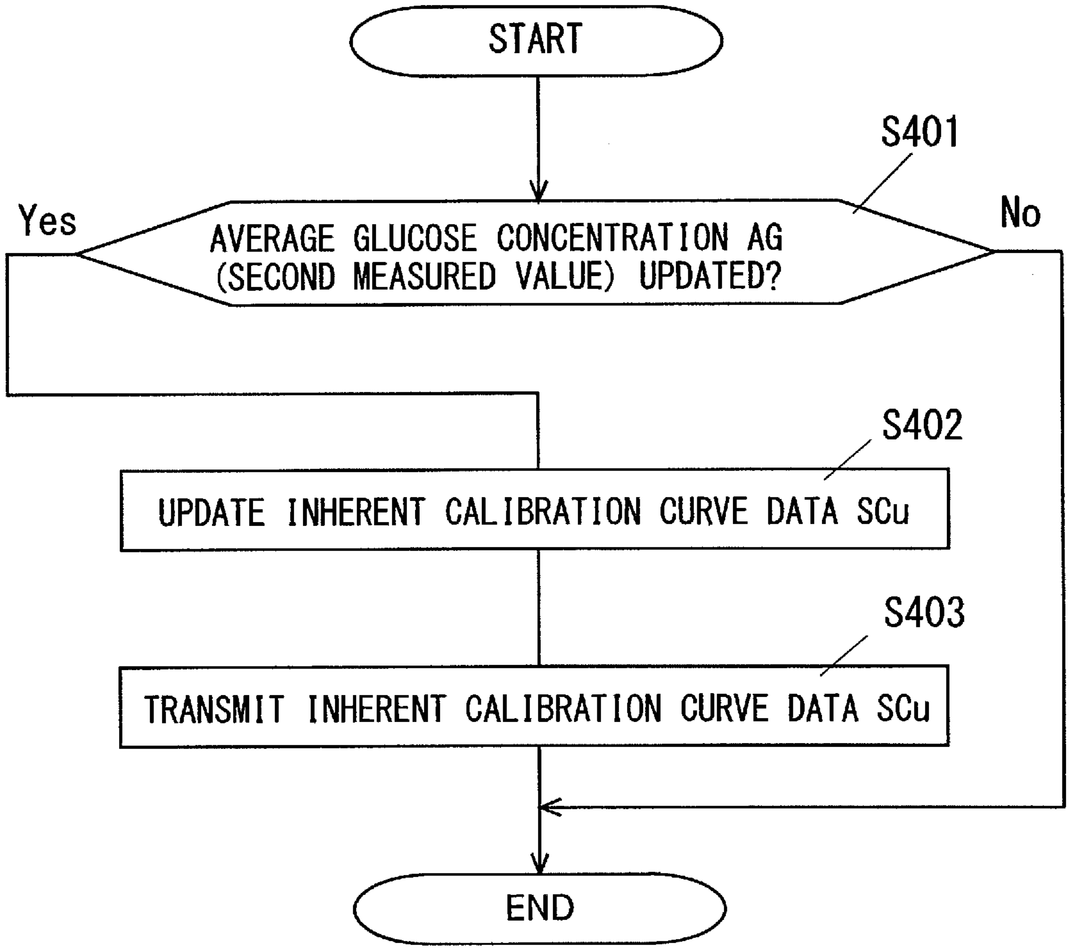
START
S401
Yes
AVERAGE GLUCOSE CONCENTRATION AG (SECOND MEASURED VALUE) UPDATED?
No
S402
UPDATE INHERENT CALIBRATION CURVE DATA SCu
S403
TRANSMIT INHERENT CALIBRATION CURVE DATA SCu
END

*FIG. 15*

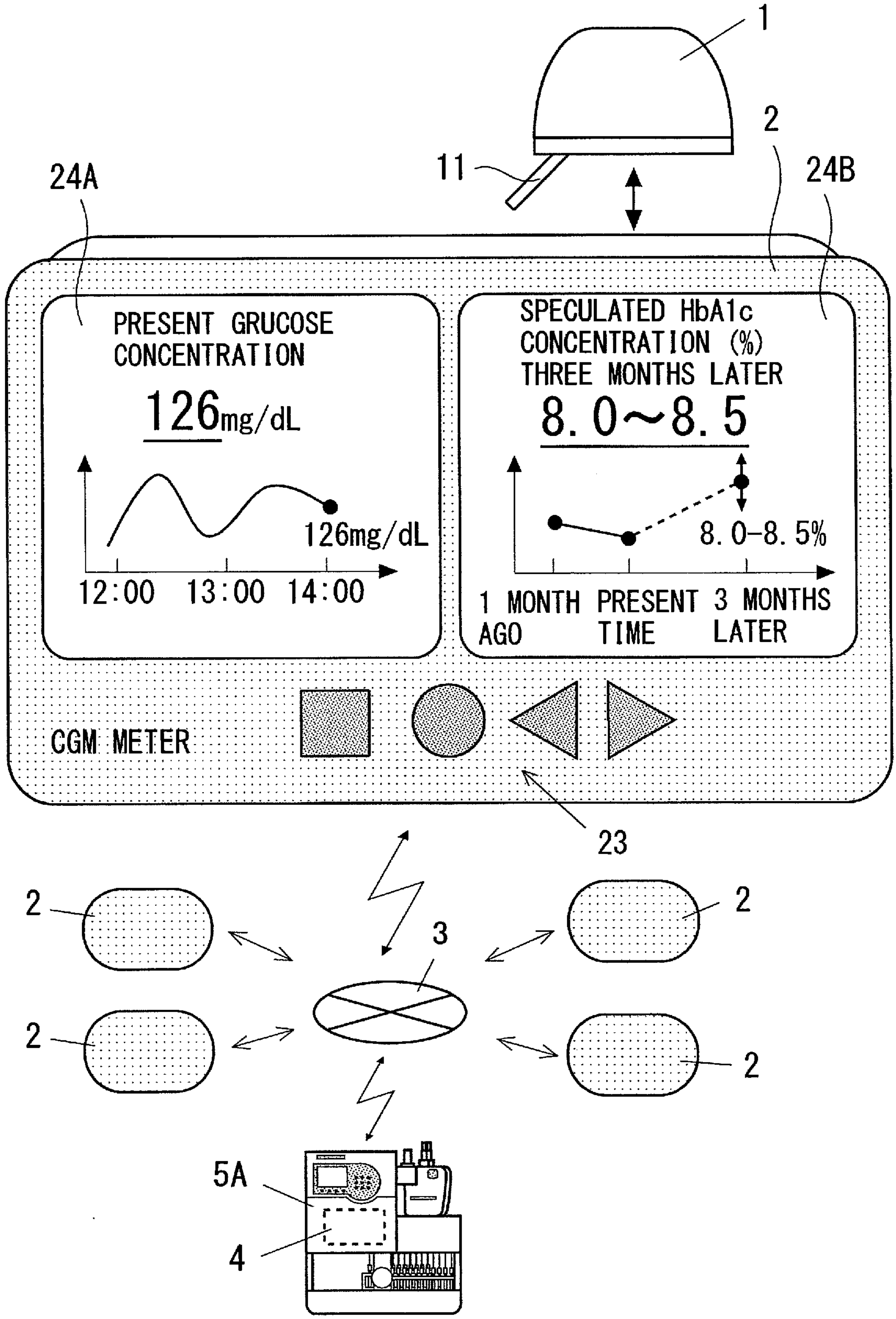
1
11
2
24A
24B
PRESENT GRUCOSE CONCENTRATION
126mg/dL
126mg/dL
12:00 13:00 14:00
SPECULATED HbA1c CONCENTRATION (%) THREE MONTHS LATER
8.0~8.5
8.0-8.5%
1 MONTH AGO
PRESENT TIME
3 MONTHS LATER
CGM METER
23
2
2
2
2
3
5A
4

FIG. 16

BASIC DISPLAY MODE I

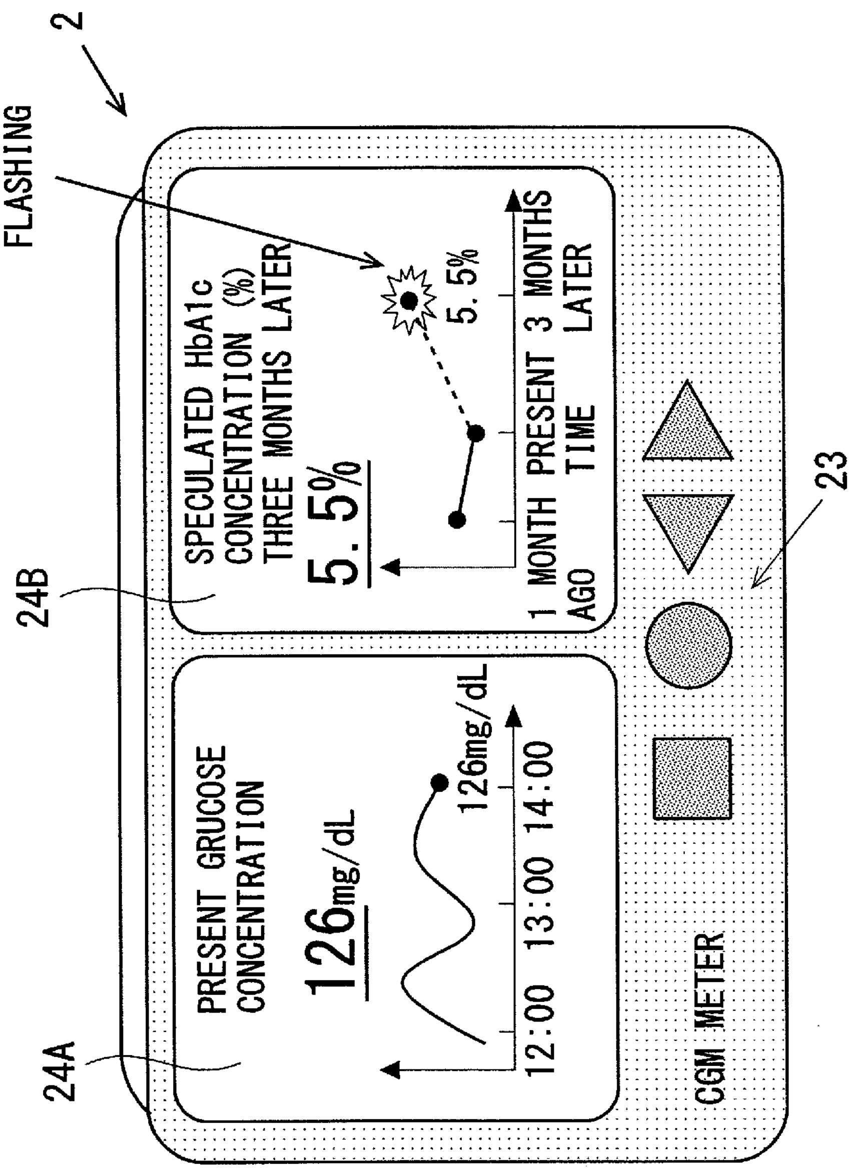

FIG. 17

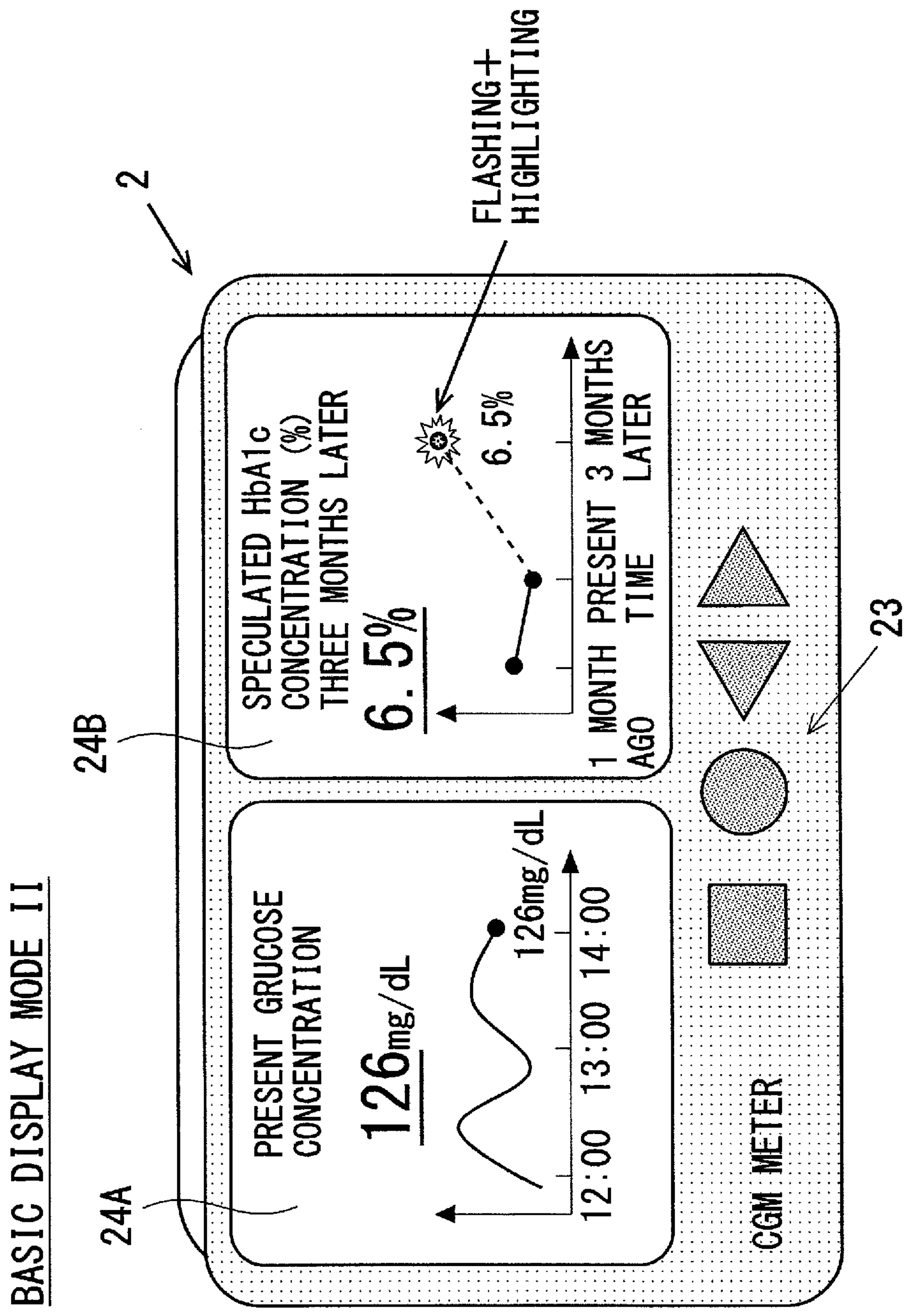
BASIC DISPLAY MODE II
2
24A
24B
PRESENT GRUCOSE CONCENTRATION
126mg/dL
126mg/dL
12:00 13:00 14:00
SPECULATED HbA1c CONCENTRATION (%) THREE MONTHS LATER
6.5%
6.5%
1 MONTH AGO
PRESENT TIME
3 MONTHS LATER
FLASHING+ HIGHLIGHTING
CGM METER
23

FIG. 18

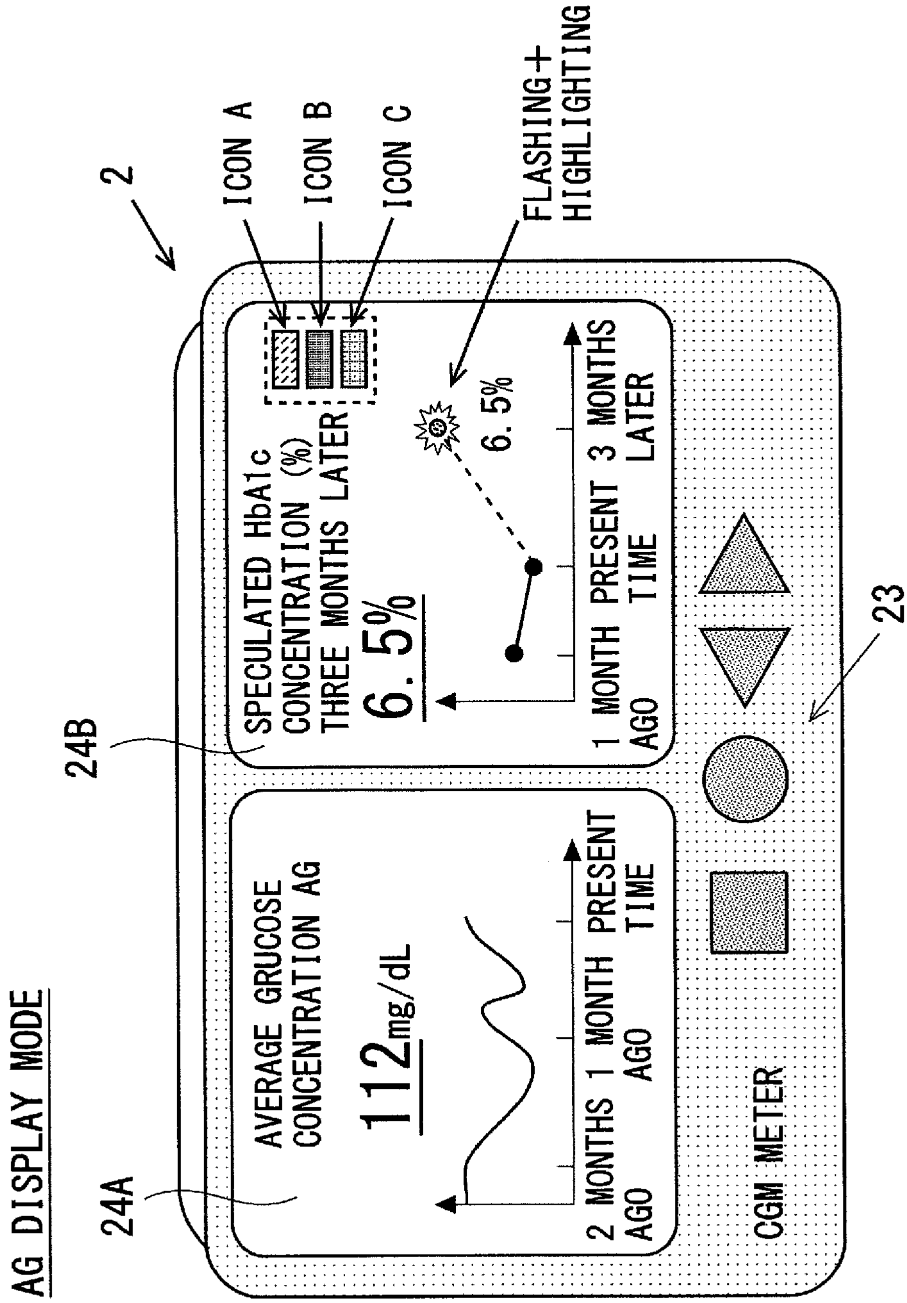
AG DISPLAY MODE
2
ICON A
ICON B
ICON C
FLASHING+
HIGHLIGHTING
24A
24B
AVERAGE GRUCOSE
CONCENTRATION AG
112mg/dL
2 MONTHS AGO
1 MONTH AGO
PRESENT TIME
SPECULATED HbA1c
CONCENTRATION (%)
THREE MONTHS LATER
6.5%
6.5%
1 MONTH AGO
PRESENT TIME
3 MONTHS LATER
CGM METER
23

FIG. 19

ANALYSIS DISPLAY MODE (ANALYSIS BASIC SCREEN)

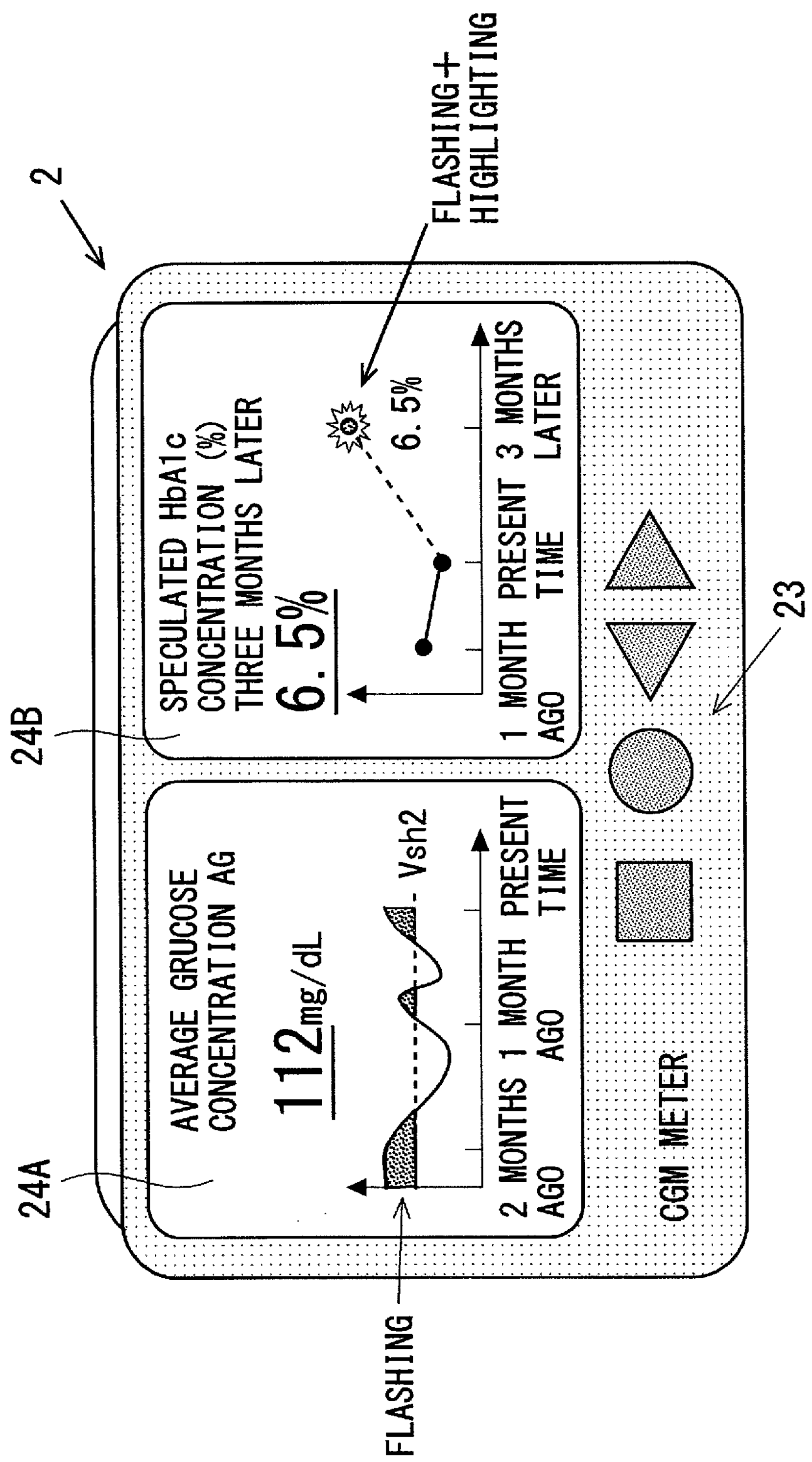

FIG. 20

ANALYSIS DISPLAY MODE (DETAILED INFORMATION SCREEN)

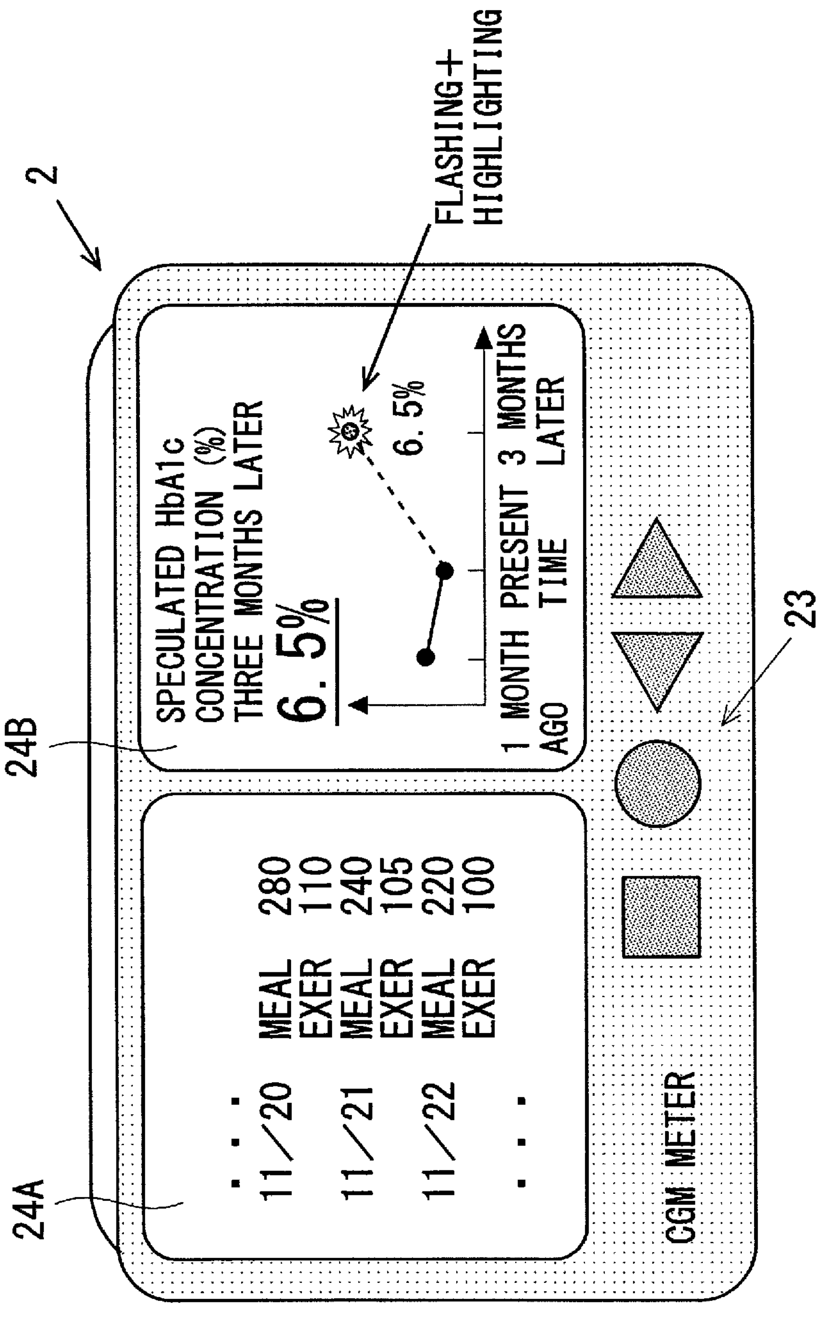

FIG. 21

SECOND ANALYSIS DISPLAY MODE

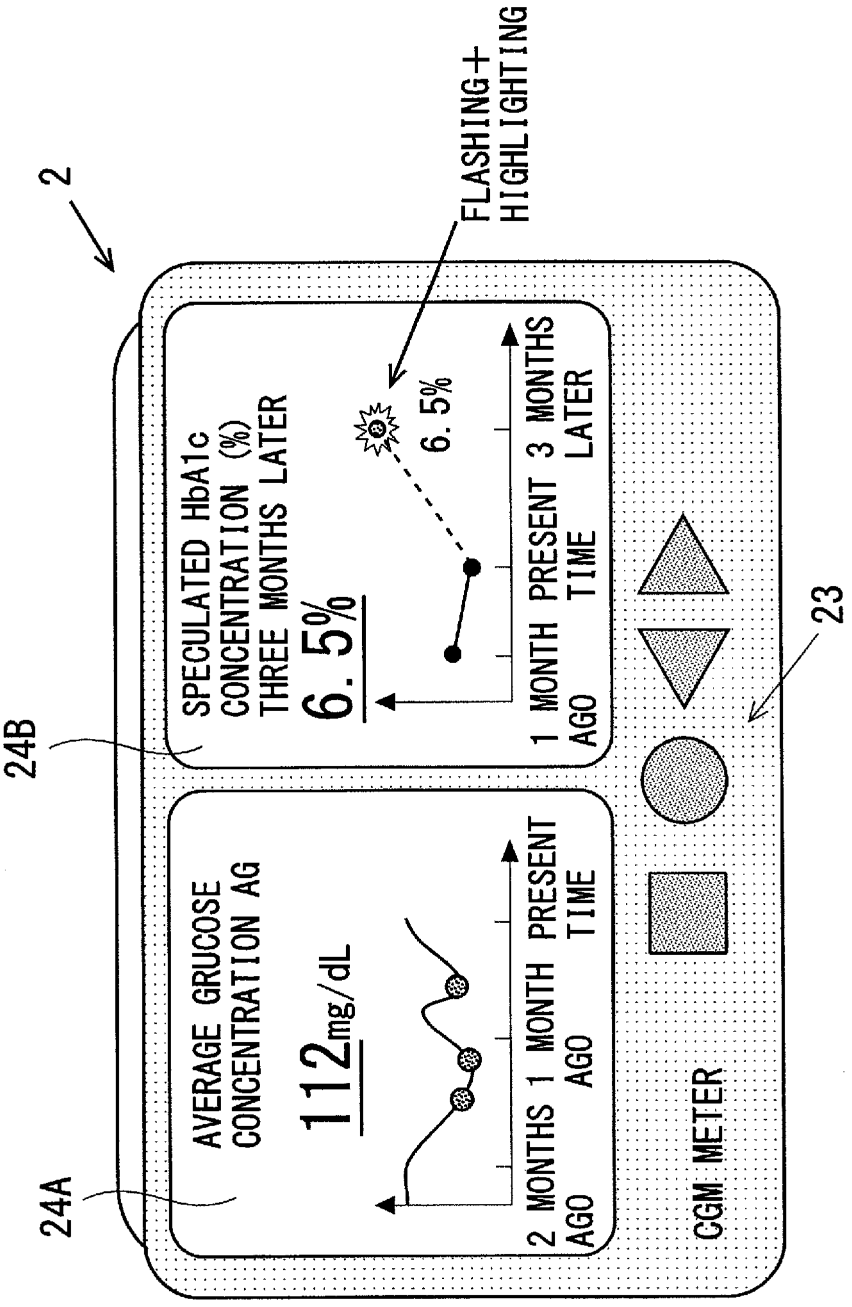

FIG. 22

NOTICE DISPLAY MODE

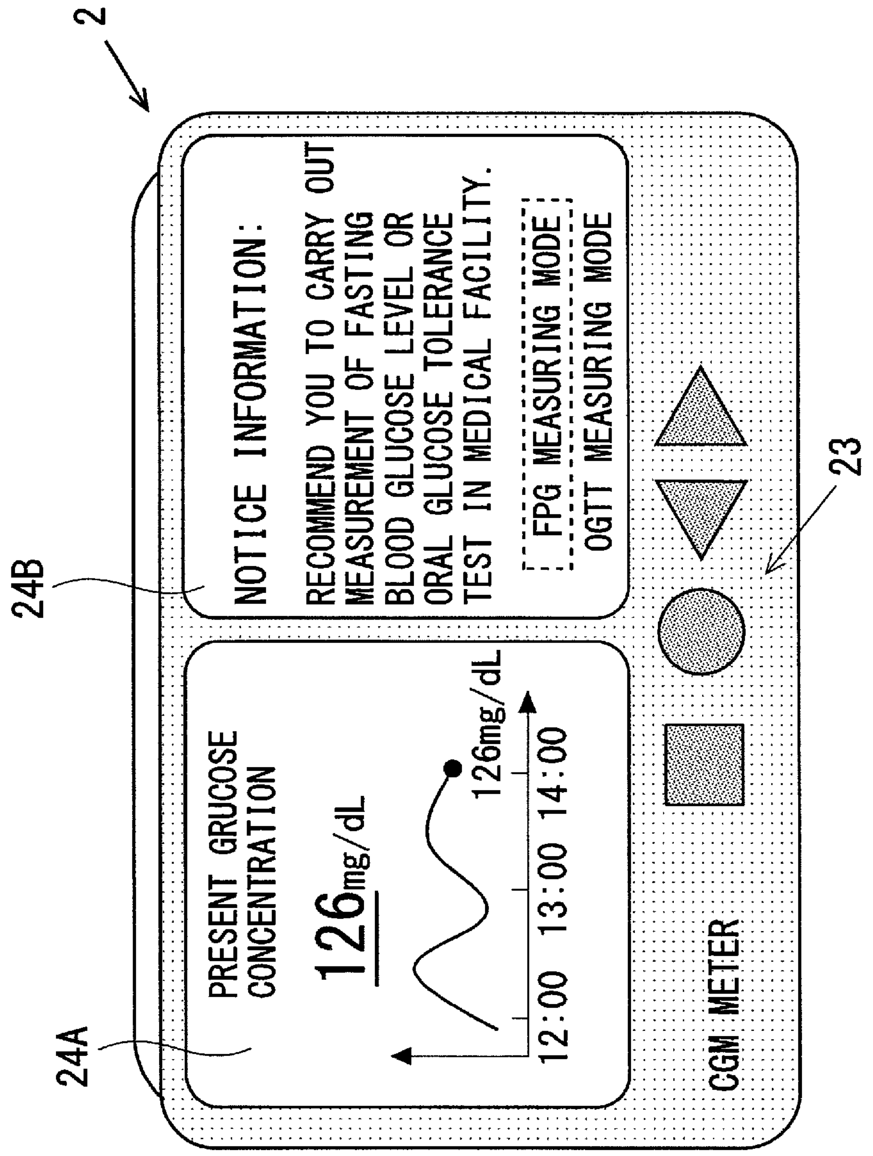

FIG. 23

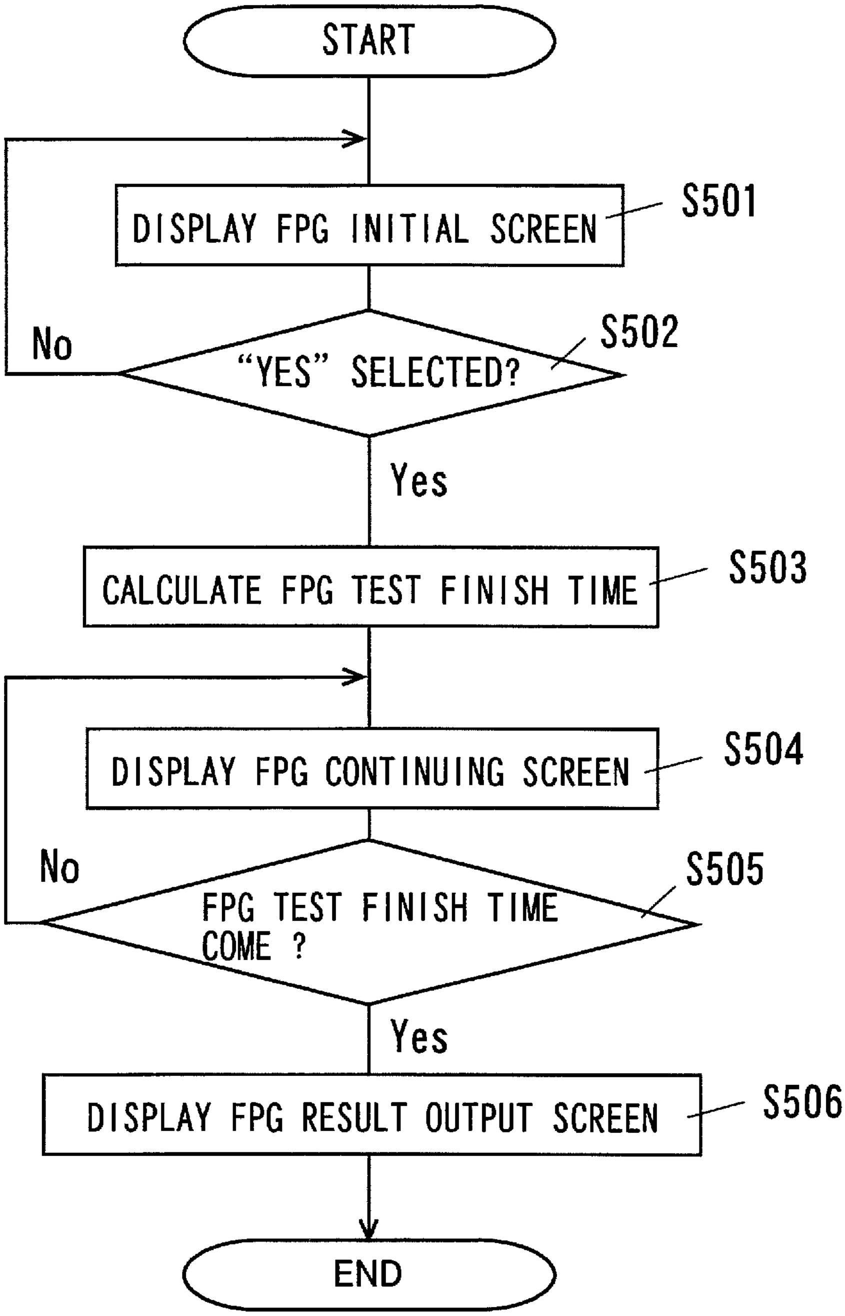
START
DISPLAY FPG INITIAL SCREEN
S501
"YES" SELECTED?
S502
No
Yes
CALCULATE FPG TEST FINISH TIME
S503
DISPLAY FPG CONTINUING SCREEN
S504
FPG TEST FINISH TIME COME ?
S505
No
Yes
DISPLAY FPG RESULT OUTPUT SCREEN
S506
END

FIG. 24

FPG MEASURING MODE (FPG INITIAL SCREEN)

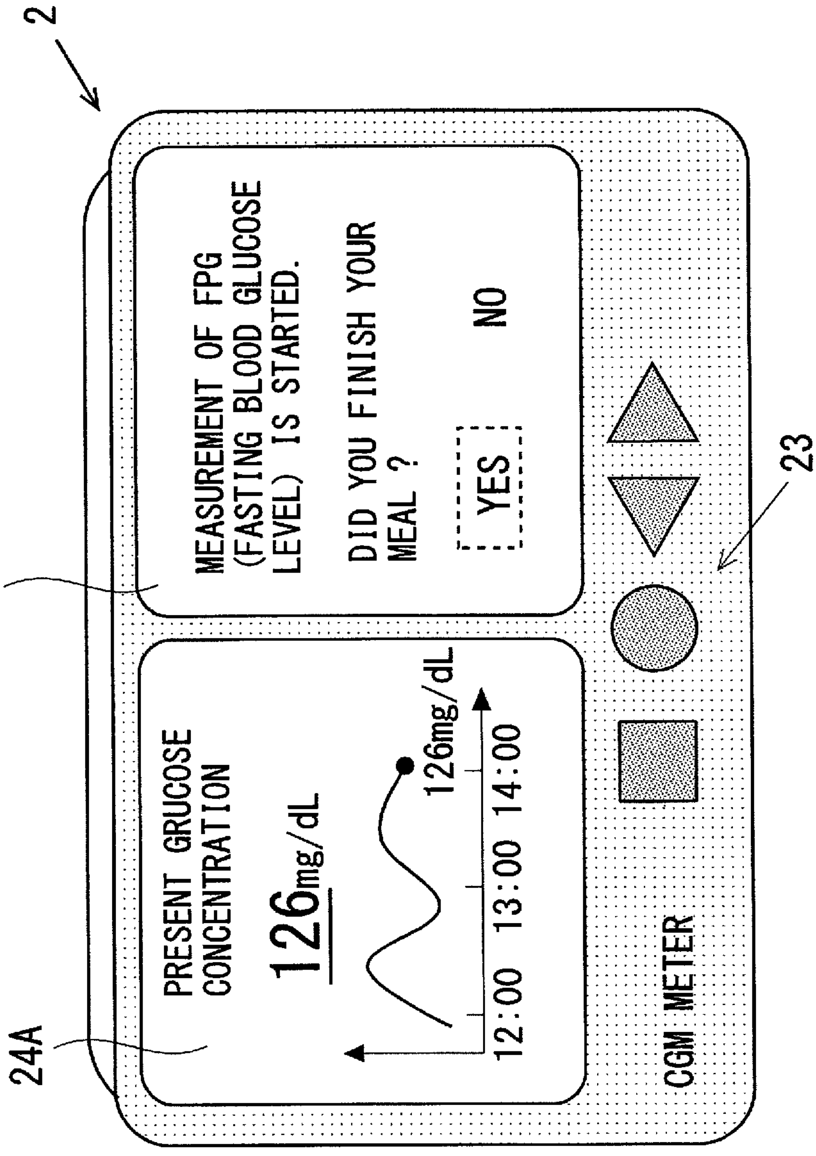

FIG. 25

FPG MEASURING MODE (FPG CONTINUING SCREEN)

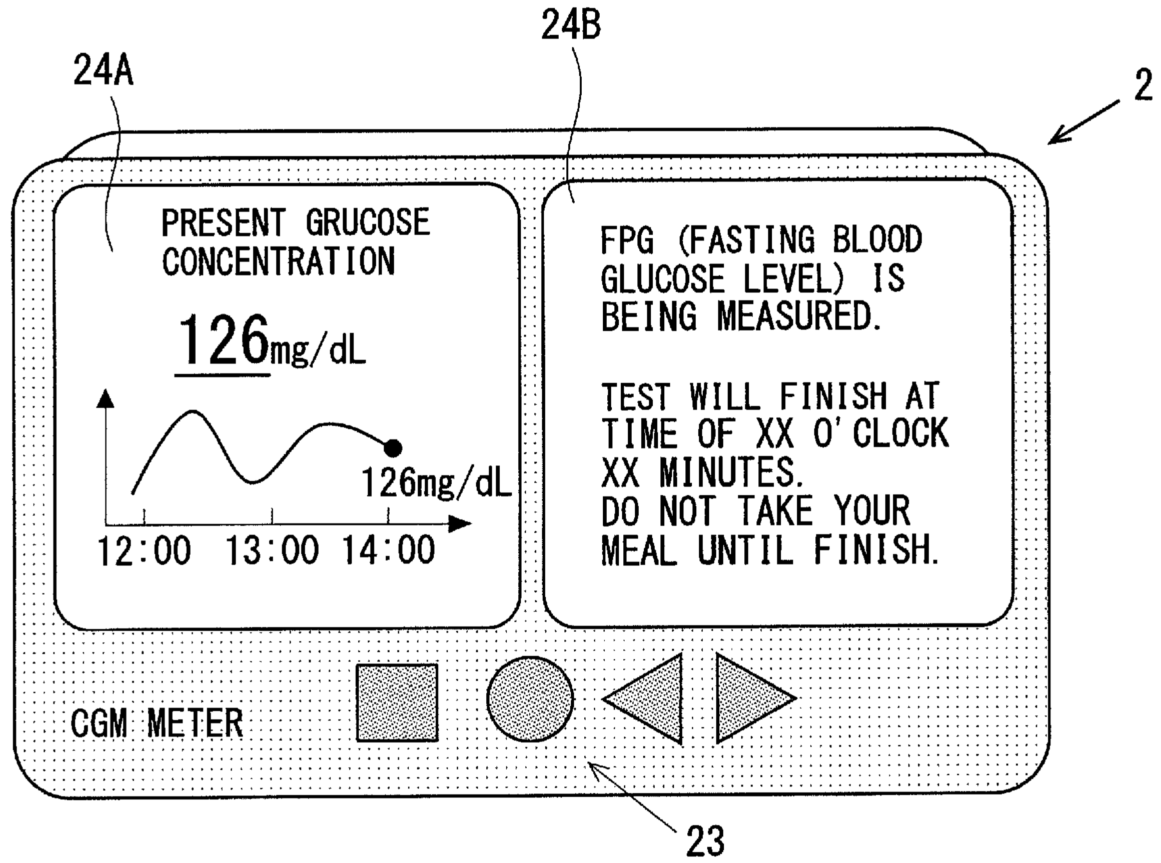

FIG. 26

FPG MEASURING MODE (FPG RESULT OUTPUT SCREEN)

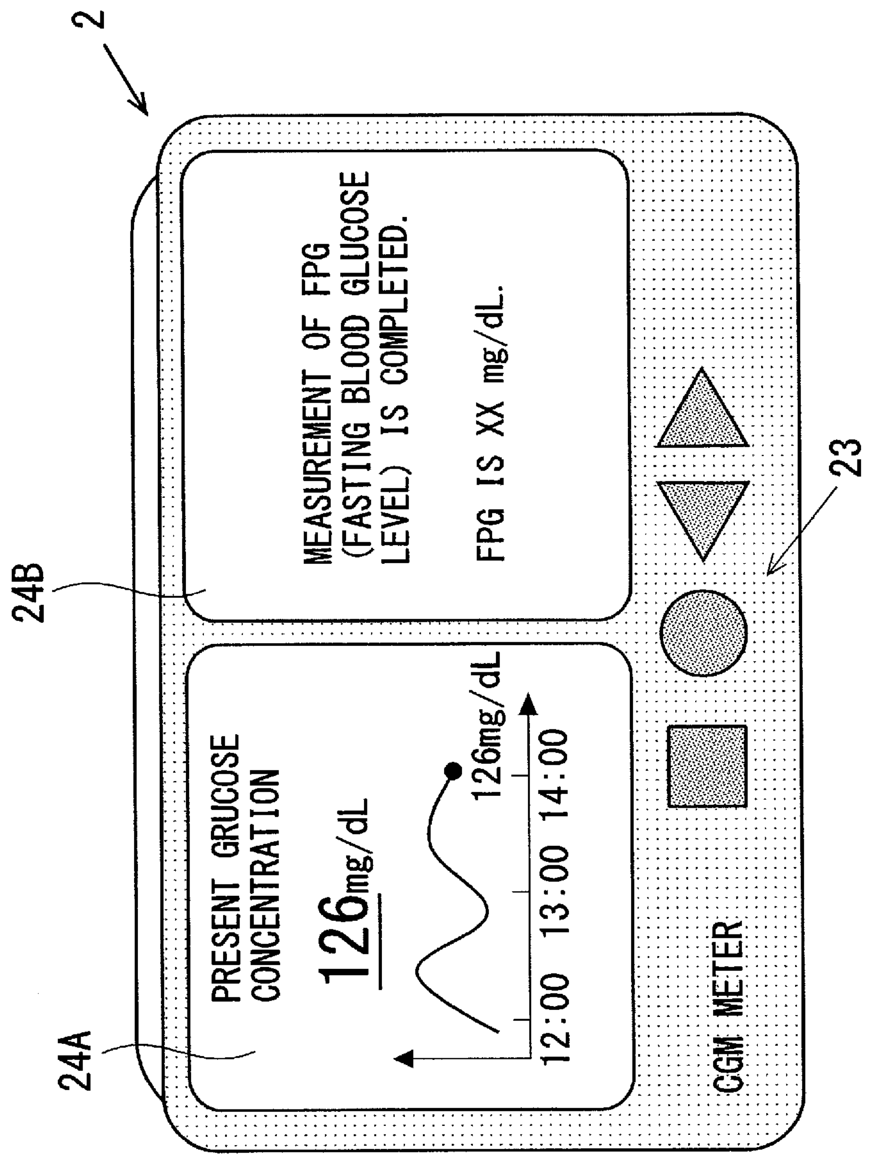

FIG. 27

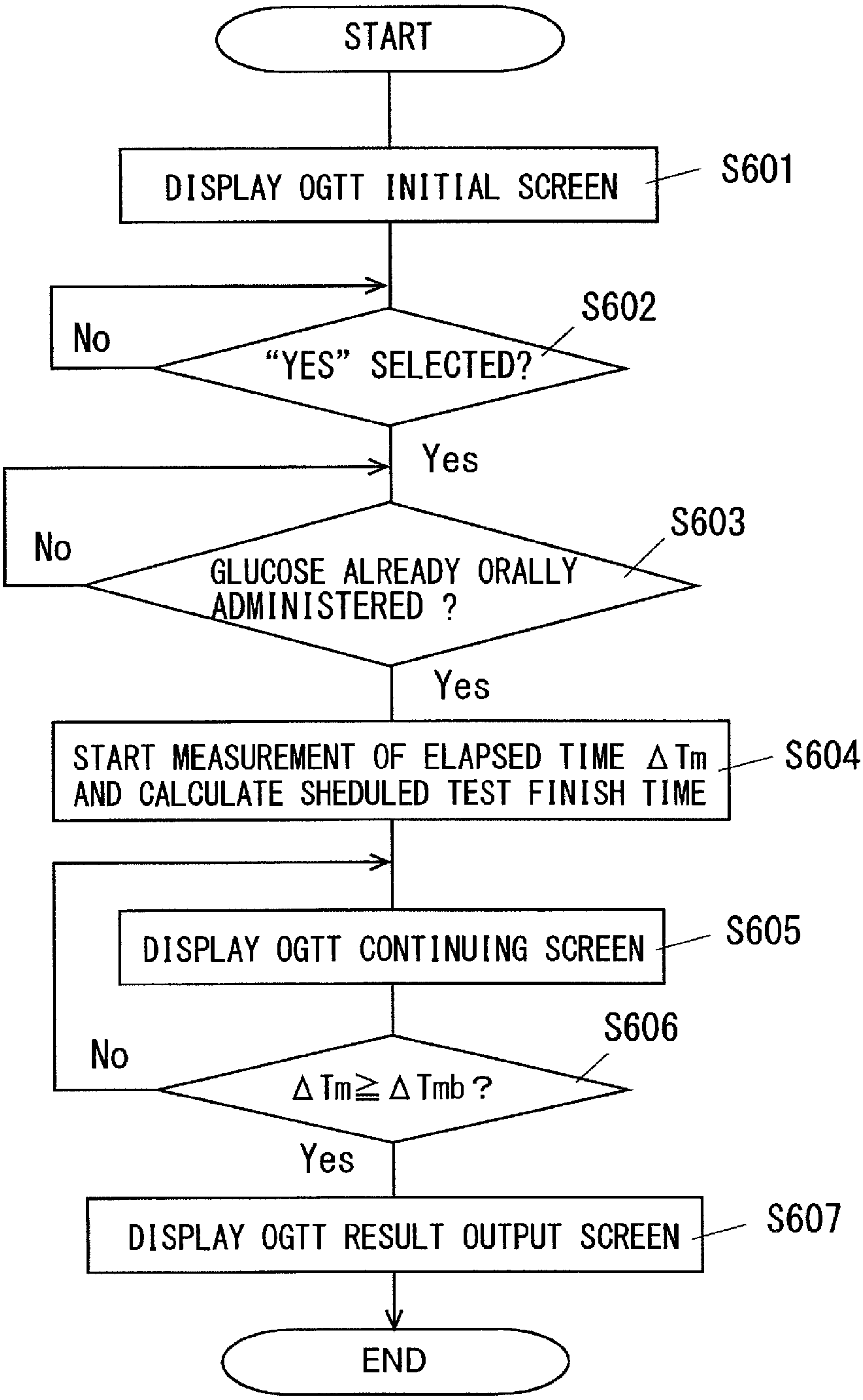
START
DISPLAY OGTT INITIAL SCREEN
S601
"YES" SELECTED?
S602
No
Yes
GLUCOSE ALREADY ORALLY ADMINISTERED ?
S603
No
Yes
START MEASUREMENT OF ELAPSED TIME ΔTm AND CALCULATE SHEDULED TEST FINISH TIME
S604
DISPLAY OGTT CONTINUING SCREEN
S605
ΔTm≧ΔTmb ?
S606
No
Yes
DISPLAY OGTT RESULT OUTPUT SCREEN
S607
END

FIG. 28

OGTT MEASURING MODE (OGTT INITIAL SCREEN)

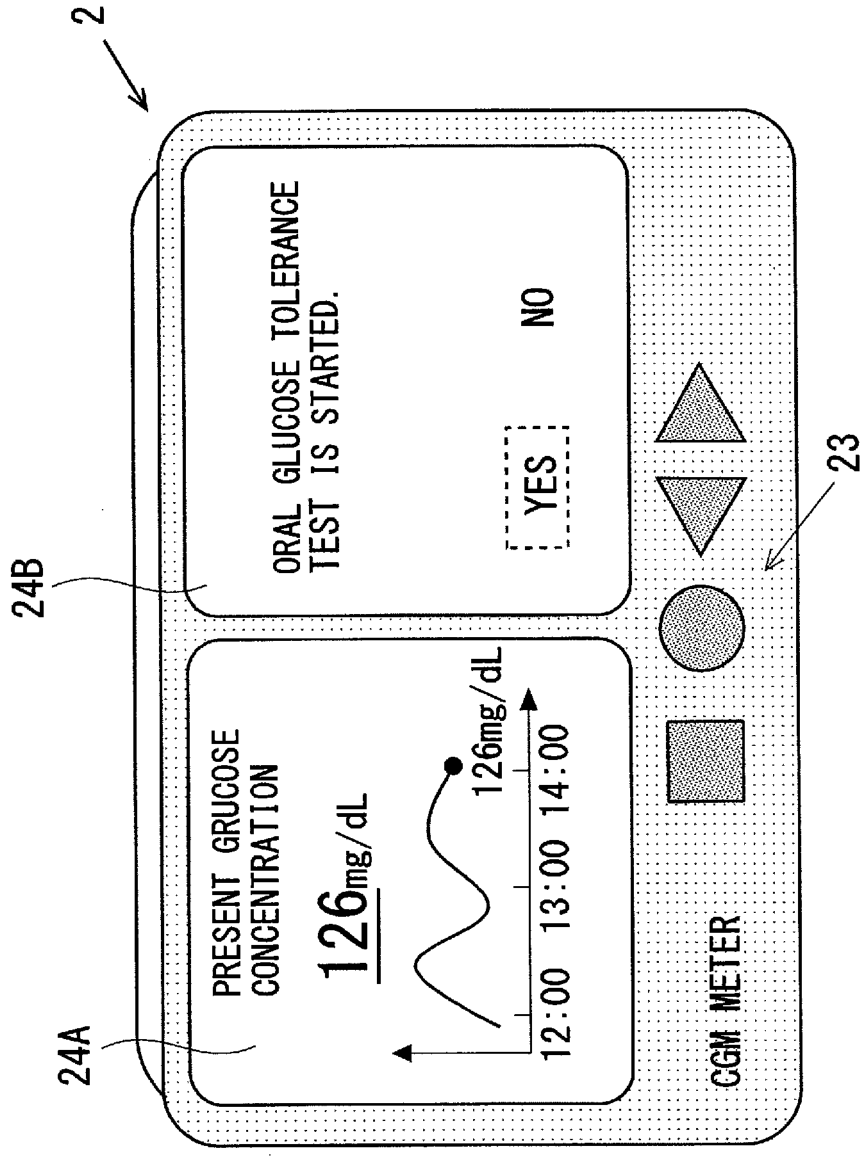

FIG. 29

OGTT MEASURING MODE (OGTT CONTINUING SCREEN)

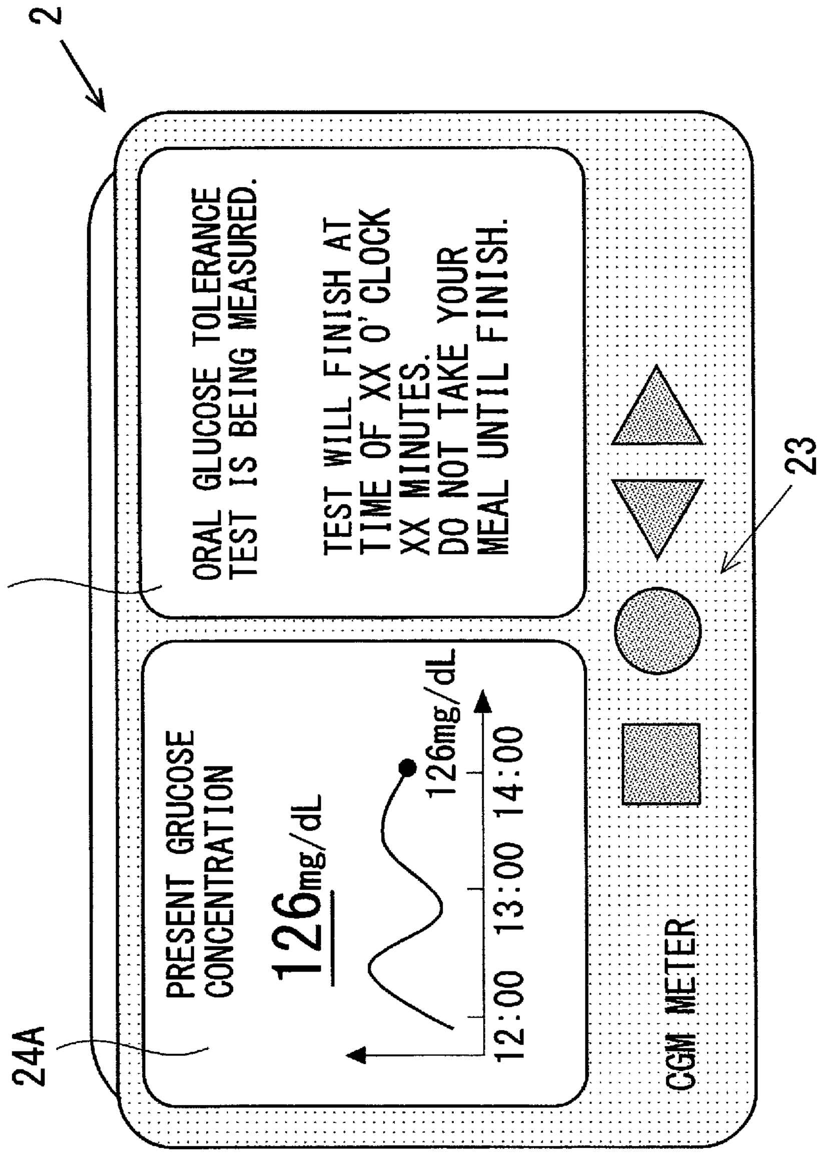

FIG. 30

OGTT MEASURING MODE (OGTT RESULT OUTPUT SCREEN)

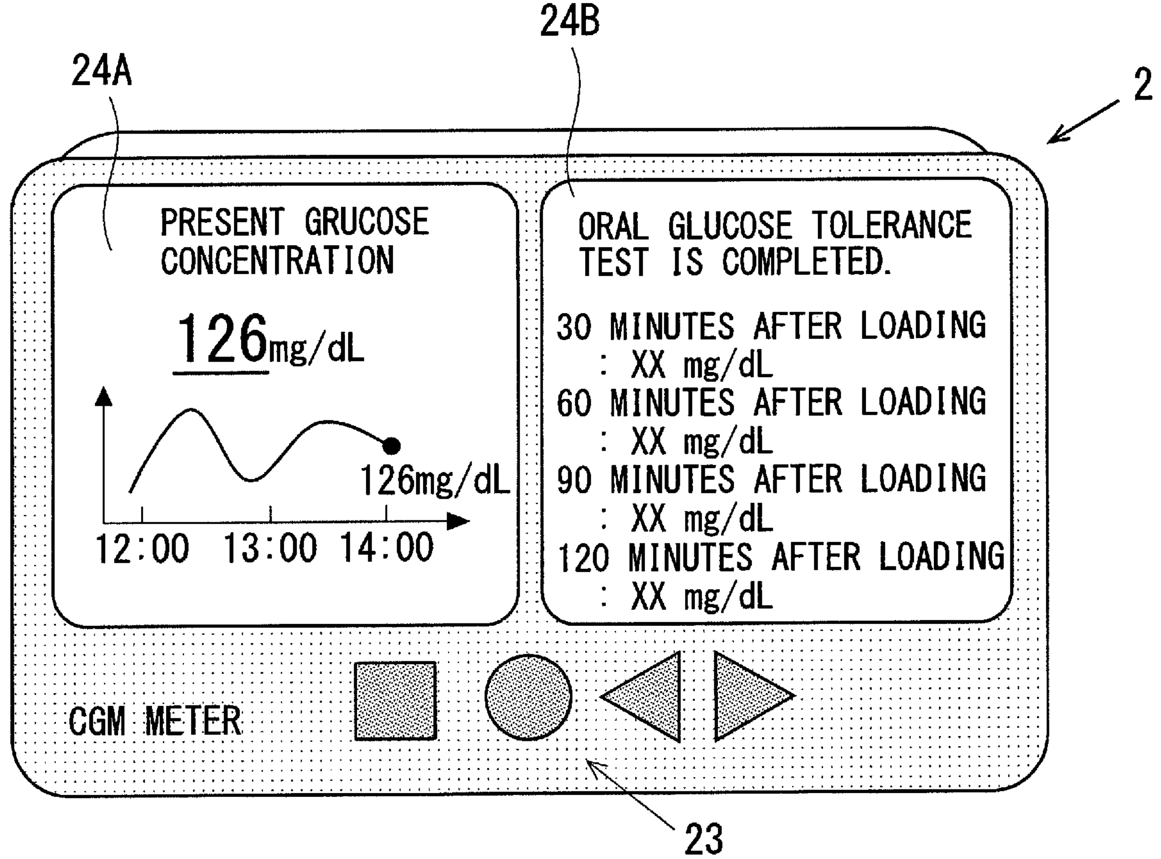

FIG. 31

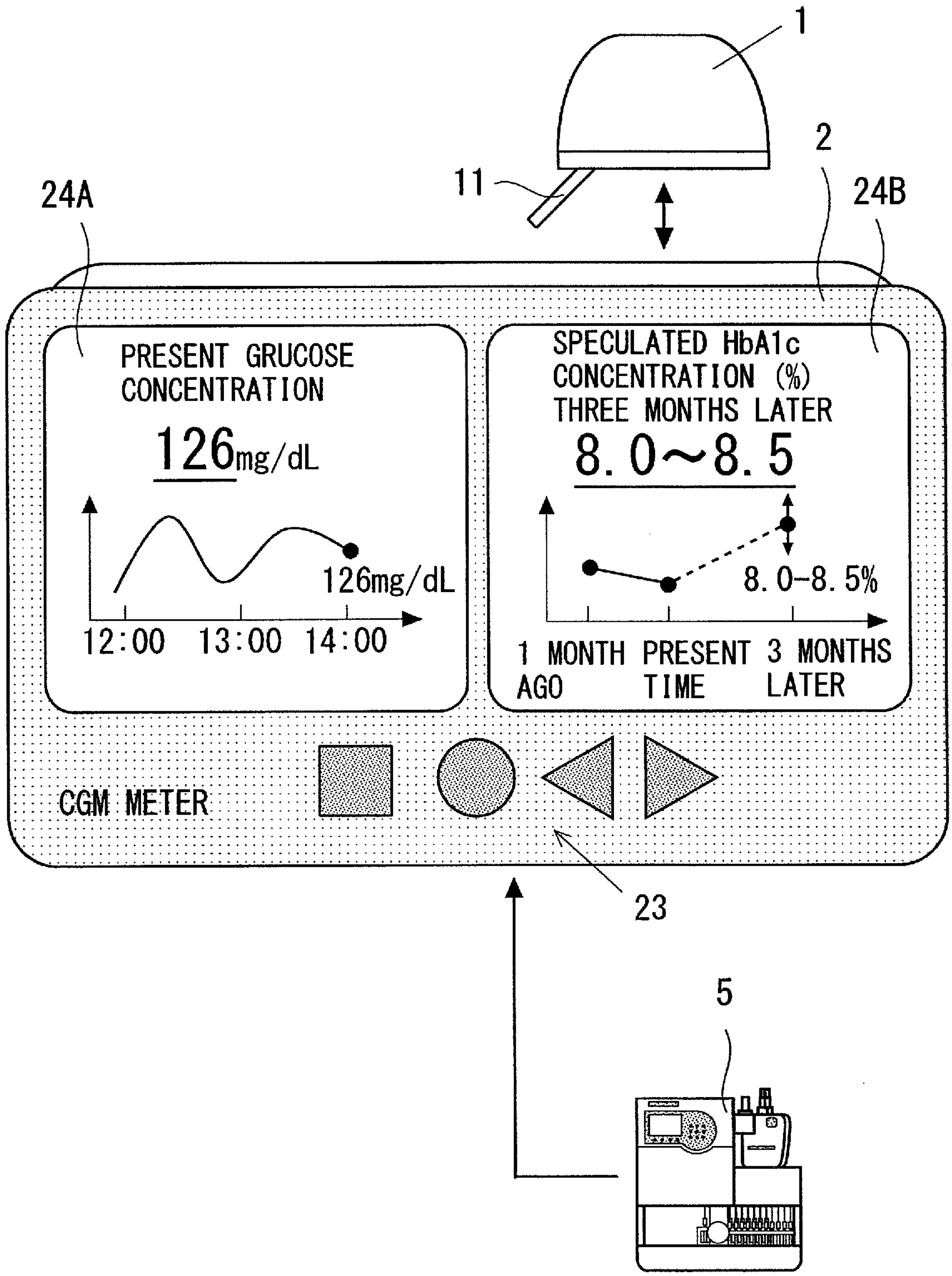
1
11
2
24A
24B
PRESENT GRUCOSE CONCENTRATION
126mg/dL
126mg/dL
12:00 13:00 14:00
SPECULATED HbA1c CONCENTRATION (%) THREE MONTHS LATER
8.0~8.5
8.0-8.5%
1 MONTH AGO
PRESENT TIME
3 MONTHS LATER
CGM METER
23
5

FIG. 32

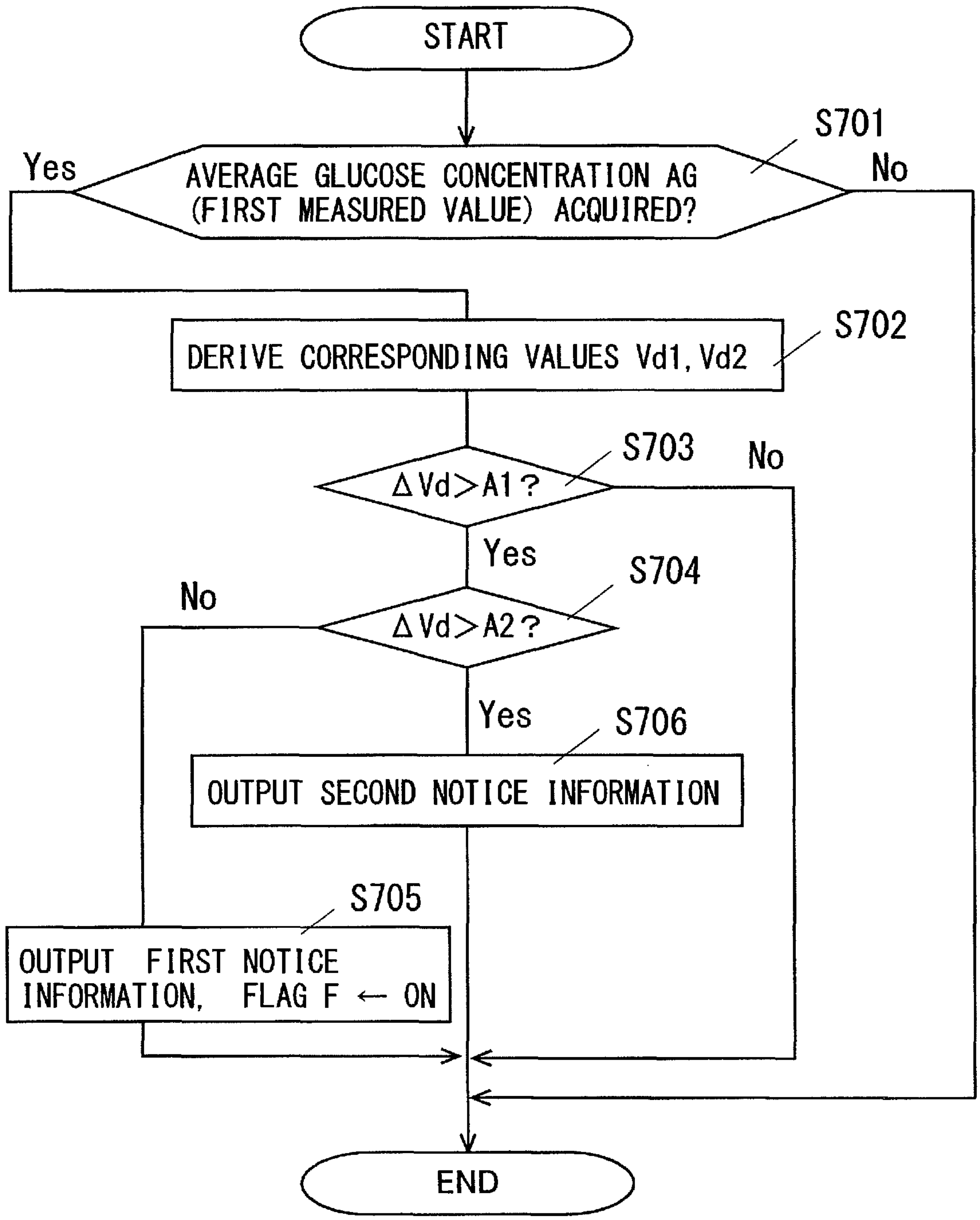
START
S701
Yes
No
AVERAGE GLUCOSE CONCENTRATION AG (FIRST MEASURED VALUE) ACQUIRED?
DERIVE CORRESPONDING VALUES Vd1, Vd2
S702
S703
No
ΔVd>A1?
Yes
S704
No
ΔVd>A2?
Yes
S706
OUTPUT SECOND NOTICE INFORMATION
S705
OUTPUT FIRST NOTICE INFORMATION, FLAG F ← ON
END

FIG. 33

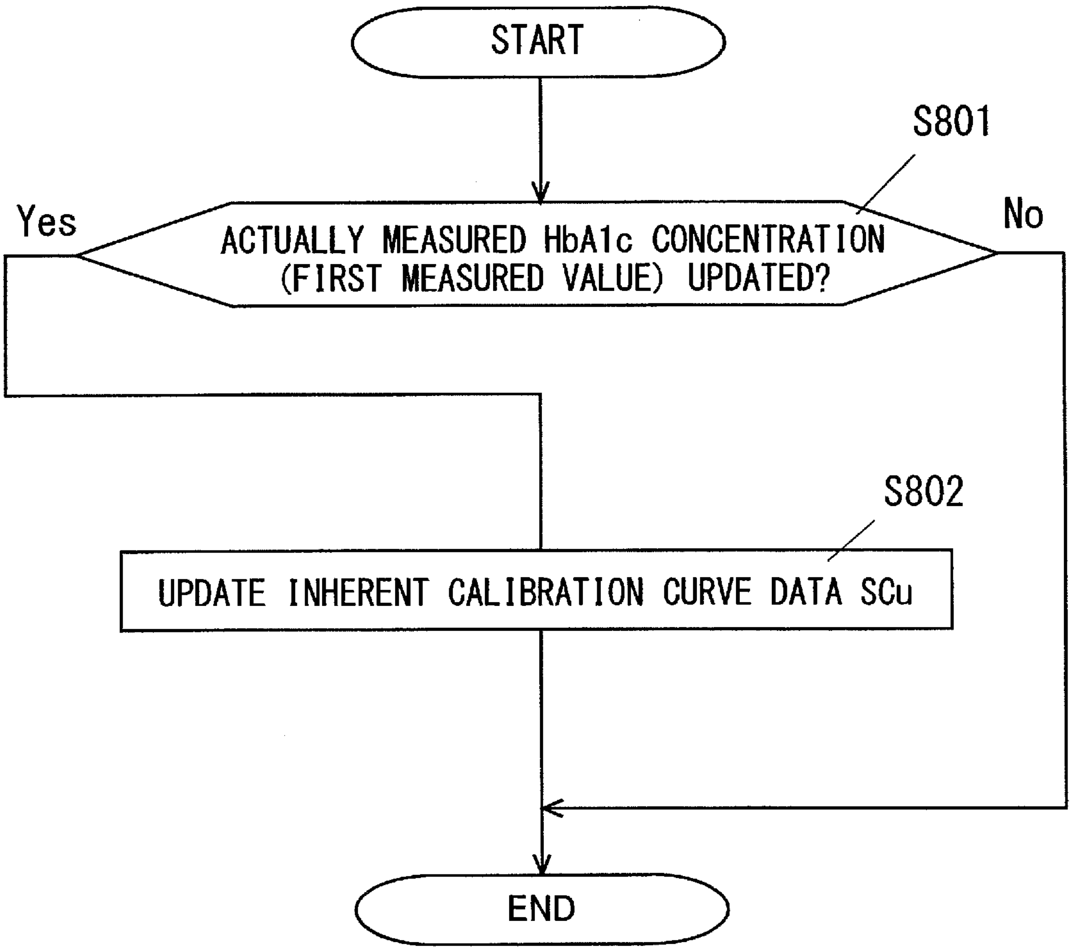
START
S801
Yes
ACTUALLY MEASURED HbA1c CONCENTRATION (FIRST MEASURED VALUE) UPDATED?
No
S802
UPDATE INHERENT CALIBRATION CURVE DATA SCu
END

FIG. 34

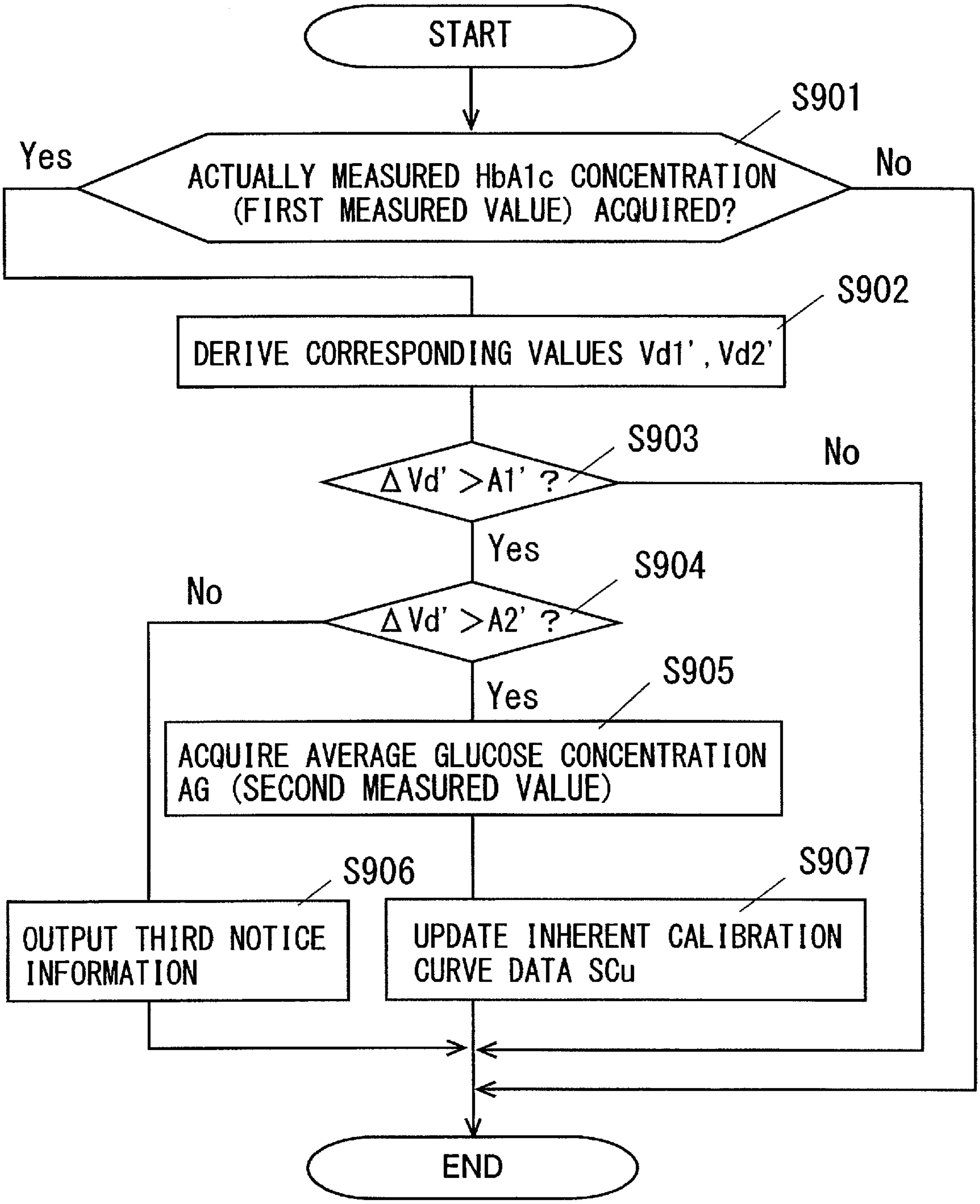
START
S901
Yes
ACTUALLY MEASURED HbA1c CONCENTRATION (FIRST MEASURED VALUE) ACQUIRED?
No
S902
DERIVE CORRESPONDING VALUES Vd1', Vd2'
S903
ΔVd' >A1' ?
No
Yes
S904
No
ΔVd' >A2' ?
S905
Yes
ACQUIRE AVERAGE GLUCOSE CONCENTRATION AG (SECOND MEASURED VALUE)
S906
S907
OUTPUT THIRD NOTICE INFORMATION
UPDATE INHERENT CALIBRATION CURVE DATA SCu
END

FIG. 35

SIMULTANEOUS DISPLAY MODE

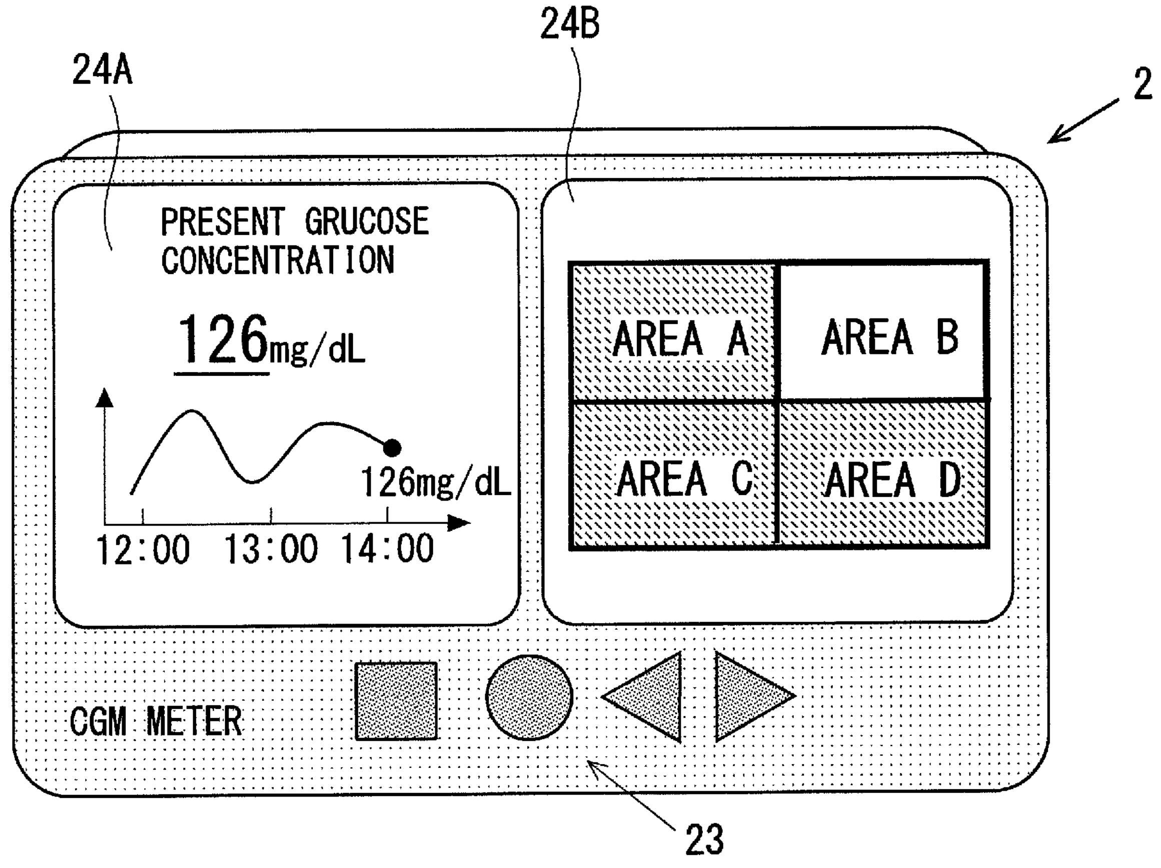

FIG. 36

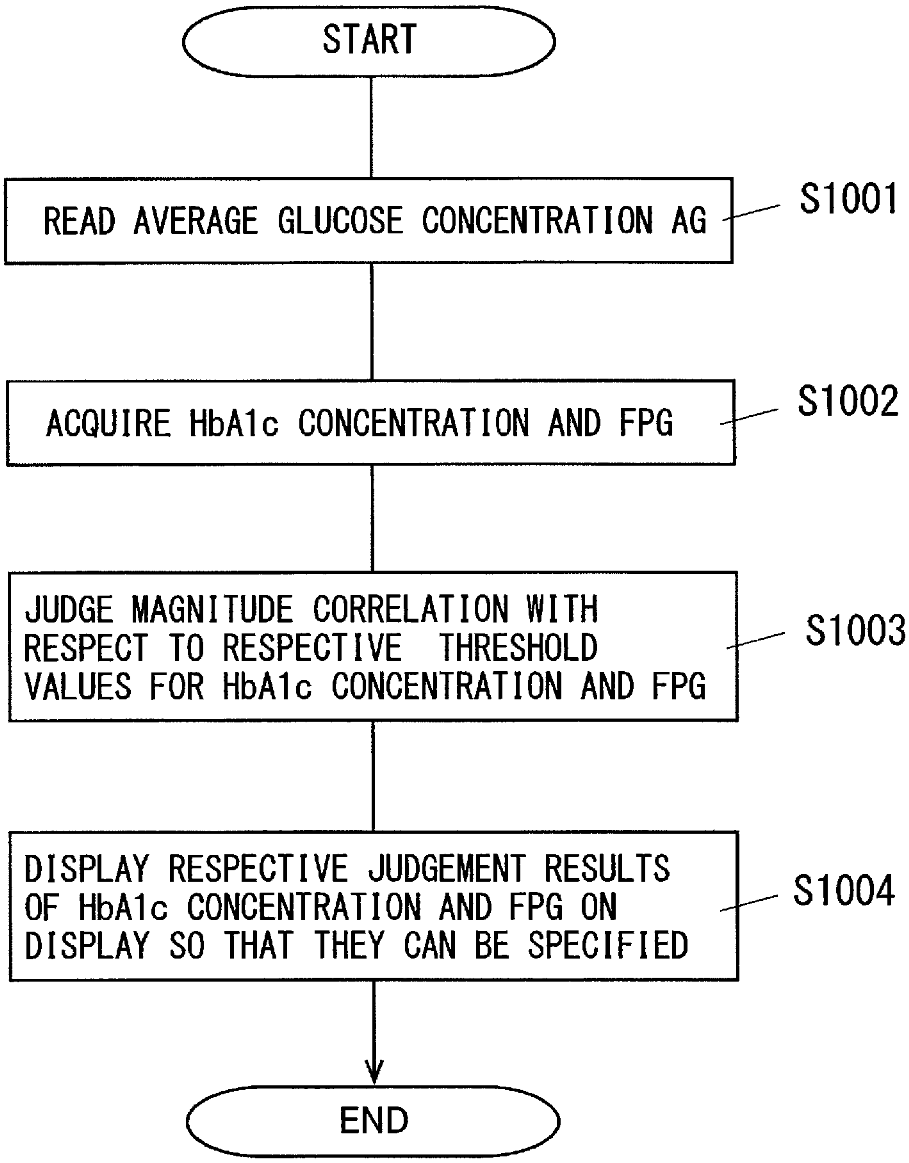
START
READ AVERAGE GLUCOSE CONCENTRATION AG
S1001
ACQUIRE HbA1c CONCENTRATION AND FPG
S1002
JUDGE MAGNITUDE CORRELATION WITH RESPECT TO RESPECTIVE THRESHOLD VALUES FOR HbA1c CONCENTRATION AND FPG
S1003
DISPLAY RESPECTIVE JUDGEMENT RESULTS OF HbA1c CONCENTRATION AND FPG ON DISPLAY SO THAT THEY CAN BE SPECIFIED
S1004
END

FIG. 37

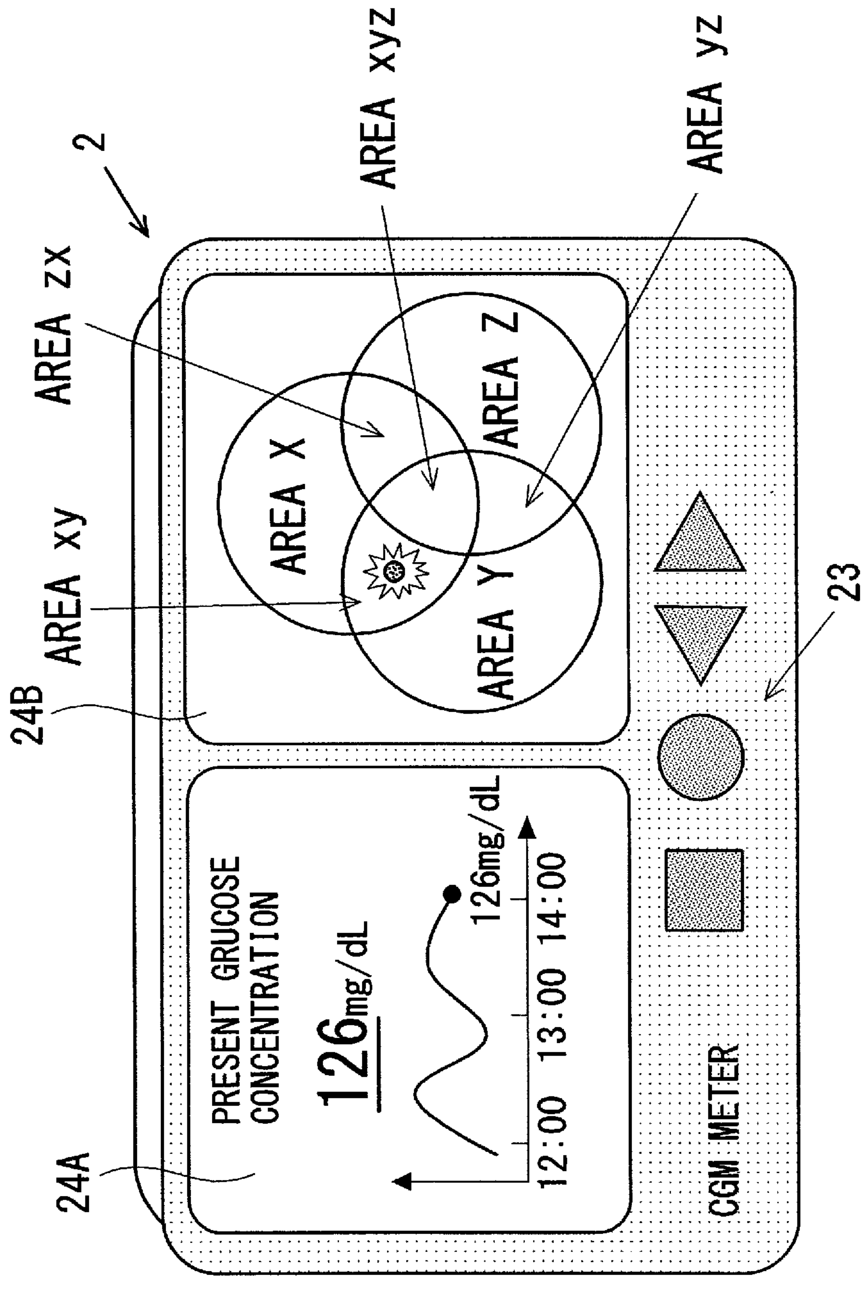
2
AREA zx
AREA xyz
AREA yz
AREA xy
AREA X
AREA Z
AREA Y
24B
24A
PRESENT GRUCOSE
CONCENTRATION
126mg/dL
126mg/dL
12:00 13:00 14:00
CGM METER
23

FIG. 38

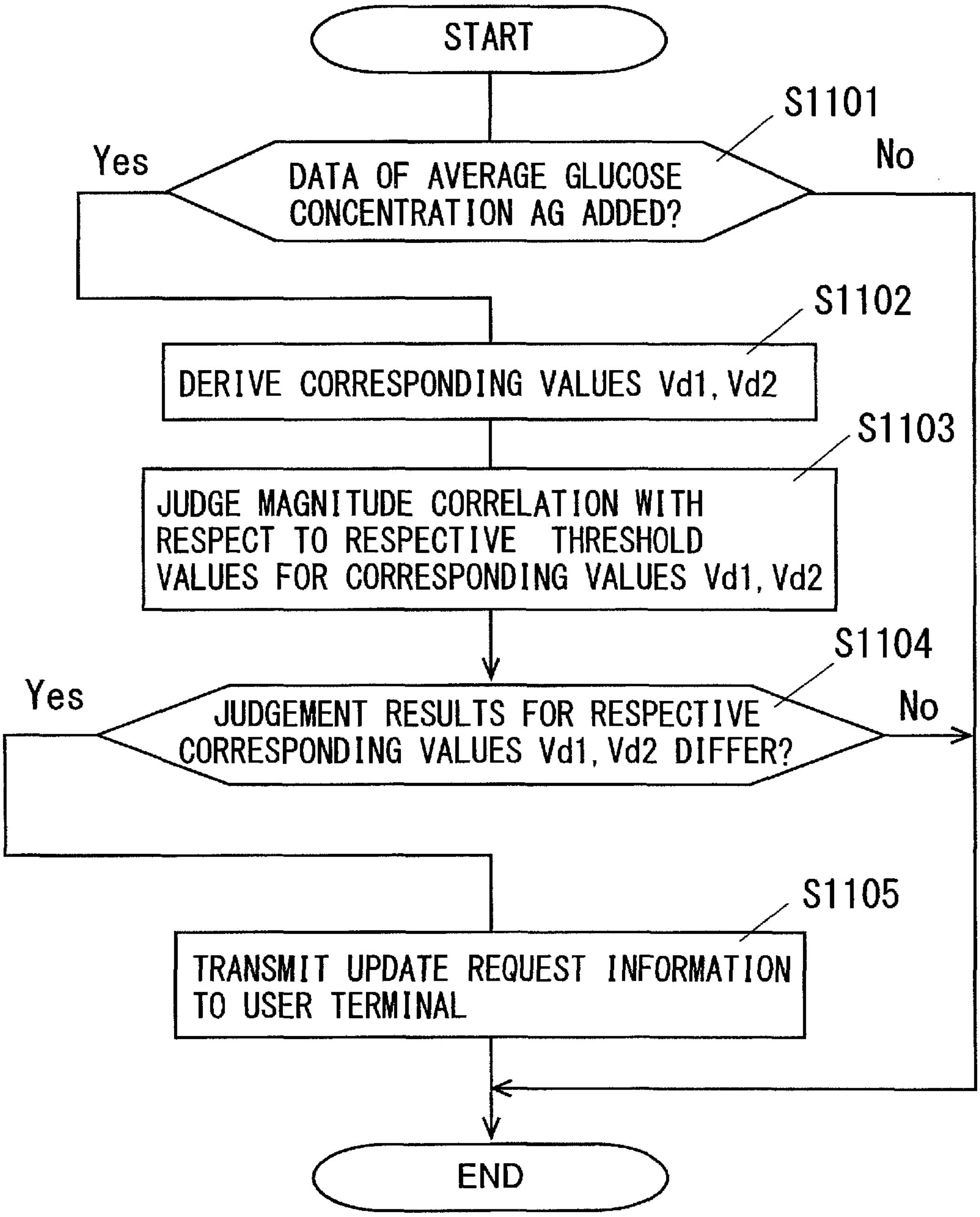
START
S1101
Yes
DATA OF AVERAGE GLUCOSE CONCENTRATION AG ADDED?
No
S1102
DERIVE CORRESPONDING VALUES Vd1, Vd2
S1103
JUDGE MAGNITUDE CORRELATION WITH RESPECT TO RESPECTIVE THRESHOLD VALUES FOR CORRESPONDING VALUES Vd1, Vd2
S1104
Yes
JUDGEMENT RESULTS FOR RESPECTIVE CORRESPONDING VALUES Vd1, Vd2 DIFFER?
No
S1105
TRANSMIT UPDATE REQUEST INFORMATION TO USER TERMINAL
END

*FIG. 39*

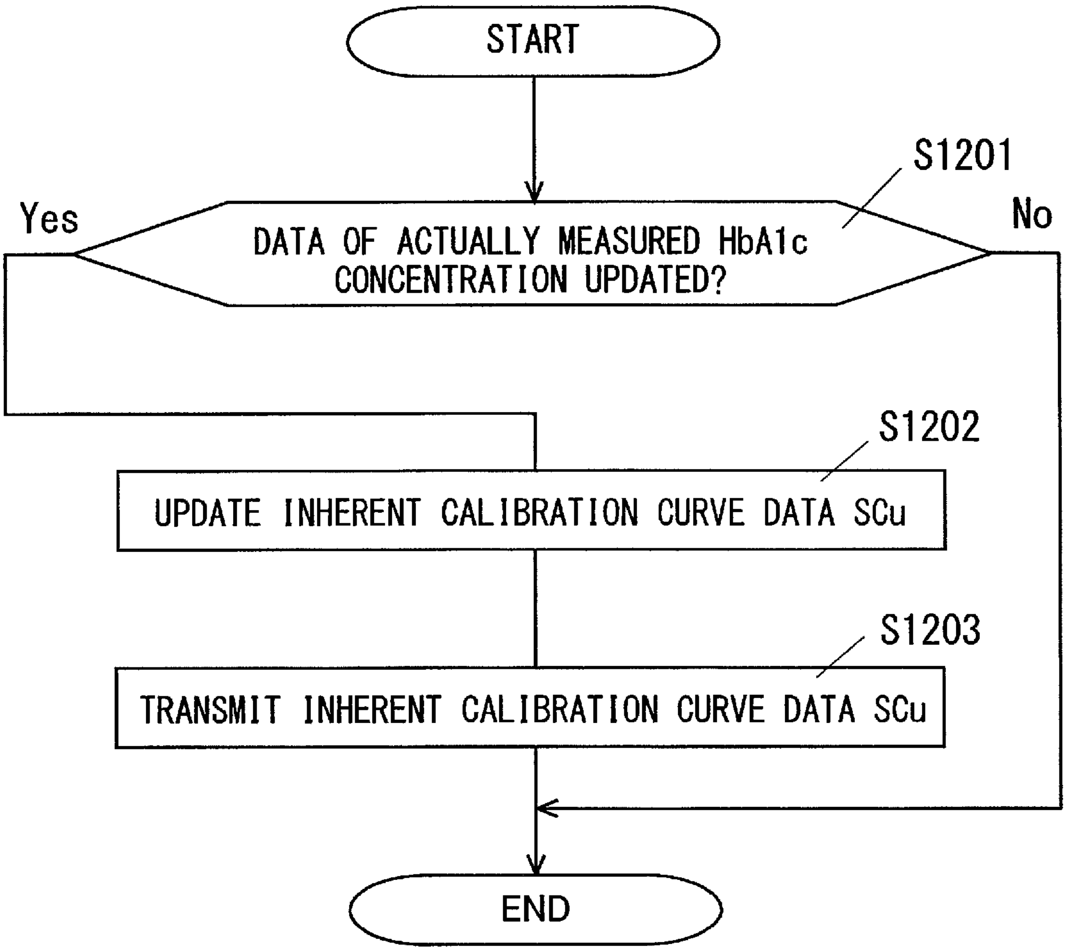
START
S1201
Yes
DATA OF ACTUALLY MEASURED HbA1c CONCENTRATION UPDATED?
No
S1202
UPDATE INHERENT CALIBRATION CURVE DATA SCu
S1203
TRANSMIT INHERENT CALIBRATION CURVE DATA SCu
END

FIG. 40

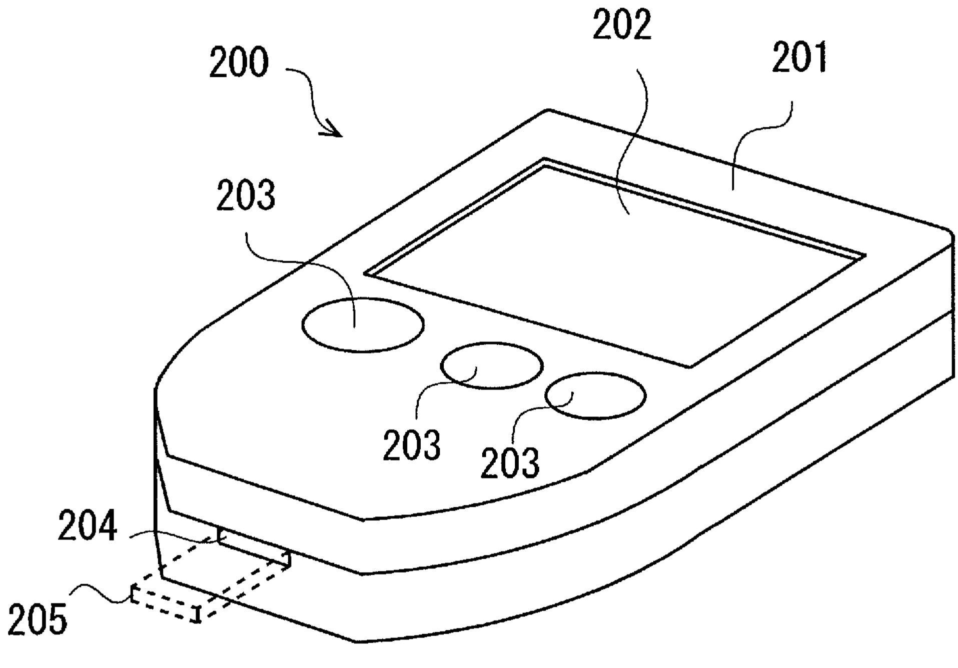

FIG. 41

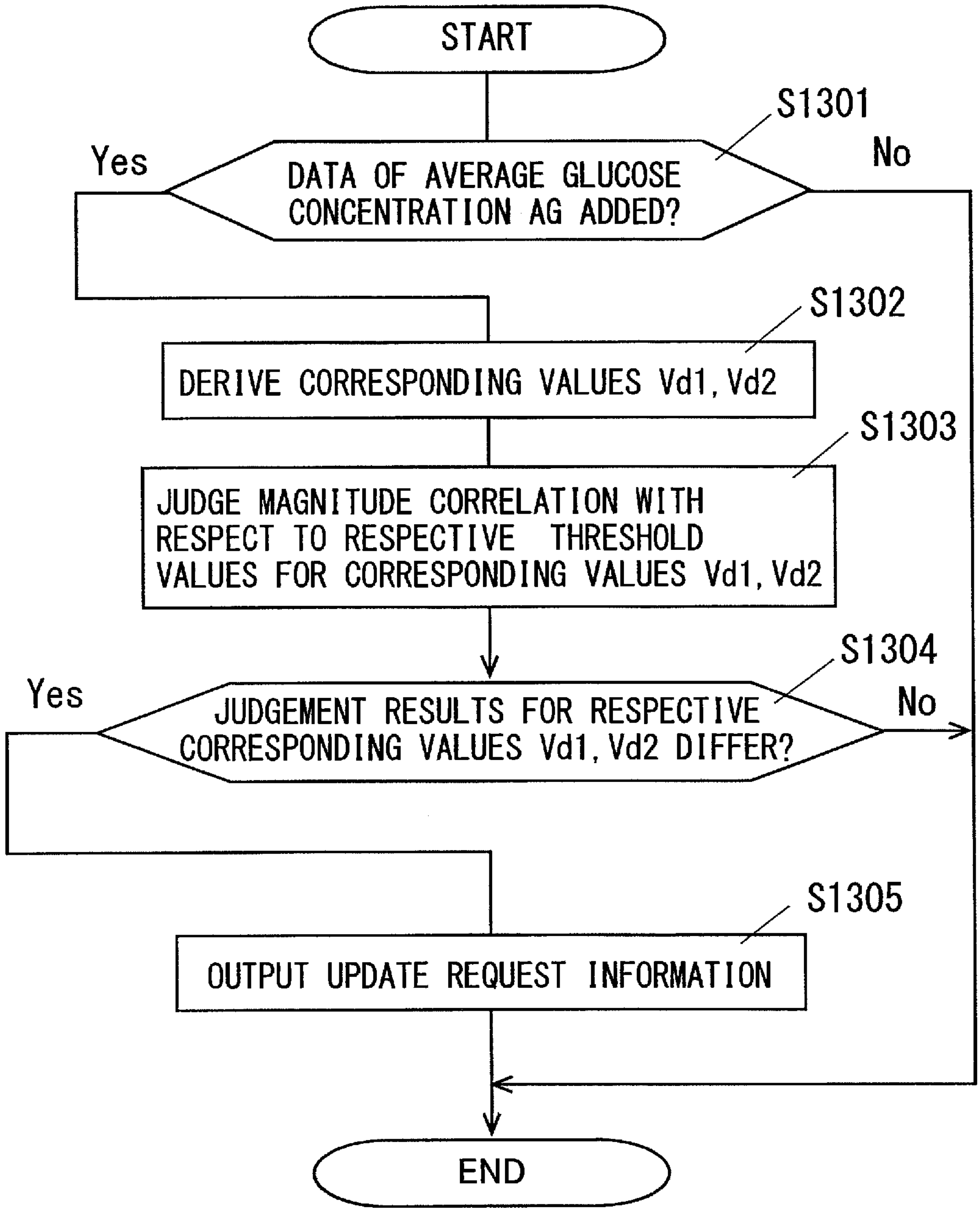
START
S1301
Yes
DATA OF AVERAGE GLUCOSE CONCENTRATION AG ADDED?
No
S1302
DERIVE CORRESPONDING VALUES Vd1, Vd2
S1303
JUDGE MAGNITUDE CORRELATION WITH RESPECT TO RESPECTIVE THRESHOLD VALUES FOR CORRESPONDING VALUES Vd1, Vd2
S1304
Yes
JUDGEMENT RESULTS FOR RESPECTIVE CORRESPONDING VALUES Vd1, Vd2 DIFFER?
No
S1305
OUTPUT UPDATE REQUEST INFORMATION
END

FIG. 42

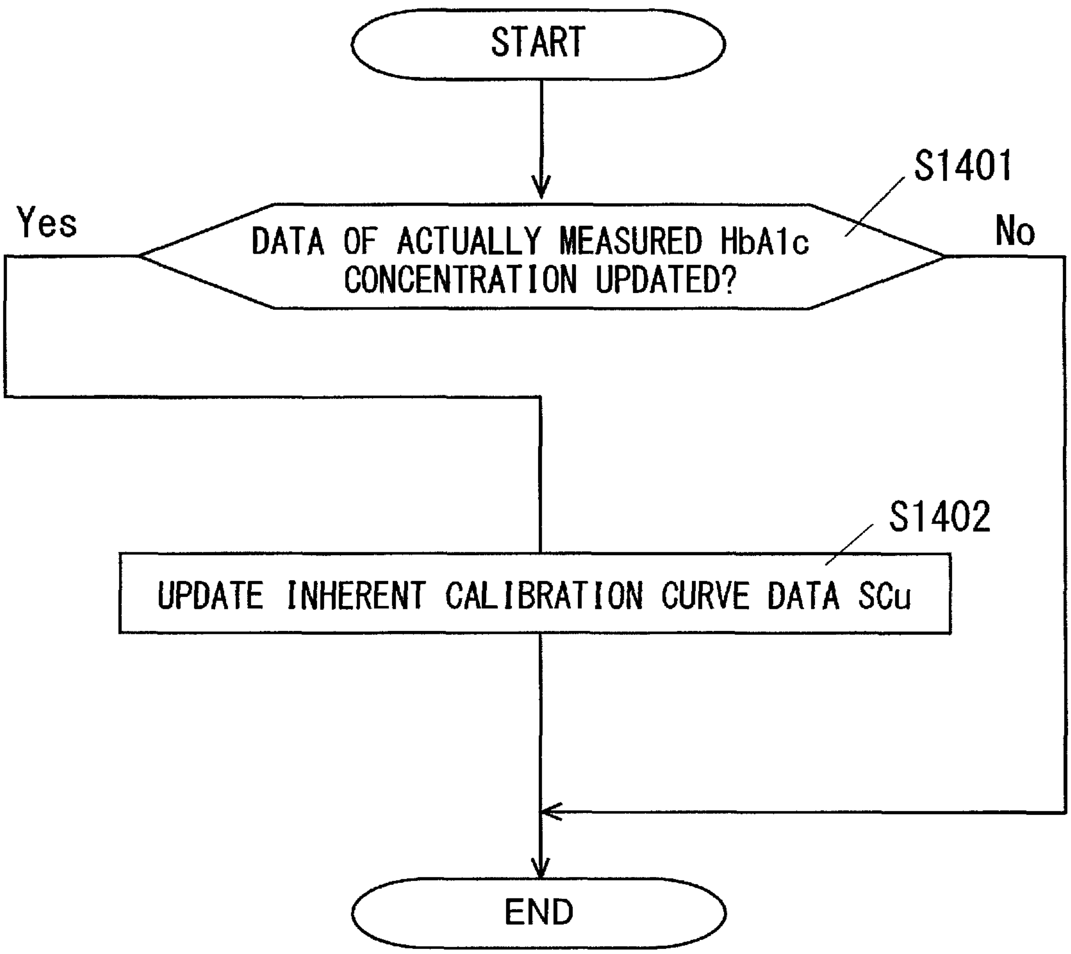
START
S1401
Yes
DATA OF ACTUALLY MEASURED HbA1c CONCENTRATION UPDATED?
No
S1402
UPDATE INHERENT CALIBRATION CURVE DATA SCu
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10332704 A
- JP 4523082 B
- WO 2007091654 A
- JP 2003528330 W
- JP 2005535885 W
- JP 2009210549 A
- US 2010162786 A1
- US 2010330598 A1


### Non-patent literature cited in the description


- **DAVID M. NATHAN.** Translating the A1c assay into Estimated Average Glucose Values. *Diabetes Care,* 08 August 2008, vol. 31
- 2010 Consensus Statement on the Worldwide Standardization of the Hemoglobin A1c Measurement. *Diabetes Care,* 08 August 2010, vol. 33
- Postchallenge Glucose, A1c, and Fasting Glucose as Predictors of Type 2 Diabetes and Cardiovascular Diseases. *Diabetes Care,* 09 September 2010, vol. 33
- Impact of A1c Screening Criterion on the Diagnosis of Pre-Diabetes Among U.S. Adults. *Diabetes Care,* 10 October 2010, vol. 33
- HbA1c 5.7-6.4% and impaired fasting plasma glucose for diagnosis of prediabetes and risk of progression to diabetes in Japan (TOPICS 3): a longitudinal cohort study. The Lancet. Early Online Publication, 25 June 2011